# EUROPEAN PATENT SPECIFICATION

(11) **EP 2 444 811 B1**
(45) Date of publication and mention of the grant of the patent: **05.08.2015**
(21) Application number: 10730815.7
(22) Date of filing: 25.05.2010
(51) Int. Cl.: G01N 33/574

(54) **METHODS FOR THE DIAGNOSIS OR PROGNOSIS OF COLORECTAL CANCER**
VERFAHREN ZUR DIAGNOSE ODER PROGNOSE VON KOLOREKTALKARZINOM
MÉTHODES POUR LE DIAGNOSTIC OU LE PRONOSTIC DU CANCER COLORECTAL

(30) Priority: 25.05.2009 ES 200930203
(43) Date of publication of application: 25.04.2012
(73) Proprietor: Consejo Superior De Investigaciones Científicas (CSIC), 28006 Madrid (ES); Fundación Centro Nacional de Investigaciones Oncológicas Carlos III, 28029 Madrid (ES)
(72) Inventor: CASAL ÁLVAREZ, José Ignacio, E-28040 Madrid (ES); BARDERAS MANCHADO, Rodrigo, E-28040 Madrid (ES); BABEL, Ingrid Henriette Suzanne, E-28040 Madrid (ES)
(74) Representative: ABG Patentes, S.L.
(86) International application number: PCT/ES2010/070350
(87) International publication number: WO 2010/136629

(56) References cited:
- WO-A2-2006/056766
- WO-A2-2008/061104
- BABEL INGRID ET AL: "Identification of Tumor-associated Autoantigens for the Diagnosis of Colorectal Cancer in Serum Using High Density Protein Microarrays" MOLECULAR & CELLULAR PROTEOMICS, vol. 8, no. 10, October 2009 (2009-10), pages 2382-2395, XP9137622 ISSN: 1535-9476
- HANASH SAMIR M ET AL: "Mining the plasma proteome for cancer biomarkers" NATURE (LONDON), vol. 452, no. 7187, April 2008 (2008-04), pages 571-579, XP002596847 ISSN: 0028-0836
- SCANLAN MATTHEW J ET AL: "Cancer-related serological recognition of human colon cancer: Identification of potential diagnostic and immunotherapeutic targets" CANCER RESEARCH, AMERICAN ASSOCIATION FOR CANCER REREARCH, US, vol. 62, no. 14, 15 July 2002 (2002-07-15), pages 4041-4047, XP002519268 ISSN: 0008-5472
- LINE AIJA ET AL: "Characterisation of tumour-associated antigens in colon cancer." CANCER IMMUNOLOGY IMMUNOTHERAPY, vol. 51, no. 10, November 2002 (2002-11), pages 574-582, XP002596848 ISSN: 0340-7004
- RAN YULIANG ET AL: "Profiling tumor-associated autoantibodies for the detection of colon cancer." CLINICAL CANCER RESEARCH, vol. 14, no. 9, 1 May 2008 (2008-05-01), pages 2696-2700, XP002596849 ISSN: 1078-0432
- EMADUDDIN MUHAMMAD ET AL: "Cell growth, global phosphotyrosine elevation, and c-Met phosphorylation through Src family kinases in colorectal cancer cells" PROCEEDINGS OF THE NATIONAL ACADEMY OF SCIENCES OF THE UNITED STATES OF AMERICA, vol. 105, no. 7, February 2008 (2008-02), pages 2358-2362, XP002596850 ISSN: 0027-8424
- PEI HAIPING ET AL: "Proteome analysis and tissue microarray for profiling protein markers associated with lymph node metastasis in colorectal cancer" JOURNAL OF PROTEOME RESEARCH, vol. 6, no. 7, 2007, pages 2495-2501, XP002596851 ISSN: 1535-3893

### LISTA DE SECUENCIAS

<110> CENTRO NACIONAL DE INVESTIGACIONES ONCOLÓGICAS CONSEJO SUPERIOR DE INVESTIGACIONES CIENTIFICAS (CSIC)
<120> METHODS FOR THE DIAGNOSIS OR PROGNOSIS OF THE COLORECTAL CANCER
<130> P5754PC00
<150> ESP200930203
   <151> 2009-05-25
<160> 6
<170> PatentIn version 3.5
<210> 1
   <211> 313
   <212> PRT
   <213> Homo sapiens
<400> 1
   Met Leu Leu Ser Lys Ile Asn Ser Leu Ala His Leu Arg Ala Ala Pro
   1               5                   10                  15
   Cys Asn Asp Leu His Ala Thr Lys Leu Ala Pro Gly Lys Glu Lys Glu
               20                  25                  30
   Pro Leu Glu Ser Gln Tyr Gln Val Gly Pro Leu Leu Gly Ser Gly Gly
           35                  40                  45
   Phe Gly Ser Val Tyr Ser Gly Ile Arg Val Ser Asp Asn Leu Pro Val
       50                  55                  60
   Ala Ile Lys His Val Glu Lys Asp Arg Ile Ser Asp Trp Gly Glu Leu
   65                  70                  75                  80
   Pro Asn Gly Thr Arg Val Pro Met Glu Val Val Leu Leu Lys Lys Val
                   85                  90                  95
   Ser Ser Gly Phe Ser Gly Val Ile Arg Leu Leu Asp Trp Phe Glu Arg
               100                 105                 110
   Pro Asp Ser Phe Val Leu Ile Leu Glu Arg Pro Glu Pro Val Gln Asp
           115                 120                 125
   Leu Phe Asp Phe Ile Thr Glu Arg Gly Ala Leu Gln Glu Glu Leu Ala
       130                 135                 140
   Arg Ser Phe Phe Trp Gln Val Leu Glu Ala Val Arg His Cys His Asn
   145                 150                 155                 160
   Cys Gly Val Leu His Arg Asp Ile Lys Asp Glu Asn Ile Leu Ile Asp
                   165                 170                 175
   Leu Asn Arg Gly Glu Leu Lys Leu Ile Asp Phe Gly Ser Gly Ala Leu
               180                 185                 190
   Leu Lys Asp Thr Val Tyr Thr Asp Phe Asp Gly Thr Arg Val Tyr Ser
           195                 200                 205
   Pro Pro Glu Trp Ile Arg Tyr His Arg Tyr His Gly Arg Ser Ala Ala
       210                 215                 220
   Val Trp Ser Leu Gly Ile Leu Leu Tyr Asp Met Val Cys Gly Asp Ile
   225                 230                 235                 240
   Pro Phe Glu His Asp Glu Glu Ile Ile Arg Gly Gln Val Phe Phe Arg
                   245                 250                 255
   Gln Arg Val Ser Ser Glu Cys Gln His Leu Ile Arg Trp Cys Leu Ala
               260                 265                 270
   Leu Arg Pro Ser Asp Arg Pro Thr Phe Glu Glu Ile Gln Asn His Pro
           275                 280                 285
   Trp Met Gln Asp Val Leu Leu Pro Gln Glu Thr Ala Glu Ile His Leu
       290                 295                 300
   His Ser Leu Ser Pro Gly Pro Ser Lys
   305                 310
<210> 2
   <211> 536
   <212> PRT
   <213> Homo sapiens
<400> 2
<210> 3
   <211> 382
   <212> PRT
   <213> Homo sapiens
<400> 3
   Met Asp Gly Glu Thr Ala Glu Glu Gln Gly Gly Pro Val Pro Pro Pro
   1               5                   10                  15
   Val Ala Pro Gly Gly Pro Gly Leu Gly Gly Ala Pro Gly Gly Arg Arg
               20                  25                  30
   Glu Pro Lys Lys Tyr Ala Val Thr Asp Asp Tyr Gln Leu Ser Lys Gln
           35                  40                  45
   Val Leu Gly Leu Gly Val Asn Gly Lys Val Leu Glu Cys Phe His Arg
       50                  55                  60
   Arg Thr Gly Gln Lys Cys Ala Leu Lys Leu Leu Tyr Asp Ser Pro Lys
   65                  70                  75                  80
   Ala Arg Gln Glu Val Asp His His Trp Gln Ala Ser Gly Gly Pro His
                   85                  90                  95
   Ile Val Cys Ile Leu Asp Val Tyr Glu Asn Met His His Gly Lys Arg
               100                 105                 110
   Cys Leu Leu Ile Ile Met Glu Cys Met Glu Gly Gly Glu Leu Phe Ser
           115                 120                 125
   Arg Ile Gln Glu Arg Gly Asp Gln Ala Phe Thr Glu Arg Glu Ala Ala
       130                 135                 140
   Glu Ile Met Arg Asp Ile Gly Thr Ala Ile Gln Phe Leu His Ser His
   145                 150                 155                 160
   Asn Ile Ala His Arg Asp Val Lys Pro Glu Asn Leu Leu Tyr Thr Ser
                   165                 170                 175
   Lys Glu Lys Asp Ala Val Leu Lys Leu Thr Asp Phe Gly Phe Ala Lys
   180 185 190
   Glu Thr Thr Gln Asn Ala Leu Gln Thr Pro Cys Tyr Thr Pro Tyr Tyr
   195 200 205
   Val Ala Pro Glu Val Leu Gly Pro Glu Lys Tyr Asp Lys Ser Cys Asp
   210 215 220
   Met Trp Ser Leu Gly Val Ile Met Tyr Ile Leu Leu Cys Gly Phe Pro
   225 230 235 240
   Pro Phe Tyr Ser Asn Thr Gly Gln Ala Ile Ser Pro Gly Met Lys Arg
   245 250 255
   Arg Ile Arg Leu Gly Gln Tyr Gly Phe Pro Asn Pro Glu Trp Ser Glu
   260 265 270
   Val Ser Glu Asp Ala Lys Gln Leu Ile Arg Leu Leu Leu Lys Thr Asp
   275 280 285
   Pro Thr Glu Arg Leu Thr Ile Thr Gln Phe Met Asn His Pro Trp Ile
   290 295 300
   Asn Gln Ser Met Val Val Pro Gln Thr Pro Leu His Thr Ala Arg Val
   305 310 315 320
   Leu Gln Glu Asp Lys Asp His Trp Asp Glu Val Lys Glu Glu Met Thr
   325 330 335
   Ser Ala Leu Ala Thr Met Arg Val Asp Tyr Asp Gln Val Lys Ile Lys
   340 345 350
   Asp Leu Lys Thr Ser Asn Asn Arg Leu Leu Asn Lys Arg Arg Lys Lys
   355 360 365
   Gln Ala Gly Ser Ser Ser Ala Ser Gln Gly Cys Asn Asn Gln
   370 375 380
<210> 4
   <211> 802
   <212> PRT
   <213> Homo sapiens
<400> 4
   Met Arg Leu Leu Leu Ala Leu Leu Gly Val Leu Leu Ser Val Pro Gly
   1               5                   10                  15
   Pro Pro Val Leu Ser Leu Glu Ala Ser Glu Glu Val Glu Leu Glu Pro
               20                  25                  30
   Cys Leu Ala Pro Ser Leu Glu Gln Gln Glu Gln Glu Leu Thr Val Ala
           35                  40                  45
   Leu Gly Gln Pro Val Arg Leu Cys Cys Gly Arg Ala Glu Arg Gly Gly
       50                  55                  60
   His Trp Tyr Lys Glu Gly Ser Arg Leu Ala Pro Ala Gly Arg Val Arg
   65                  70                  75                  80
   Gly Trp Arg Gly Arg Leu Glu Ile Ala Ser Phe Leu Pro Glu Asp Ala
                   85                  90                  95
   Gly Arg Tyr Leu Cys Leu Ala Arg Gly Ser Met Ile Val Leu Gln Asn
               100                 105                 110
   Leu Thr Leu Ile Thr Gly Asp Ser Leu Thr Ser Ser Asn Asp Asp Glu
           115                 120                 125
   Asp Pro Lys Ser His Arg Asp Pro Ser Asn Arg His Ser Tyr Pro Gln
       130                 135                 140
   Gln Ala Pro Tyr Trp Thr His Pro Gln Arg Met Glu Lys Lys Leu His
   145                 150                 155                 160
   Ala Val Pro Ala Gly Asn Thr Val Lys Phe Arg Cys Pro Ala Ala Gly
                   165                 170                 175
   Asn Pro Thr Pro Thr Ile Arg Trp Leu Lys Asp Gly Gln Ala Phe His
               180                 185                 190
   Gly Glu Asn Arg Ile Gly Gly Ile Arg Leu Arg His Gln His Trp Ser
           195                 200                 205
   Leu Val Met Glu Ser Val Val Pro Ser Asp Arg Gly Thr Tyr Thr Cys
       210                 215                 220
   Leu Val Glu Asn Ala Val Gly Ser Ile Arg Tyr Asn Tyr Leu Leu Asp
   225 230 235 240
   Val Leu Glu Arg Ser Pro His Arg Pro Ile Leu Gln Ala Gly Leu Pro
   245 250 255
   Ala Asn Thr Thr Ala Val Val Gly Ser Asp Val Glu Leu Leu Cys Lys
   260 265 270
   Val Tyr Ser Asp Ala Gln Pro His Ile Gln Trp Leu Lys His Ile Val
   275 280 285
   Ile Asn Gly Ser Ser Phe Gly Ala Asp Gly Phe Pro Tyr Val Gln Val
   290 295 300
   Leu Lys Thr Ala Asp Ile Asn Ser Ser Glu Val Glu Val Leu Tyr Leu
   305 310 315 320
   Arg Asn Val Ser Ala Glu Asp Ala Gly Glu Tyr Thr Cys Leu Ala Gly
   325 330 335
   Asn Ser Ile Gly Leu Ser Tyr Gln Ser Ala Trp Leu Thr Val Leu Pro
   340 345 350
   Glu Glu Asp Pro Thr Trp Thr Ala Ala Ala Pro Glu Ala Arg Tyr Thr
   355 360 365
   Asp Ile Ile Leu Tyr Ala Ser Gly Ser Leu Ala Leu Ala Val Leu Leu
   370 375 380
   Leu Leu Ala Gly Leu Tyr Arg Gly Gln Ala Leu His Gly Arg His Pro
   385 390 395 400
   Arg Pro Pro Ala Thr Val Gln Lys Leu Ser Arg Phe Pro Leu Ala Arg
   405 410 415
   Gln Phe Ser Leu Glu Ser Gly Ser Ser Gly Lys Ser Ser Ser Ser Leu
   420 425 430
   Val Arg Gly Val Arg Leu Ser Ser Ser Gly Pro Ala Leu Leu Ala Gly
   435 440 445
   Leu Val Ser Leu Asp Leu Pro Leu Asp Pro Leu Trp Glu Phe Pro Arg
   450 455 460
   Asp Arg Leu Val Leu Gly Lys Pro Leu Gly Glu Gly Cys Phe Gly Gln
   465                 470                 475                 480
   Val Val Arg Ala Glu Ala Phe Gly Met Asp Pro Ala Arg Pro Asp Gln
                   485                 490                 495
   Ala Ser Thr Val Ala Val Lys Met Leu Lys Asp Asn Ala Ser Asp Lys
               500                 505                 510
   Asp Leu Ala Asp Leu Val Ser Glu Met Glu Val Met Lys Leu Ile Gly
           515                 520                 525
   Arg His Lys Asn Ile Ile Asn Leu Leu Gly Val Cys Thr Gln Glu Gly
       530                 535                 540
   Pro Leu Tyr Val Ile Val Glu Cys Ala Ala Lys Gly Asn Leu Arg Glu
   545                 550                 555                 560
   Phe Leu Arg Ala Arg Arg Pro Pro Gly Pro Asp Leu Ser Pro Asp Gly
                   565                 570                 575
   Pro Arg Ser Ser Glu Gly Pro Leu Ser Phe Pro Val Leu Val Ser Cys
               580                 585                 590
   Ala Tyr Gln Val Ala Arg Gly Met Gln Tyr Leu Glu Ser Arg Lys Cys
           595                 600                 605
   Ile His Arg Asp Leu Ala Ala Arg Asn Val Leu Val Thr Glu Asp Asn
       610                 615                 620
   Val Met Lys Ile Ala Asp Phe Gly Leu Ala Arg Gly Val His His Ile
   625                 630                 635                 640
   Asp Tyr Tyr Lys Lys Thr Ser Asn Gly Arg Leu Pro Val Lys Trp Met
                   645                 650                 655
   Ala Pro Glu Ala Leu Phe Asp Arg Val Tyr Thr His Gln Ser Asp Val
               660                 665                 670
   Trp Ser Phe Gly Ile Leu Leu Trp Glu Ile Phe Thr Leu Gly Gly Ser
           675                 680                 685
   Pro Tyr Pro Gly Ile Pro Val Glu Glu Leu Phe Ser Leu Leu Arg Glu
       690                 695                 700
   Gly His Arg Met Asp Arg Pro Pro His Cys Pro Pro Glu Leu Tyr Gly
   705 710 715 720
   Leu Met Arg Glu Cys Trp His Ala Ala Pro Ser Gln Arg Pro Thr Phe
   725 730 735
   Lys Gln Leu Val Glu Ala Leu Asp Lys Val Leu Leu Ala Val Ser Glu
   740 745 750
   Glu Tyr Leu Asp Leu Arg Leu Thr Phe Gly Pro Tyr Ser Pro Ser Gly
   755 760 765
   Gly Asp Ala Ser Ser Thr Cys Ser Ser Ser Asp Ser Val Phe Ser His
   770 775 780
   Asp Pro Leu Pro Leu Gly Ser Ser Ser Phe Pro Phe Gly Ser Gly Val
   785 790 795 800
   Gln Thr
<210> 5
   <211> 487
   <212> PRT
   <213> Homo sapiens
<400> 5
   Met Glu Thr Val Gln Leu Arg Asn Pro Pro Arg Arg Gln Leu Lys Lys
   1 5 10 15
   Leu Asp Glu Asp Ser Leu Thr Lys Gln Pro Glu Glu Val Phe Asp Val
   20 25 30
   Leu Glu Lys Leu Gly Glu Gly Ser Tyr Gly Ser Val Tyr Lys Ala Ile
   35 40 45
   His Lys Glu Thr Gly Gln Ile Val Ala Ile Lys Gln Val Pro Val Glu
   50 55 60
   Ser Asp Leu Gln Glu Ile Ile Lys Glu Ile Ser Ile Met Gln Gln Cys
   65 70 75 80
   Asp Ser Pro His Val Val Lys Tyr Tyr Gly Ser Tyr Phe Lys Asn Thr
   85 90 95
   Asp Leu Trp Ile Val Met Glu Tyr Cys Gly Ala Gly Ser Val Ser Asp
   100 105 110
   Ile Ile Arg Leu Arg Asn Lys Thr Leu Thr Glu Asp Glu Ile Ala Thr
   115 120 125
   Ile Leu Gln Ser Thr Leu Lys Gly Leu Glu Tyr Leu His Phe Met Arg
   130 135 140
   Lys Ile His Arg Asp Ile Lys Ala Gly Asn Ile Leu Leu Asn Thr Glu
   145 150 155 160
   Gly His Ala Lys Leu Ala Asp Phe Gly Val Ala Gly Gln Leu Thr Asp
   165 170 175
   Thr Met Ala Lys Arg Asn Thr Val Ile Gly Thr Pro Phe Trp Met Ala
   180 185 190
   Pro Glu Val Ile Gln Glu Ile Gly Tyr Asn Cys Val Ala Asp Ile Trp
   195 200 205
   Ser Leu Gly Ile Thr Ala Ile Glu Met Ala Glu Gly Lys Pro Pro Tyr
   210 215 220
   Ala Asp Ile His Pro Met Arg Ala Ile Phe Met Ile Pro Thr Asn Pro
   225 230 235 240
   Pro Pro Thr Phe Arg Lys Pro Glu Leu Trp Ser Asp Asn Phe Thr Asp
   245 250 255
   Phe Val Lys Gln Cys Leu Val Lys Ser Pro Glu Gln Arg Ala Thr Ala
   260 265 270
   Thr Gln Leu Leu Gln His Pro Phe Val Arg Ser Ala Lys Gly Val Ser
   275 280 285
   Ile Leu Arg Asp Leu Ile Asn Glu Ala Met Asp Val Lys Leu Lys Arg
   290 295 300
   Gln Glu Ser Gln Gln Arg Glu Val Asp Gln Asp Asp Glu Glu Asn Ser
   305 310 315 320
   Glu Glu Asp Glu Met Asp Ser Gly Thr Met Val Arg Ala Val Gly Asp
   325 330 335
   Glu Met Gly Thr Val Arg Val Ala Ser Thr Met Thr Asp Gly Ala Asn
   340 345 350
   Thr Met Ile Glu His Asp Asp Thr Leu Pro Ser Gln Leu Gly Thr Met
   355 360 365
   Val Ile Asn Ala Glu Asp Glu Glu Glu Glu Gly Thr Met Lys Arg Arg
   370 375 380
   Asp Glu Thr Met Gln Pro Ala Lys Pro Ser Phe Leu Glu Tyr Phe Glu
   385 390 395 400
   Gln Lys Glu Lys Glu Asn Gln Ile Asn Ser Phe Gly Lys Ser Val Pro
   405 410 415
   Gly Pro Leu Lys Asn Ser Ser Asp Trp Lys Ile Pro Gln Asp Gly Asp
   420 425 430
   Tyr Glu Phe Leu Lys Ser Trp Thr Val Glu Asp Leu Gln Lys Arg Leu
   435 440 445
   Leu Ala Leu Asp Pro Met Met Glu Gln Glu Ile Glu Glu Ile Arg Gln
   450 455 460
   Lys Tyr Gln Ser Lys Arg Gln Pro Ile Leu Asp Ala Ile Glu Ala Lys
   465 470 475 480
   Lys Arg Arg Gln Gln Asn Phe
   485
<210> 6
   <211> 512
   <212> PRT
   <213> Homo sapiens
<400> 6

## Description

### Field of the Invention

The present invention is comprised within the field of biomedicine. It specifically relates to obtaining data useful for the diagnosis, prognosis or monitoring the progress of colorectal cancer (CRC), as well as to methods for the diagnosis or prognosis of CRC based on autoantibodies against proteins or on the expression products of the genes encoding said proteins, as well as to a method for diagnosing metastases in patients with CRC. The invention also relates to a kit suitable for putting said methods into practice.

### Background of the Invention

Colorectal cancer (CRC) is the second most prevalent cancer in the Western world. The disease develops over decades and involves multiple genetic events. Despite the fact that CRC is one of the best characterized solid tumors from the genetic point of view, it continues to be one of the main causes of death in developed countries because of the late diagnosis of patients due, among other reasons, to the fact that some diagnostic tests, such as colonoscopy, are performed too late.

Today there are few proteins that have been described as effective biomarkers of CRC, which include the carcinoembryonic antigen (CEA), CA19.9 and CA125 (Crawford et al. 2003. Journal of Surgical Oncology 84 (4), 239-248; Duffy et al. 2007 Eur J Cancer 43 (9), 1348-1360) and they are not specific enough to perform clinical screenings with a view to detect CRC (Locker et al. 25 2006. J Clin Oncol 24 (33), 5313-5327).

Proteomic analyses are being actively used for identifying new biomarkers. In different earlier proteomic studies, differentially expressed proteins in CRC tissue have been identified by means of using antibody microarrays and 2D-DIGE (two-dimensional difference gel electrophoresis), including isoforms and post-translational modifications responsible for modifications in signaling pathways (Alfonso et al. 2005. Proteomics 5(10), 2602-2611; Kopf et al. 2005. Proteomics 5(9), 2412-2416; Madoz-Gurpide et al. 2007. Mol Cell Proteomics 6 (12), 2150-2164; Alfonso et al. 2008. Journal of Proteome Research 7 (10), 4247-4255). These two approaches allowed identifying a wide collection of potential tumor markers of CRC tissue which are currently under research.

However, the implementation of non-invasive and simpler diagnostic methods which allow the early detection of CRC must be based on identifying proteins or antibodies detectable in serum or plasma (Hanash et al. 2008. Nature 452 (7187), 571-579; Hudson et al. 2007. Proceedings of the National Academy of Sciences of the United States of America 104 (44), 17494-17499).

The existence of an immune response to cancer and tumors in humans has been demonstrated by the presence of autoantibodies in the serum from patients with cancer. Thus, different human proteins (autoantigens) can be affected before or during the formation of the tumor, being able to produce an immune response once released (Hudson et al. 2007. Proceedings of the National Academy of Sciences of the United States of America 104 (44), 17494-17499; Wang et al. 2005. The New England Journal of Medicine 353 (12), 1224-1235; Sreekumar et al. 2004. J Natl Cancer Inst 96 (11), 834-843). Said autoantibodies can be detected in early stages of the disease and even before the cancer can be detected by means of other techniques, indicating their high potential as biomarkers of the disease. These tumor proteins can either be affected by isolated mutations, can have anomalous folding, overexpression, aberrant glycosylation, can be truncated or undergo aberrant degradation as in the case of p53, HER2, NY-ESO1 or MUC1, respectively (Chen et al. 1997. Proceedings of the National Academy of Sciences of the United States of America 94 (5), 1914-1918; Schubert et al. 2000. Nature 404 (6779), 770-774; Ulanet et al. 2003. Proceedings of the National Academy of Sciences of the United States of America 100 (21), 12361-12366). In fact, tumor-associated autoantigens (TAAs) have been characterized in CRC using other approaches (Scanlan et al. 1998. International Journal of Cancer 76 (5), 652-658). Nevertheless, the diagnostic validity of the autoantibodies associated with CRC identified until now still requires an independent validation for their generalized use in the diagnosis/prognosis of CRC.

Therefore, there is a need for biomarkers which allow the diagnosis of CRC, its classification in the different stages of tumor progression, the prognosis of the progress of the disease, the evaluation of its response to a determined treatment and the detection of the recurrence or the dissemination of CRC, by means of a simple, effective and non-invasive method.

On the other hand, the use of Pim1 as a marker, alone or in combination with other markers, of prostate cancer is described by WO 2006/056766 and WO2008/061104.

### Summary of the Invention

Several assays performed by the inventors have allowed identifying that autoantibodies to Pim1, SRC, MAPKAPK3, FGFR4, STK4 and ACVR2B proteins, as well as the expression products of the genes encoding said proteins, can be used as biomarkers of colorectal cancer (CRC). Furthermore, they have also been able to identify that autoantibodies to the proteins mentioned in Tables 2 and 3 (see below) can be used as biomarkers of lung or liver metastasis in patients with CRC.

Therefore, the present description describes a method for the detection of autoantibodies to said proteins (Pim1, SRC, MAPKAPK3, FGFR4, STK4 and ACVR2B) potentially useful as markers of CRC as well as to methods of obtaining data, methods for the diagnosis, prognosis or tracking of the progress of CRC, and methods for the diagnosis or prognosis of lung or liver metastasis in patients with CRC, and to a kit suitable for putting said methods and its applications into practice.

The present invention therefore provides a response to the need for biomarkers which allow the diagnosis of CRC, its classification in the different stages of tumor progression, the prognosis of the progress of the disease, the evaluation of its response to a determined treatment and the detection of the recurrence or the dissemination (metastasis) of CRC, by means of a simple, effective and non-invasive method.

Blood is usually the optimal biological fluid used in non-invasive methods for massive screening for the purpose of diagnosing large populations of subjects. On one hand, serum and plasma are easy to obtain, and on the other hand, blood circulation facilitates the contact of the blood with all the tissues of the human body, including the contact with tumor tissue and its representative antigens in the case of patients with cancer. The release of these TAAs probably occurs at a very low concentration in plasma and probably experience proteolysis in a short time period. In contrast, antibodies are very stable molecules which have been used for years in different clinical immunoassays, which facilitates normalizing the assays. The use of the autoantibodies is also beneficial in the sense that the immune system amplifies the response facilitating its identification and quantification.

In the present invention, the serum from patients with CRC and sera of subjects without CRC (control sera or reference sera) have been examined for the purpose of identifying a signature (fingerprint) of autoantibodies produced by patients suffering CRC in response to said CRC and their respective reactive proteins. To that end, sera from patients with CRC and control sera were tested using high-density protein microarrays. Protein microarrays offer a series of advantages with respect to other approaches used for identifying TAAs: i) the proteins printed in the array are known beforehand, preventing a subsequent identification and eliminating the possible selection of mimotopes, and ii) there is no predisposition to select any protein because they are all printed at a similar concentration. This combination of factors results in a high sensitivity for identifying biomarkers.

The antibody signature identified allowed differentiating between sera from patients with CRC and control subjects. A total of 43 proteins were identified which presented a differential expression in sera from patients with CRC and in control sera (p<0.04) in the protein array. The combination of the 6 best immunoreactive antigens: Pim1, MAPKAPK3, STK4, SRC, FGFR4 and ACVR2B was capable of detecting CRC with 100% specificity and sensitivity using the data obtained from the protein array. The increased or decreased levels of expression of said proteins were confirmed by means of membrane immunodetection and immunohistochemistry using both cell lines and tumor tissue of CRC as tissue microarrays.

The combination formed by the purified proteins Pim1, MAPKAPK3 and ACVR2B was tested by means of an ELISA using sera from patients with CRC and control sera. The ELISA allowed distinguishing between sera from patients with CRC and control sera with a specificity and sensitivity of 73.9% and 83.3%, respectively (AUC=0.86).

These studies allowed determining the presence of a specific antibody signature of CRC showing the presence of new specific biomarkers of the disease with potential for diagnosing CRC using sera from patients with CRC with greater specificity and sensitivity than with the biomarkers of CRC described up until now.

The ELISA technique is much more sensitive than other techniques such as membrane immunodetection or immunohistochemistry. This high sensitivity could explain why the prevalence of autoantibodies in patients with cancer is much greater than in other previous studies, in addition to the detection of reactivity in control subjects. In fact, the diagnostic assay could be based on autoantibodies with high prevalence given that no autoantigens with exclusive immunoreactivity were found in the serum from patients with CRC.

Therefore, it is described a method for the detection of an autoantibody to a protein which comprises: a) contacting a biological sample with said protein or with a fragment thereof susceptible of being recognized by said autoantibody; and b) detecting the formation of an autoantibody-protein, or fragment thereof, complex susceptible of being recognized by said autoantibody; wherein said protein is selected from the group consisting of the Pim1, SRC, MAPKAPK3, FGFR4, STK4 and ACVR2B proteins and combinations thereof.

It is also described a method of obtaining data in a biological sample from a subject which comprises detecting at least one autoantibody to a protein, wherein said autoantibody is selected from the group consisting of an autoantibody to the Pim1 protein, an autoantibody to the SRC protein, an autoantibody to the MAPKAPK3 protein, an autoantibody to the FGFR4 protein, an autoantibody to the STK4 protein, and an autoantibody to the ACVR2B protein, and, if desired, determining the level of said autoantibody in said sample.

In an aspect, the invention relates to a method of obtaining data in a biological sample from a subject which comprises detecting the expression product of the Pim1 gene and the expression product of at least one gene selected from the group consisting of the *SRC, MAPKAPK3, FGFR4, STK4* and *ACVR2B* genes, and, if desired, quantifying the level of expression of said expression product of said genes in said sample wherein said sample comprises colorectal cancer (CRC) tumor cells.

In another aspect, the invention relates to a method for diagnosing if a subject suffers colorectal cancer (CRC), which comprises comparing the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from said subject, with the reference level for said autoantibodies, wherein if the level of said autoantibody to the Pim1 protein in said sample is greater than the corresponding reference level for said autoantibody, and wherein if the level, of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said subject is diagnosed with CRC.

In another aspect, the invention relates to a method for diagnosing if a subject suffers colorectal cancer (CRC), which comprises comparing the level of expression of an expression product of the Pim1 gene and the level of expression of at least one expression product a gene, wherein said gene is selected from the group consisting of the *SRC, MAPKAPK3, FGFR4, STK4* and *ACVR2B* genes, in a sample from said subject, with the reference level for said expression product of said genes, wherein if the level of said expression product of the *Pim1* gene is greater than the corresponding reference level for said expression product of said gene and the level, of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, said subject is diagnosed with CRC.

In another aspect, the invention relates to a method for evaluating the prognosis or tracking of the progress of a patient suffering colorectal cancer (CRC), which comprises comparing the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from said patient suffering CRC, with the reference level for said autoantibody, wherein if the level of said autoantibody to the Pim1 protein is greater than the corresponding reference level for said autoantibody, and wherein if the level, of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said patient suffers a CRC with a poor prognosis or presents a CRC with an unfavorable progress.

In another aspect, the invention relates to a method for evaluating the prognosis or tracking of the progress of a patient suffering colorectal cancer (CRC), which comprises comparing the level of expression of an expression product of the Pim1 gene and the level of expression of at least one expression product of a gene, wherein said gene is selected from the group consisting of the *SRC, MAPKAPK3, FGFR4, STK4* and *ACVR2B* genes, in a sample from said patient suffering CRC, with the reference level for said expression product of said genes, wherein if the level of said expression product of the *Pim1* gene is greater than the corresponding reference level for said expression product of said gene and the level, of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, said patient suffers a CRC with a poor prognosis or presents a CRC with an unfavorable progress.

The description also describes a method for diagnosing lung metastasis in a patient suffering colorectal cancer (CRC), which comprises comparing the level of at least one autoantibody to a protein in a biological sample from said patient, wherein said protein is a protein selected from the group of proteins mentioned in Table 2, with the reference level for said autoantibody, wherein if the level of autoantibody to said protein in said biological sample from said patient is greater than the reference level for said autoantibody, the CRC patient presents lung metastasis.

The description also describes a method for diagnosing liver metastasis in a patient suffering colorectal cancer (CRC) which comprises comparing the level of at least one autoantibody to a protein in a biological sample from said patient, wherein said protein is a protein selected from the group of proteins mentioned in Table 3, with the reference level for said autoantibody, wherein if the level of autoantibody to said protein in said biological sample from said patient is greater than the reference level for said autoantibody, the CRC patient presents liver metastasis.

In another aspect, the invention relates to a kit comprising:
- the elements necessary for detecting at least one autoantibody to the Pim1 protein and an autoantibody selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, or alternatively
- the elements necessary for detecting an expression product of the Pim1 gene and at least one expression product of a gene selected from the group consisting of the *SRC, MAPKAPK3, FGFR4, STK4* and ACVR2B genes.

In another aspect, the invention relates to the use of said kit for diagnosing if a subject suffers CRC; or for evaluating the prognosis or tracking of the progress of a patient suffering CRC.

### Brief Description of the Drawings

The following figures form part of the present specification and are included to furthermore demonstrate certain aspects of the present invention. The invention can be better understood by means of reference to one or more of these figures combined with the detailed description of specific embodiments herein presented.

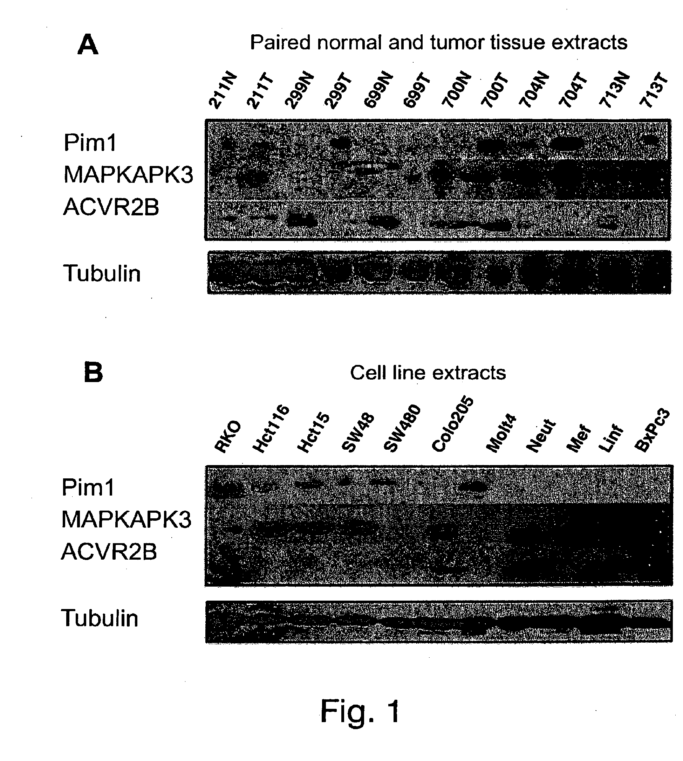

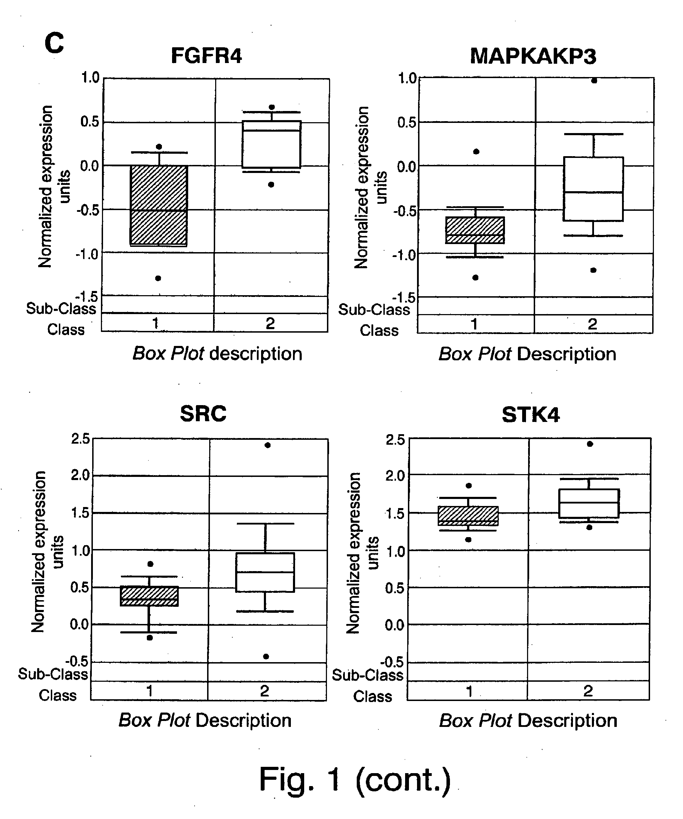

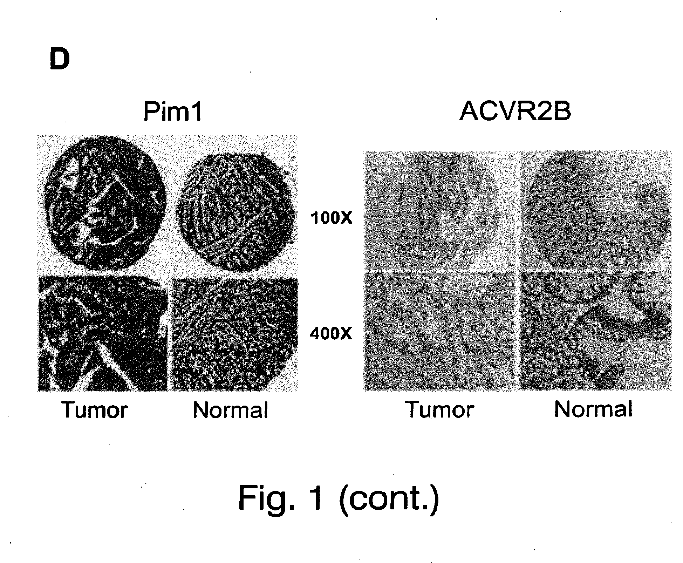

Figure 1 shows the analysis of the expression of Pim1, MAPKAPK3 and ACVR2B, in cell lines and tumor tissue. A, 50 µg of protein extract of paired normal (N) and tumor (T) tissues from 6 patients with CRC (Duke's A, B and C stages) were run separately in 10% SDS-PAGE gels and transferred to nitrocellulose membranes; the membrane immunodetections were performed with commercial antibodies obtained to Pim1, MAPKAPK3 and ACVR2B, using anti-tubulin as control of the assay. The signal was developed using ECL (Amersham) or SuperSignal Femto (Pierce). B, The membrane immunodetections were performed with commercial antibodies obtained to ACVR2B, Pim1 and MAPKAPK3 using anti-tubulin as control of the assay. 50 µg of cell extracts of 6 CRC cell lines (Rko, Hct116, SW48, SW480, Hct15, Colo205) and 5 cell lines of other diseases or normal cell lines were used as reference in the assay [(BxPc3 (pancreatic adenocarcinoma 25), Molt4 (Lymphoblastoid), Neut (Neutrophils), MEF (murine embryonic fibroblasts) and Linf (lymphocytes)] were run separately in 10% SDS-PAGE gels and transferred to nitrocellulose membranes. The signal was developed using ECL (Amersham) or SuperSignal Femto (Pierce). C, The relative levels of the expression of the genes *FGFR4* (Notterman, Alon, Sierk, and Levine, (2001) Cancer Res. 61, 3124-3130), *MAPKAPK3* (Ki, Jeung et al. 2007 Int. J. Cancer 121, 2005-2012), *SRC* (Ki, Jeung et al. 2007 Int. J. Cancer 121, 2005-2012) and *STK4* (Watanabe, Kobunai et al. 2006 Cancer Res. 66, 9804-9808) were evaluated using the public DNA microarray database Oncomine (www.oncomine.org). D, Analysis of the expression of Pim1 and ACVR2B in tissue using specific tissue microarrays (TMA) of CRC. The images were taken at different magnifications (100x and 400x). The expression of Pim1 was observed in epithelial cells surrounding the tumor tissue crypts with cytoplasmic staining. The staining of ACVR2B was mainly located at the membrane level of the epithelial cells in normal tissue with a clear reduction of its expression in tumor tissue.

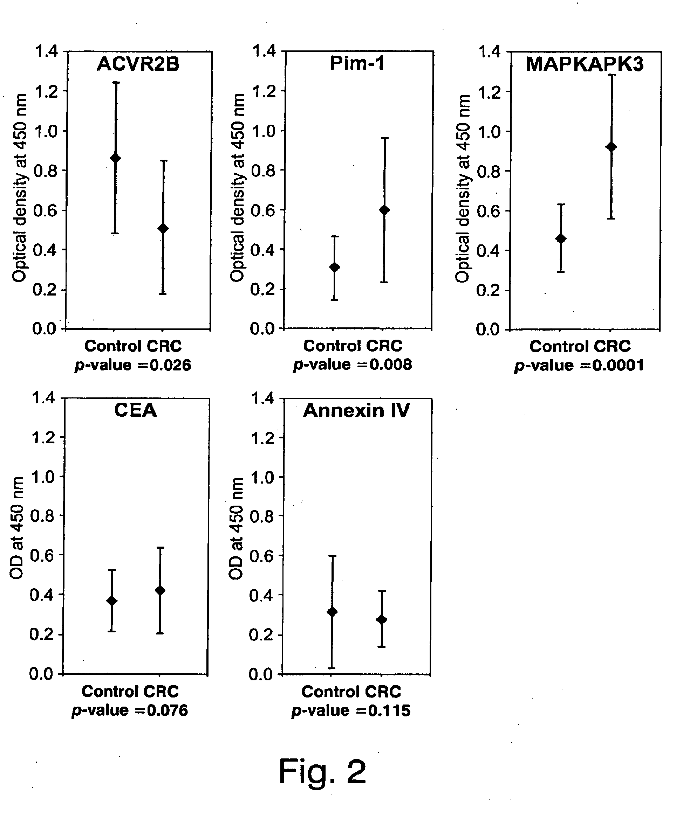

Figure 2 shows the verification of the selected TAAs (Pim1, MAPKAPK3 and ACVR2B) by means of ELISA. ELISA values of Pim1, MAPKAPK3 and ACVR2B using CEA and Annexin IV as controls. The error bars represent the standard deviation (SD) of the assay.

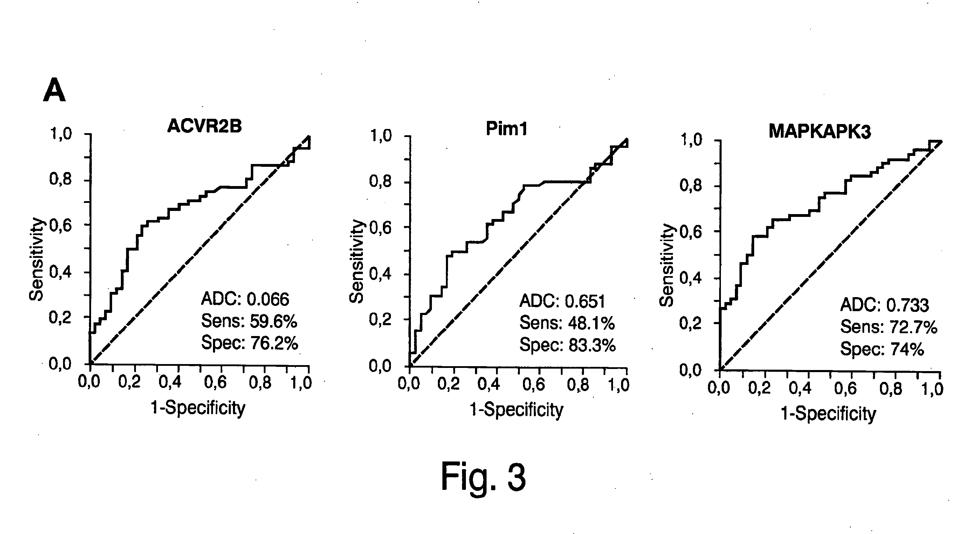

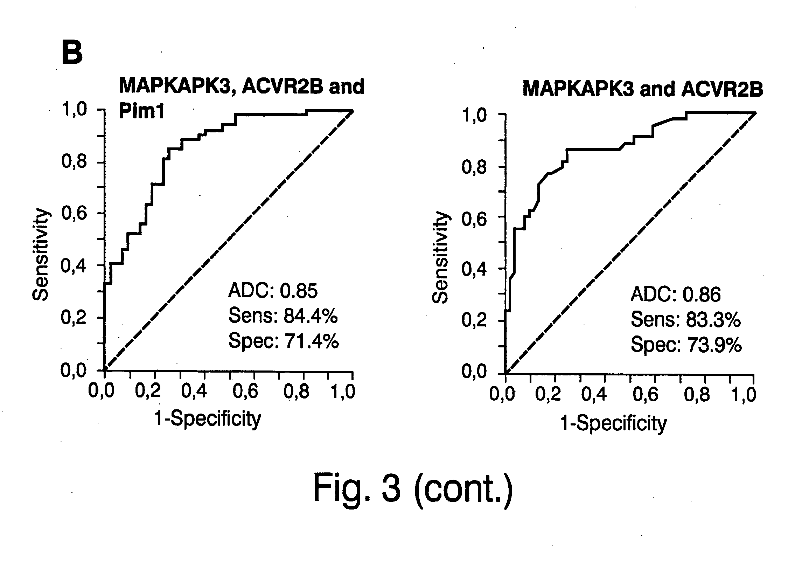

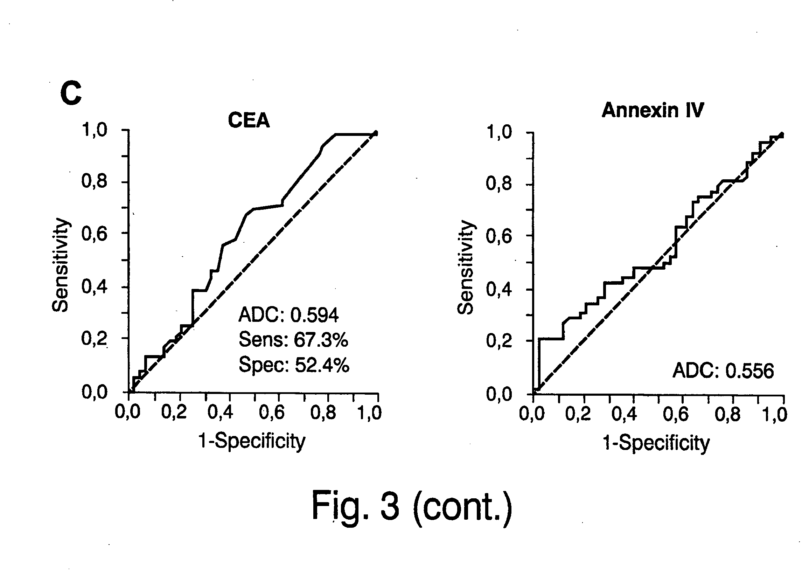

Figure 3 shows the ROC curves of the selected TAAs and consists of a graphic representation of the behavior of said TAAs . A, ROC curves using the ELISA values of ACVR2B, Pim1 and MAPKAPK3 individually. B, ROC curves using different combinations of the selected proteins [(MAPKAPK3 and ACVR2B and Pim 1) and (MAPKAPK3 and ACVR2B)]. C, ROC curves using the CEA and Annexin IV controls. [AUC: area under the curve; Sens: Sensitivity; Spec: Specificity].

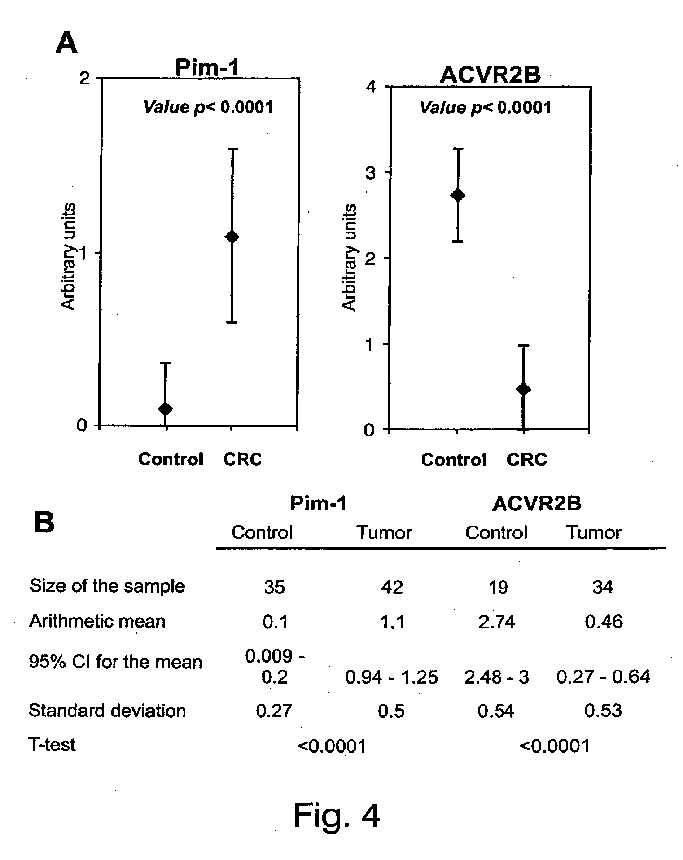

Figure 4 shows the immunohistochemical analysis of Pim1 and ACVR2B. A, Result of the immunohistochemical analysis of Pim1 and ACVR2B in CRC tissue and normal adjacent mucosa from 45 patients with CRC quantified by 2 independent researchers on different days, according to the following criteria: 0, without labeling; 1, weak labeling; 2, normal labeling; 3, strong labeling. The error bars represent the SD of each assay. B, statistical analysis of the results of the TMA. The size of the sample, the mean, the 95% CI for the mean, the standard deviation and the T-test are indicated.

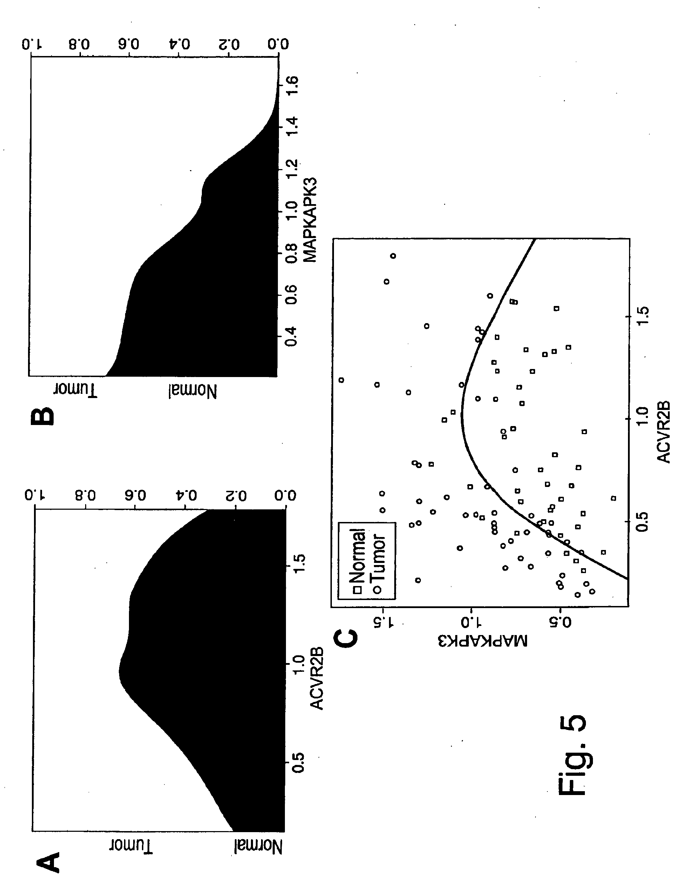

Figure 5 shows the correlation of autoantibodies to MAPKAPK3 and ACVR2B in serum from subjects with CRC. A and B, show the distribution of the signal intensity of both markers in serum from patients with CRC [CRC (tumor) serum] and in serum from healthy subjects [healthy control (normal) serum]. C, shows the graph of the signal in each serum (tumor and normal) of MAPKAPK3 and ACVR2B, where the absence of correlation between the signal of both markers can be seen. The higher the signal for ACVR2B the greater the possibility of belonging to the normal group; the opposite situation is observed for MAPKAPK3.

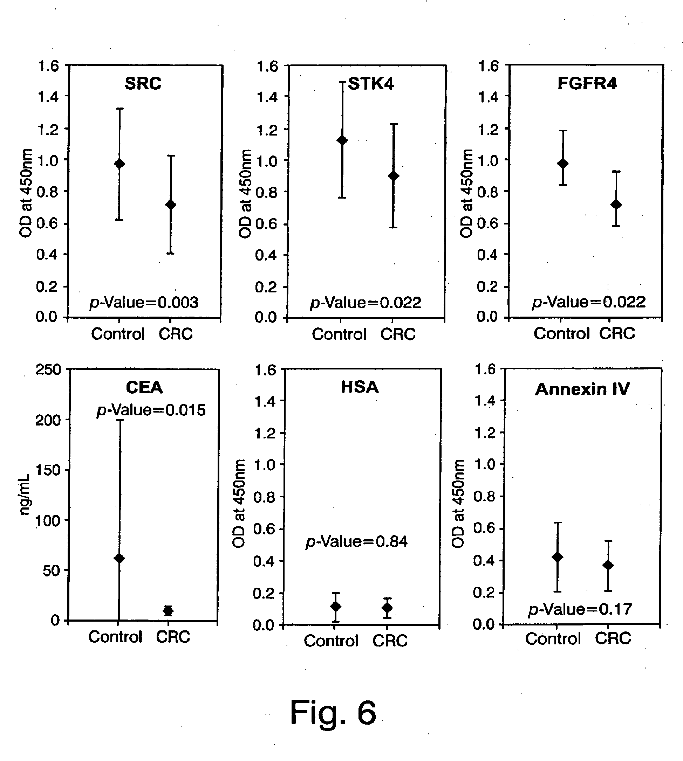

Figure 6 shows the ELISA analysis of samples of serum using an ELISA with the TAAs STK4 and FGFR4. A total of 94 samples of serum (52 from patients with CRC and 42 controls) were used for the implementation and the analysis based on an ELISA of recombinant TAAs. CEA and Annexin IV were used as controls. The results show the mean absorbance values obtained for SRC, STK4, FGFR4, HSA and Annexin IV in serum from reference populations (controls) and with CRC. The error bars represent the SD of the assay. The serum CEA concentration was determined using a kit specific for immunoassays (MP Biomedicals).

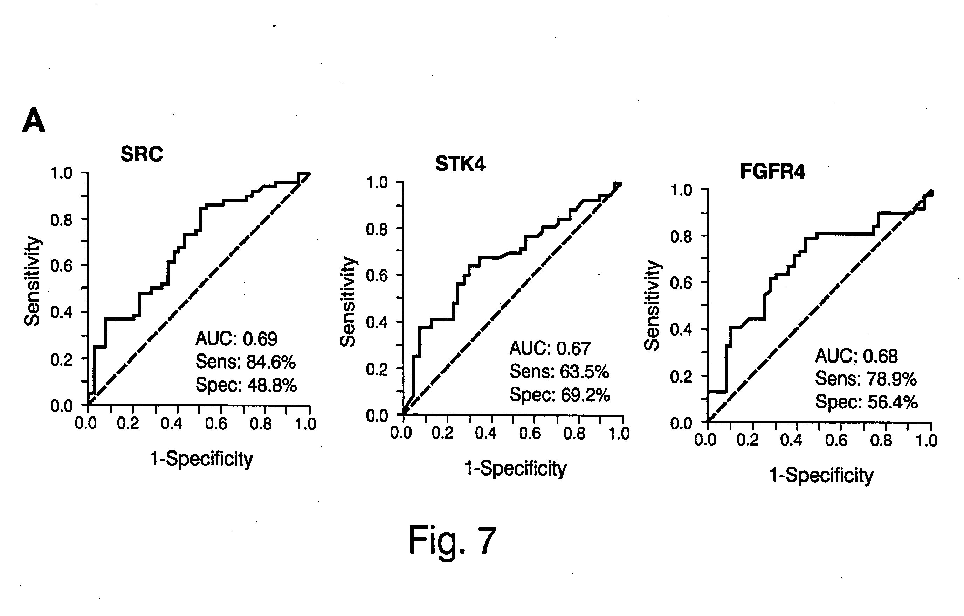

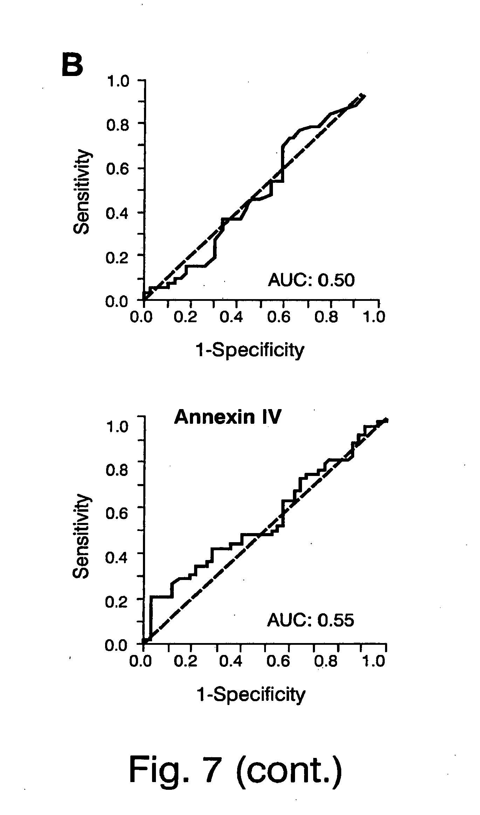

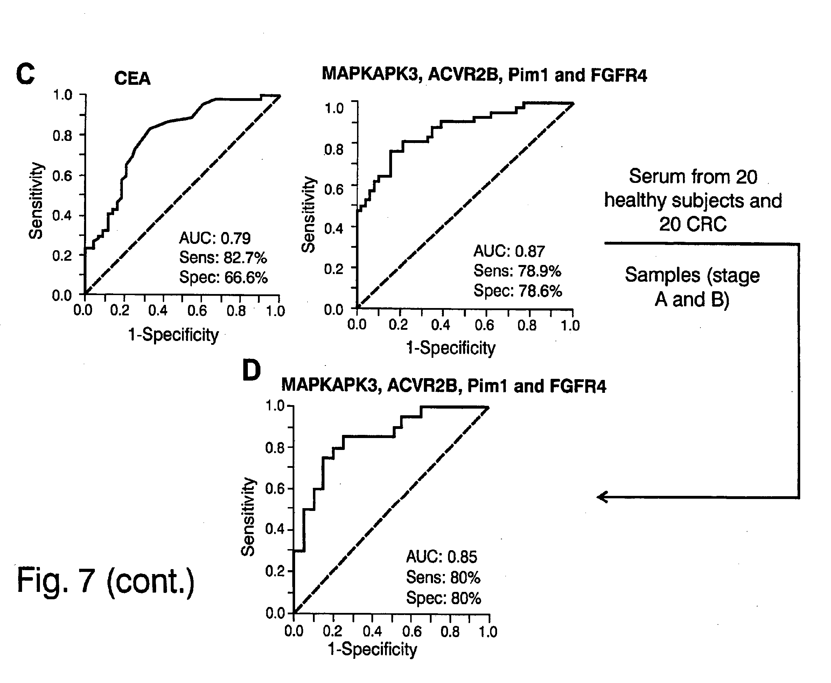

Figure 7 shows the validation of SRC, STK4 and FGFR4 as potential biomarkers in CRC. A, graph of SRC, STK4 and FGFR4 by discriminating between serum from patients with CRC and serum from reference subjects (controls) independently in a validation group of a total of 94 samples (52 from patients with CRC and 42 controls). B, specificity (Spec) and sensitivity (Sens) obtained using the HAS and Annexin IV controls for independently discriminating patients with CRC from different subjects. C, specificity and sensitivity obtained from the analysis of ROC curves using an optimal combination of biomarkers (MAPKAPK3, ACVR2B, Pim1 and FGFR4). D, specificity and sensitivity of an optimal combination of biomarkers for the early stages of CRC (MAPKAPK3, ACVR2B, Pim1 and FGFR4). [AUC: Area under the curve; Sens: Sensitivity; Spec: Specificity].

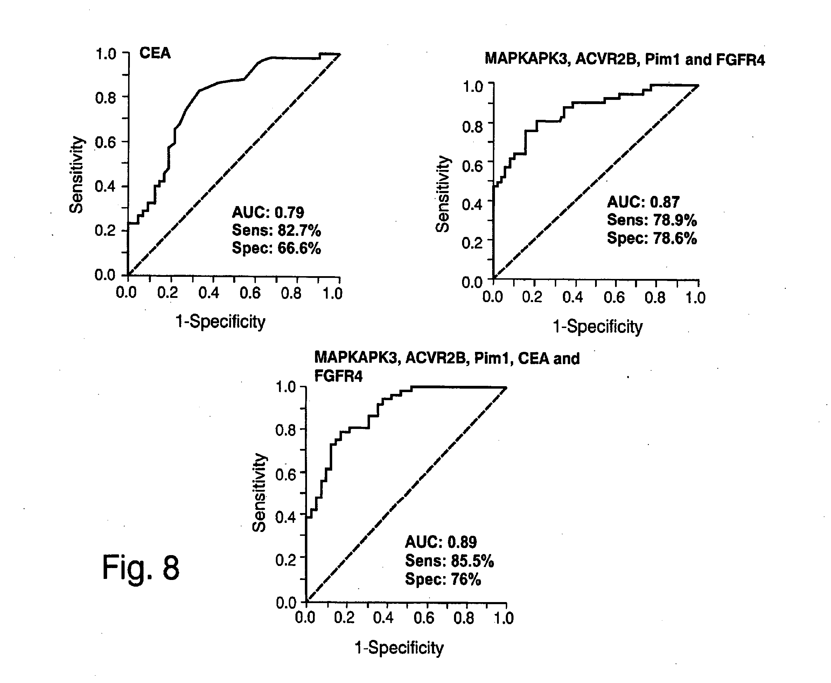

Figure 8 shows the validation of a combination of markers for the diagnosis of CRC. The role of CEA alone and with an optimal combination of markers for the diagnosis of CRC (MAPKAPK3, ACVR2B, Pim1 and FGFR4). It also shows the combination of the autoantibodies to MAPKAPK3, ACVR2B, Pim1 and FGFR4 and CEA by independently discriminating serum from patients with CRC from serum from reference subjects (controls) in a validation group of a total of 94 samples (52 from patients with CRC and 42 from controls), indicating that the markers provided by this invention combined with CEA significantly improve the detection of CRC.

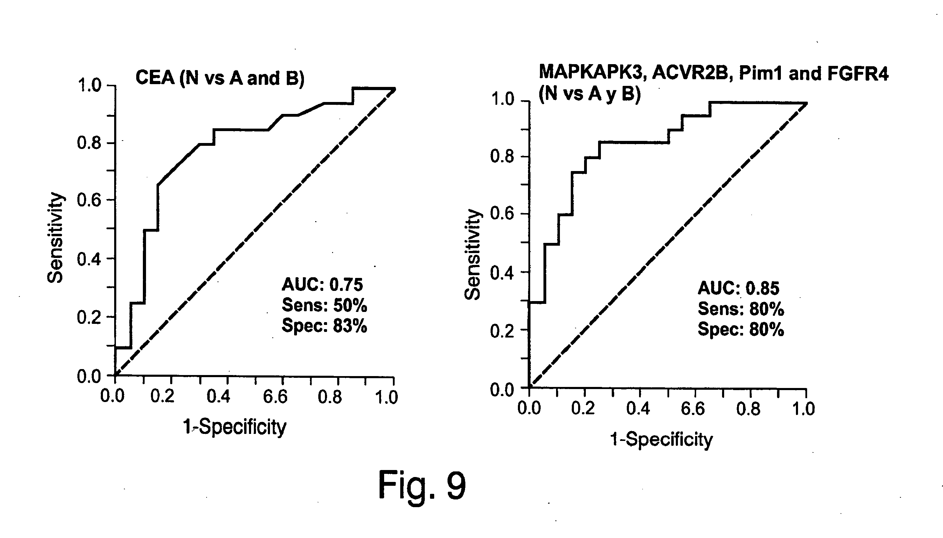

Figure 9 shows the validation of a combination of markers for the diagnosis of CRC in early stages. The role of CEA alone and with an optimal combination of markers for the diagnosis of CRC (MAPKAPK3, ACVR2B, Pim1 and FGFR4) for discriminating CRC in an early stage using 20 control healthy subjects and 20 sera from patients with CRC in stages A and B. The combination of the autoantibodies to MAPKAPK3, ACVR2B, Pim1 and FGFR4 and CEA did not improve the prediction capacity for the diagnosis of CRC, indicating that said combination of markers provided by this invention is more suitable for the diagnosis of CRC in early stages than CEA alone.

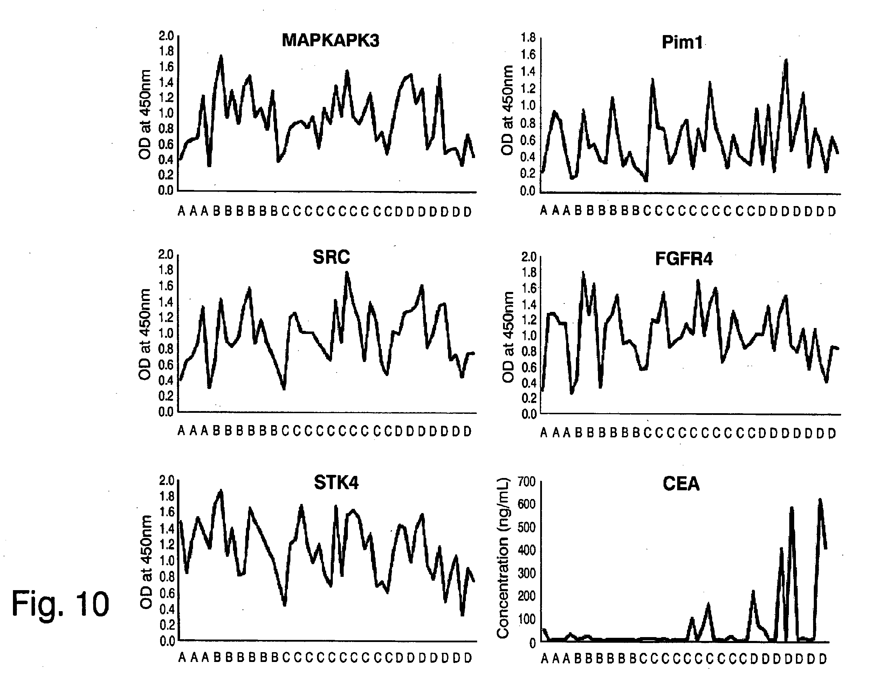

Figure 10 shows a graph of the values obtained by means of an ELISA of the concentration of MAPKAPK3, Pim1, SRC, FGFR4 and STK4 and CEA in serum from patients with CRC. The concentration of CEA was greater in later stages of CRC than in early stages of CRC (where its concentration was rather low). The presence of autoantibodies in serum from patients with CRC with respect to the selected biomarkers provided by this invention was constant during all the steps, allowing a better diagnosis of CRC not only in later stages but also in early stages of CRC.

### Detailed Description of the Invention

### Definitions

To facilitate their understanding, the meaning of some terms and expressions as they are used in the present description are indicated below.

The term "antibody", as it is used herein, relates to immunoglobulin molecules and immunologically active portions of immunoglobulin molecules, i.e., molecules containing an antigen binding site which specifically binds (immunoreacts) with an antigen, such as, for example, a protein. There are 5 isotypes or main classes of immunoglobulins: immunoglobulin M (IgM), immunoglobulin D (IgD), immunoglobulin G (IgG), immunoglobulin A (IgA) and immunoglobulin E (IgE).

The term "autoantibody", as it is used herein, is applied to an antibody which reacts with an antigen present in the actual organism of a subject, even if the reaction occurs only *in vitro*, and whether or not it causes *in vivo* pathological effects.

The term "autoantibody to the Pim1 protein", as it is used herein, relates to an autoantibody capable of reacting with the Pim1 protein, or with a variant or with a fragment of said protein, provided that said variant or said fragment is functionally equivalent, i.e., susceptible of being recognized by said autoantibody. In a particular embodiment, said autoantibody to the Pim1 protein is an IgG; in another particular embodiment, said autoantibody to the Pim1 protein is an IgM.

The term "autoantibody to the SRC protein", as it is used herein, relates to an autoantibody capable of reacting with the SRC protein, or with a variant or with a fragment of said protein, provided that said variant or said fragment is functionally equivalent, i.e., susceptible of being recognized by said autoantibody. In a particular embodiment, said autoantibody to the SRC protein is an IgG; in another particular embodiment, said autoantibody to the SRC protein is an IgM.

The term "autoantibody to the MAPKAPK3 protein", as it is used herein, relates to an autoantibody capable of reacting with the MAPKAPK3 protein, or with a variant or with a fragment of said protein, provided that said variant or said fragment is functionally equivalent, i.e., susceptible of being recognized by said autoantibody. In a particular embodiment, said autoantibody to the MAPKAPK3 protein is an IgG; in another particular embodiment, said autoantibody to the MAPKAPK3 protein is an IgM.

The term "autoantibody to the FGFR4 protein", as it is used herein, relates to an autoantibody capable of reacting with the FGFR4 protein, or with a variant or with a fragment of said protein, provided that said variant or said fragment is functionally equivalent, i.e., susceptible of being recognized by said autoantibody. In a particular embodiment, said autoantibody to the FGFR4 protein is an IgG; in another particular embodiment, said autoantibody to the FGFR4 protein is an IgM.

The term "autoantibody to the STK4 protein", as it is used herein, relates to an autoantibody capable of reacting with the STK4 protein, or with a variant or with a fragment of said protein, provided that said variant or said fragment is functionally equivalent, i.e., susceptible of being recognized by said autoantibody. In a particular embodiment, said autoantibody to the STK4 protein is an IgG; in another particular embodiment, said autoantibody to the STK4 protein is an IgM.

The term "autoantibody to the ACVR2B protein", as it is used herein, relates to an autoantibody capable of reacting with the ACVR2B protein, or with a variant or with a fragment of said protein, provided that said variant or said fragment is functionally equivalent, i.e., susceptible of being recognized by said autoantibody. In a particular embodiment, said autoantibody to the ACVR2B protein is an IgG; in another particular embodiment, said autoantibody to the ACVR2B protein is an IgM.

The term "colorectal cancer" or "CRC", also called colon cancer, as it is used herein, includes any type of neoplasias of the colon, rectum and appendix, as well as any histological subtype typically occurring in colon cancer, e.g., Transitional cell carcinoma, Squamous cell carcinoma and adenocarcinoma, any clinical subtype, e.g., surface, invasive muscle or metastatic disease cancer, or any TNM stage including T0-T4, N0-N2 and M0-M1 tumors. Patients can be classified in different groups with respect to the stage of the tumor. The classification of colon cancer is an estimate of the penetration of a particular cancer. It is carried out for investigative purposes, diagnostic purposes and for determining the best method of treatment. The system for the classification of colorectal cancers depends on the extent of local invasion, on the degree of lymphatic nodes involved and on if distal metastasis exists. The most common classification system is the TNM (for tumors/nodes/metastasis) system, of the "American Joint Committee on Cancer" (AJCC). The TNM system assigns a number based on three categories. "T" indicates the degree of invasion of the intestinal wall, "N" the degree of involvement of lymphatic nodes and "M" the degree of metastasis. The broadest stage of cancer is usually mentioned as a number I, II, III, IV derived from the TNM value clustered by the prognosis, a higher number indicates a more advanced cancer and a worse prognosis. Details of the system are indicated in Table 1.

**Table 1**

| TNM system for the classification of CRC | | |
|---|---|---|
| **AJCC Stage** | **TNM Stage** | **Criteria of TNM stages for CRC** |
| Stage 0 | Tis N0 M0 | Tis: The tumor confined to the mucosa; cancer-in-situ |
| Stage I | T1 N0 M0 | T1: The tumor invades the mucosa |
| Stage I | T2 N0 M0 | T2: The tumor invades the actual muscles |
| Stage II-A | T3 N0 M0 | T3: The tumor invades the subserosal layer or beyond (other organs not involved) |
| Stage II-B | T4 N0 M0 | T4: The tumor invades adjacent organs or perforates the visceral peritoneum |
| Stage III-A | T1-2 N1 M0 | N1: Metastasis of 1 to 3 regional lymphatic nodes. T1 or T2. |
| Stage III-B | T3-4 N1 M0 | N1: Metastasis of 1 to 3 regional lymphatic nodes. T3 or T4. |
| Stage III-C | any T, N2 M0 | N2: Metastasis of 4 or more regional lymphatic nodes. Any T. |
| Stage IV | any T, any N, M1 | M1: Presence of distal metastasis. Any T, any N. |

The term "quantifying", as it is used herein, relates to the measurement of the amount or concentration, preferably in a quantitative, semi-quantitative or relative manner of a product, for example, autoantibodies to a determined protein (e.g., Pim1, SRC, MAPKAPK3, FGFR4, STK4, ACVR2B, etc., or to the proteins mentioned in Tables 2 and 3), expression products (e.g., RNA or protein) of the genes encoding a determined protein (e.g., *Pim1, SRC, MAPKAPK3, FGFR4, STK4, ACVR2B,* etc.), etc. The quantification of a product can be carried out directly or indirectly. The direct measurement relates to the measurement of the amount or concentration of said product based on the signal which is obtained directly from said product and which is correlated directly with the number of molecules of the product in question present in the analyzed sample. Said signal (which can also be referred to as intensity signal) can be obtained, for example, by measuring an intensity value of a chemical or physical property of the product in question. The indirect measurement of the amount or concentration of a product includes the measurement obtained from a secondary component (e.g., a component different from the autoantibodies) or a biological measurement system (e.g., the measurement of cell responses, ligands, "tags", enzymatic reaction products, etc.).

The quantification of the level of expression of an expression product of a gene can be carried out directly or indirectly. The direct measurement relates to the measurement of the amount or concentration of an expression product of a gene based on the signal which is obtained directly from the expression product of said gene and which is correlated directly with the number of molecules of the expression product of said gene present in the analyzed sample. Said signal, which can also be referred to as intensity signal, can be obtained, for example, by measuring an intensity value of a chemical or physical property of the expression product of the gene in question (e.g., *Pim1, SRC, MAPKAPK3, FGFR4, STK4, ACVR2B,* etc.). The indirect measurement of the amount or concentration of an expression product of a gene includes the measurement obtained from a secondary component (e.g., a component different from the expression products of the gene in question) or a biological measurement system (e.g., the measurement of cell responses, ligands, "tags", enzymatic reaction products, etc.).

The term "diagnosis", as it is used herein, generally relates to the process by which a disease, nosological entity, syndrome, or any disease-health condition is identified. Particularly, the term "diagnosis of colorectal cancer or CRC" relates to the capacity to identify or detect the presence of CRC; this detection, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical tools; illustrative, non-limiting examples of said statistical tools include determining confidence intervals, determining the p-value, the Student's t-test or Fisher's discriminant functions, etc. (see, for example, Dowdy and Wearden, Statistics for Research, John Wiley & Sons, New York 1983). The confidence intervals are preferably at least 90%, at least 95%, at least 97%, at least 98% or at least 99%. The p-value is preferably less than 0.1, less than 0.05, less than 0.01, less than 0.005 or less than 0.0001. The teachings of the present invention preferably allow correctly detecting the disease (CRC) in at least 60%, in at least 70%, in at least 80%, or in at least 90% of the subjects of a determined group or population analyzed.

The term "fragment" applied to a protein, as it is used herein, relates to a portion of a protein, for example, a protein selected from the group consisting of the Pim1, SRC, MAPKAPK3, FGFR4, STK4 and ACVR2B proteins or their variants.

The expression "fragment of a protein susceptible of being recognized by an autoantibody which recognizes said protein", as it is used herein, relates to a fragment of a protein which is recognized by an autoantibody to said protein, such that a stable autoantibody-protein fragment complex is formed. By way of non-limiting illustration, said protein can be a protein selected from the group of proteins consisting of Pim1, SRC, MAPKAPK3, FGFR4, STK4 and ACVR2B proteins.

The expression "functionally equivalent" applied to proteins variants or fragments, as it is used herein, means that the variant or the fragment of the protein in question essentially maintains the immunological properties of said protein in question. Said immunological properties can be determined by means of conventional methods such as those described in the Examples included in this description (e.g., by means of ELISA assays, etc.).

The term *"Pim1* gene", as it is used herein, relates to the gene or to the nucleic acid sequence encoding the Pim1 protein, as it is herein defined, and furthermore includes, by extension, the nucleic acid sequence encoding a fragment of said functionally equivalent Pim1 protein.

The term *"SRC* gene", as it is used herein, relates to the gene or to the nucleic acid sequence encoding the SRC protein, as it is herein defined, and furthermore includes, by extension, the nucleic acid sequence encoding a fragment of said functionally equivalent SRC protein.

The term "MAPKAPK3 gene", as it is used herein, relates to the gene or to the nucleic acid sequence encoding the MAPKAPK3 protein, as it is herein defined, and furthermore includes, by extension, the nucleic acid sequence encoding a fragment of said functionally equivalent MAPKAPK3 protein.

The term *"FGFR4* gene", as it is used herein, relates to the gene or to the nucleic acid sequence encoding the FGFR4 protein, as it is herein defined, and furthermore includes, by extension, the nucleic acid sequence encoding a fragment of said functionally equivalent FGFR4 protein.

The term *"STK4* gene", as it is used herein, relates to the gene or to the nucleic acid sequence encoding the STK4 protein, as it is herein defined, and furthermore includes, by extension, the nucleic acid sequence encoding a fragment of said functionally equivalent STK4 protein.

The term *"ACVR2B* gene", as it is used herein, relates to the gene or to the nucleic acid sequence encoding the ACVR2B protein, as it is herein defined, and furthermore includes, by extension, the nucleic acid sequence encoding a fragment of said functionally equivalent ACVR2B protein.
The term "identity", applied in the comparison between the amino acid sequences of 2 proteins, as it is used herein, relates to the proportion of identical amino acids between 2 amino acid sequences which are compared. The degree of identity usually expressed as a percentage (%) of identity) existing between 2 amino acid sequences can be easily identified by a person skilled in the art, for example, with the aid of a suitable computer program for comparing sequences; by way of non-limiting illustration, the degree of identity between two amino acid sequences can be determined by conventional methods, for example, by means of methods and computer algorithms known by the persons skilled in the art; by way of illustration, the degree of identity between 2 amino acid sequences can be determined by means of using the BLAST algorithm (BLAST Manual, Altschul et al., NCBI NLM NIH Bethesda, Md. 20894, Altschul et al., J. Mol. Biol. 1990; 215:403-410).

The term "immunoassay", as it is used herein, relates to any analytical technique based on a conjugation reaction between an antigen, for example, a protein or a suitable fragment thereof, and an antibody which recognizes said antigen. By way of illustration, said protein can be a protein selected from the group of proteins consisting of the Pim1, SRC, MAPKAPK3, FGFR4, STK4 and ACVR2B proteins or of the proteins mentioned in Tables 2 and 3. Alternatively suitable fragments of said proteins can be used, i.e., fragments of proteins susceptible of being recognized by antibodies which recognize the proteins in question; by way of illustration, said protein fragments can be fragments of the Pim1, SRC, MAPKAPK3, FGFR4, STK4 and ACVR2B proteins, or of the proteins mentioned in Tables 2 and 3, susceptible of being recognized by the autoantibodies which recognize said proteins.

The term "marker", as it is used herein, relates to a indicator reagent which allows detecting an antigen-antibody type complex, such as an enzyme catalyzing a detectable reaction, a compound generating a signal when it forms part of said complex, etc. By way of non-limiting illustration, said marker can be an enzyme (e.g., peroxidase, glycosidase, alkaline phosphatase, glucose-6-phosphate dehydrogenase, β-galactosidase, β-glucosidase, β-glucuronidase, etc.), a fluorescent compound or fluorophore (e.g., fluoresceine, rhodamine, etc.), a (chemo)luminescent compound (e.g., dioxetanes, acridiniums, phenanthridiniums, ruthenium, luminol, etc.), a radioactive element (sulfur, iodine, etc.), etc. In a particular embodiment, said marker is a peroxidase. The selection of a particular marker is not critical, provided that it is capable of producing a signal by itself or together with one or more additional substances.

The term "metastasis", as it is used herein, relates to the process by which a tumor, in this case CRC, extends to tissues of the organism different from the primary site of origin of the tumor.

The term "biological sample", as it is used herein, relates but is not limited to biological tissues and/or fluids of a subject, obtained by means of any method known by a person skilled in the art which serves to carry out any of the methods provided by the present invention; i.e., said biological sample must be a sample susceptible of containing antibodies, e.g., autoantibodies to the Pim1, SRC, MAPKAPK3, FGFR4, STK4 and/or ACVR2B proteins, as well as to the proteins mentioned in Tables 2 and 3, or susceptible of containing the expression products (RNA or proteins) of the genes encoding the Pim1, SRC, MAPKAPK3, FGFR4, STK4 and ACVR2B proteins. By way of non-limiting illustration, said biological sample can be a blood, urine, saliva, serum, plasma sample, a buccal or buccal-pharyngeal swab, a surgical specimen, a specimen obtained from a biopsy or autopsy, etc.

The term "level", as it is used herein, generally relates to a quantifiable, semiquantifiable, or relative amount of a product, for example, autoantibodies, expression products of the genes, etc., as well as to any other value or parameter related to said expression product or which can be derived therefrom. Said values or parameters comprise signal intensity values obtained from any of the physical or chemical properties of the product in question. The levels of a product can generally be based on quantitative and/or semiquantitative analyses; by way of illustration, quantitative methods can be used for determining a relative or absolute amount of a specific product in the biological sample assayed, and semiquantitative methods can be used for establishing the level of said specific product above a baseline value without needing to assign an absolute or relative numerical value.

By way of non-limiting illustration, the "level of an autoantibody" to a protein (e.g., Pim1, SRC, MAPKAPK3, FGFR4, STK4, ACVR2B, as well as to the proteins mentioned in Tables 2 and 3), relates but is not limited to the quantifiable, semiquantifiable, or relative amount of the autoantibodies to said proteins (e.g., Pim1, SRC, MAPKAPK3, FGFR4, STK4, ACVR2B, and proteins mentioned in Tables 2 and 3), as well as to any other value or parameter related to said autoantibodies or which can be derived therefrom. Said values or parameters comprise signal intensity values obtained from any of the physical or chemical properties of the autoantibodies to said proteins (e.g., Pim1, SRC, MAPKAPK3, FGFR4, STK4, ACVR2B, and proteins mentioned in Tables 2 and 3) obtained either by means of direct measurement, e.g., intensity values of mass spectroscopy, nuclear magnetic resonance, etc., or by means of indirect measurement, e.g., by means of any of the systems of measurement described herein, for example, by means of the measurement obtained from a secondary component (e.g., a component different from the autoantibodies) or a biological measurement system (e.g., the measurement of cell responses, ligands, "tags" or enzymatic reaction products). The determination of the level of an autoantibody to a protein (e.g., Pim1, SRC, MAPKAPK3, FGFR4, STK4, ACVR2B, as well as to the proteins mentioned in Tables 2 and 3) can be performed using any available method known by the person skilled in the art, for example, by means of an immunoassay. The level of an autoantibody to a protein (e.g., Pim1, SRC, MAPKAPK3, FGFR4, STK4, ACVR2B, or to the proteins mentioned in Tables 2 and 3) determined in a biological sample from the subject under study is said to be "greater" than the reference level of said autoantibody when, according to the invention, the level of said autoantibody in the biological sample from the subject is at least 1.5 times, 5 times, 10 times, 20 times, 30 times, 40 times, 50 times, 60 times, 70 times, 80 times, 90 times, 100 times or even more, with respect to the reference level of said autoantibody. Similarly, the level of an autoantibody to a protein (e.g., Pim1, SRC, MAPKAPK3, FGFR4, STK4, ACVR2B, or to the proteins mentioned in Tables 2 and 3) determined in a biological sample from the subject under study is said to be "less" than the reference level of said autoantibody when, according to the invention, the level of said autoantibody in the biological sample from the subject is at least 1.5 times, 5 times, 10 times, 20 times, 30 times, 40 times, 50 times, 60 times, 70 times, 80 times, 90 times, 100 times, or even more, lower than the reference level of said autoantibody.

Likewise, by way of non-limiting illustration, the "level of expression of an expression product" of a gene or, in other words, amount of expression product of a gene, as it is used herein, relates but is not limited to the quantifiable, semiquantifiable, or relative amount of an expression product of a gene determined (e.g., *Pim1, SRC, MAPKAPK3, FGFR4, STK4* or *ACVR2B),* as well as to any other value or parameter related to said expression product or which can be derived therefrom. Said values or parameters comprise signal intensity values obtained from any of the physical or chemical properties of the expression product of said gene (e.g., *Pim1, SRC, MAPKAPK3, FGFR4, STK4* or *ACVR2B)* obtained either by means of direct measurement, or by means of indirect measurement. The determination of the level of expression of an expression product of a gene (e.g., *Pim1, SRC, MAPKAPK3, FGFR4, STK4* or *ACVR2B)* can be performed using any available method known by the person skilled in the art. The level of expression of an expression product of a gene (e.g., *Pim1, SRC, MAPKAPK3, FGFR4, STK4* or *ACVR2B),* determined in a biological sample from the subject under study is said to be "greater" than the reference level of said expression product of said gene when, according to the invention, the level of said expression product of said gene in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, with respect to the reference level of said expression product of said gene. Similarly, the level of expression of an expression product of a gene (e.g., *Pim1, SRC, MAPKAPK3, FGFR4, STK4* or *ACVR2B),* determined in a biological sample from the subject under study is said to be "less" than the reference level of said expression product of said gene when, according to the invention, the level of said expression product in said biological sample from the subject is at least 1.5 times, 5 times, 10 times, 20 times, 30 times, 40 times, 50 times, 60 times, 70 times, 80 times, 90 times, 100 times, or even more, lower than the reference level for said expression product of said gene.

The term "reference level", as it is used herein, generally relates to the level of a product, for example, autoantibodies to proteins (e.g., Pim1, SRC, MAPKAPK3, FGFR4, STK4, ACVR2B, as well as to the proteins mentioned in Tables 2 and 3), expression products of the genes (e.g., *Pim1, SRC, MAPKAPK3, FGFR4, STK4* or *ACVR2B,* etc.), etc., present in control subjects. In a particular embodiment, said control subjects are subjects who do not suffer a determined disease (e.g., CRC), whereas in another particular embodiment, said control subject is the actual subject under study, which is particularly useful for evaluating the tracking of a disease (e.g., CRC) or for evaluating the effectiveness of a treatment for said disease (e.g., CRC), etc., for which the reference level of a given product can be the level of said product determined in a sample from the same subject under study but taken days, weeks, months or even years before for the purpose of evaluating the tracking of the disease, or taken before, for example, the application in the subject of a treatment for said disease for the purpose of evaluating its effectiveness.

Due to the variability that can occur between the different subjects in terms of the production of autoantibodies to proteins (e.g., Pim1, SRC, MAPKAPK3, FGFR4, STK4, ACVR2B, as well as to the proteins mentioned in Tables 2 and 3), or in terms of the production of expression products of genes (e.g., *Pim1, SRC, MAPKAPK3, FGFR4, STK4* or *ACVR2B,* etc.), the reference level could be obtained from a set of samples from a population of healthy subjects (e.g., subjects who do not suffer CRC) and by calculating the mean level of the product in question (autoantibody or expression product of a gene) in said population of healthy subjects.

The reference level of a determined product, for example, autoantibodies to proteins (e.g., Pim1, SRC, MAPKAPK3, FGFR4, STK4, ACVR2B, as well as to the proteins mentioned in Tables 2 and 3), expression products of the genes (e.g., *Pim1, SRC, MAPKAPK3, FGFR4, STK4* or *ACVR2B,* etc.), etc., can be determined from a reference sample which can be analyzed, for example, simultaneously or consecutively, together with the biological sample from the subject under study (test sample). The reference level can generally be derived from the normal distribution limits of a physiological amount found in a population of control subjects. Said physiological amount can be determined by several well-known techniques, depending on the nature of the product in question (autoantibody, expression product of a gene, etc.), as is described in this description.

According to the present invention, said reference level allows discriminating the presence of CRC and, therefore, it can be used in the diagnosis, prognosis or tracking of the progress of a CRC.

The term "prediction", as it is used herein, relates but is not limited to the probability that a patient, such as a patient suffering CRC, will respond favorably or unfavorably to a determined treatment, and to the extent of said responses, or that the patient will survive, after the surgical elimination of a primary tumor and/or the chemotherapy for a time period without a recurrence of the CRC occurring.

The term "expression product" of a gene (or of a nucleic acid sequence), as it is used herein, relates to the product resulting from the transcription (RNA) or from the expression (protein) of said gene or nucleic acid sequence, as well as to any form resulting from the processing of the product resulting from the transcription or from the expression of said gene or nucleic acid sequence.

The term "prognosis", as it is used herein, generally relates to the set of data within medical science concerning the probability that determined situations will occur in the course of time or natural history of a disease; i.e., it is the prediction of the events which will occur in the development of a disease in statistical terms. Particularly, the term "prognosis of CRC", as it is used herein, relates to the set of data which allows assigning a probability that determined situations will occur in the course of the CRC. Thus, according to the present invention, it includes the capacity to assign a probability that determined situations will occur in the course of the disease of CRC, when a method for the classification of samples is applied based either on the comparison of the level of autoantibodies to at least one of the proteins selected from the group consisting of the Pim1, SRC, MAPKAPK3, FGFR4, STK4 and ACVR2B proteins, or to all or some of the proteins mentioned in Tables 2 and 3, with the reference level for said autoantibodies, or in the comparison of the level of at least one expression product of a gene selected from the group consisting of the *Pim1, SRC, MAPKAPK3, FGFR4, STK4* and *ACVR2B* genes, with the reference level for said expression product of the gene. This assignment, as it is understood by a person skilled in the art, does not claim to be correct in 100% of the analyzed samples. However, it requires that a statistically significant amount of the analyzed samples are correctly classified. The amount that is statistically significant can be established by a person skilled in the art by means of using different statistical tools, e.g., by means of determining confidence intervals, determining the p-value, Student's t-test, Fisher's discriminant functions, etc. The confidence intervals are preferably at least 90%, at least 95%, at least 97%, at least 98% or at least 99%. The p-value is preferably less than 0.1, less than 0.05, less than 0.01, less than 0.005 or less than 0.0001.

By way of non-limiting illustration, the term prognosis, as it is used herein, relates to the probability of death due to CRC or to the progression of CRC, including recurrence or capacity of metastatic dissemination, as well as to the prediction of response to a determined treatment of CRC. The progress of the disease can be tracked using any assessment criterion used in the field of cancer and known by the person skilled in the art. The assessment parameters useful for describing the progress of a disease include but are not limited to:
- disease-free progress which, as it is used herein, describes the proportion of patients in complete remission who have not had a relapse of the disease during the time period under study;
- objective response which, as it is used herein, describes the proportion of subjects of a treated population in which a complete or partial response is observed;
- time to progression (TTP), which is a measurement of the time after the disease is diagnosed or treated until the disease deteriorates; it is considered that the disease has progressed if the symptoms of the cancer, including increased tumor mass, metastasis, increased metastasis, etc., have deteriorated in relation to the initial measurements;
- disease-free survival (DFS) which, as it is used herein, is defined as the time after the treatment in which a patient survives without signs of deterioration;
- 6-month progression-free survival or "PFS6" rate which, as it is used herein, relates to the percentage of people in whom the disease does not progress in the first 6 months after beginning therapy;
- median survival (MS) which, as it is used herein, relates to the time in which half the patients enrolled in the study are still alive;
- distant relapse-free survival (DRFS) which, as it is used herein, relates to the time elapsing from the date of surgery until the metastasis or until the last visit; and
- overall survival (OS) which, as it is used herein, relates to the time elapsing from the date of surgery until the last visit or until the death of the subject.

The term "Pim1 protein", as it is used herein, includes the Pim1 protein of a subject, preferably of a human being, and variants thereof; in a particular embodiment, said Pim1 protein is the protein the accession number of which is NP_002639 and its amino acid sequence is shown in SEQ ID NO: 1.

The term "SRC protein", as it is used herein, includes the SRC protein of a subject, preferably of a human being, and variants thereof; in a particular embodiment, said SRC protein is the protein the accession number of which is NP005408 and its amino acid sequence is shown in SEQ ID NO: 2.

The term "MAPKAPK3 protein", as it is used herein, includes the MAPKAPK3 protein of a subject, preferably of a human being, and variants thereof; in a particular embodiment, said SRC protein is the protein the accession number of which is NP_004626 and its amino acid sequence is shown in SEQ ID NO: 3.

The term "FGFR4 protein", as it is used herein, includes the FGFR4 protein of a subject, preferably of a human being, and variants thereof; in a particular embodiment, said FGFR4 protein is the protein the accession number of which is NP_002002 and its amino acid sequence is shown in SEQ ID NO: 4.

The term "STK4 protein", as it is used herein, includes the STK4 protein of a subject, preferably of a human being, and variants thereof; in a particular embodiment, said STK4 protein is the protein the accession number of which is NP_006273 and its amino acid sequence is shown in SEQ ID NO: 5.

The term "ACVR2B protein", as it is used herein, includes the ACVR2B protein of a subject, preferably of a human being, and variants thereof; in a particular embodiment, said ACVR2B protein is the protein the accession number of which is NP_001097 and its amino acid sequence is shown in SEQ ID NO: 6.

The term "tracking of the progress", as it is used herein, relates to the supervision of the development of a disease such as, for example, but without being limited to, the evaluation of the response to a determined treatment for said disease (e.g., CRC) or the detection of the recurrence or of the dissemination of CRC.

The term "subject", as it is used herein, relates to an animal, preferably a mammal, and, more preferably, a human being. For the sake of clarity, subjects suffering CRC are occasionally referred to in this description as "patients with CRC" or by means of a similar expression.

A protein is "substantially homologous" to a determined protein when its amino acid sequence has suitable alignment with the amino acid sequence of said determined protein, for example, when its degree of identity with respect to said determined protein is at least 50%, typically at least 70%, advantageously at least 80%, preferably at least 85%, more preferably at least 90%, even more preferably at least 95%, and, still more preferably at least 99%. By way of non-limiting illustration, in a particular embodiment, a protein is substantially homologous to the Pim1 protein when its amino acid sequence has a degree of identity respect to the amino acid sequence shown in SEQ ID NO: 1, of at least 50%, typically at least 70%, advantageously at least 80%, preferably at least 85%, more preferably at least 90%, even more preferably at least 95%, and, still more preferably at least 99%. The proteins substantially homologous to the SRC, MAPKAPK3, FGFR4, STK4 and ACVR2B proteins can be defined in the same manner, but replacing the amino acid sequence shown in SEQ ID NO: 1 with the amino acid sequences shown in SEQ ID NO: 2, SEQ ID NO: 3, SEQ ID NO: 4, SEQ ID NO: 5 and SEQ ID NO: 6, respectively.

The term "variant", as it is used herein, relates to a protein substantially homologous to other protein, for example, to the Pim1, SRC, MAPKAPK3, FGFR4, STK4 or ACVR2B protein. A variant generally includes additions, deletions or substitutions of amino acids. The term variant also includes the proteins resulting from post-translational modifications such as, for example, but without being limited to, glycosylation, phosphorylation or methylation. According to the present invention, said variants are recognized by autoantibodies to the protein in question.

### Method of detection of autoantibodies

The description describes a method for the detection of an autoantibody to a protein, hereinafter "method of detection of autoantibodies", which comprises
a) contacting a biological sample with said protein or with a fragment thereof susceptible of being recognized by said autoantibody; and
b) detecting the formation of an autoantibody-protein, or fragment thereof, complex susceptible of being recognized by said autoantibody;
wherein said protein is selected from the group consisting of the Pim1, SRC, MAPKAPK3, FGFR4, STK4, ACVR2B proteins and combinations thereof.

The biological sample will generally be a sample susceptible of containing antibodies from a subject, and it can be obtained by conventional methods known by the persons skilled in the art, depending on the nature of the sample. In a particular embodiment, said biological sample is a blood, plasma or serum sample, which can be obtained by any conventional method, for example, by means of an extraction of blood, etc. Blood is usually the optimal biological fluid to be used in non-invasive methods for massive screening for diagnostic purposes in large subject populations. On one hand, serum and plasma are easy to obtain, and on the other hand, blood circulation facilitates the contact of the blood with all the tissues of the human body, including the contact with tumor tissue and its representative antigens in the case of patients with cancer.

The method of detection of autoantibodies can generally be performed by means of an immunoassay; illustrative non-limiting examples of immunoassays known in the state of the art include immunoblotting, enzyme-linked immunosorbent assay (ELISA), linear immunoassay (LIA), radioimmunoassay (RIA), immunofluorescence (IF), immunohistochemistry (IHQ), protein microarrays, etc.

In step a) of the method of detection of autoantibodies, a biological sample in which the presence of autoantibodies to the proteins Pim1, SRC, MAPKAPK3, FGFR4, STK4 and/or ACVR2B is to be analyzed is contacted with said proteins or with fragments thereof susceptible of being recognized by said autoantibodies, under conditions which allow the formation of an autoantibody-protein, or fragment thereof, complex susceptible of being recognized by said autoantibody. If the biological sample contains autoantibodies to said proteins, then said autoantibody-protein, or fragment thereof, complex susceptible of being recognized by said autoantibody will be formed; otherwise, said complex will not be formed. The conditions suitable for the formation of the autoantibody-protein, or fragment thereof, complex susceptible of being recognized by said autoantibody occurring are known by persons skilled in the art.

Although said proteins (Pim1, SRC, MAPKAPK3, FGFR4, STK4 and/or ACVR2B) could be together in one and the same medium, in practice it is advantageous for said proteins to be separated from one another. Said proteins can be in solution or suspension in a suitable medium, or they can alternatively be deposited or supported on a support (e.g., a microtiter plate, beads (magnetic or non-magnetic), columns, matrices, membranes, etc. These materials can be used in the suitable forms, such as films, sheets, plates, etc., or they can be used to coat inert carriers (e.g., paper, glass, plastic films, etc.). In a particular embodiment, said biological sample is contacted with said Pim1, SRC, MAPKAPK3, FGFR4, STK4 and/or ACVR2B proteins, or with fragments thereof susceptible of being recognized by said autoantibodies, separated from one another, and deposited on a suitable support.

In a particular embodiment, the autoantibodies to said proteins are identified independently, whereas in another particular embodiment the autoantibodies to said proteins are identified simultaneously.

In a particular embodiment, the biological sample to be studied is contacted with a single protein selected from among the Pim1, SRC, MAPKAPK3, FGFR4, STK4 and ACVR2B proteins, or with a fragment thereof susceptible of being recognized by said autoantibody, for the purpose of identifying autoantibodies to said protein. In another particular embodiment, said biological sample is contacted with two or more of said Pim1, SRC, MAPKAPK3, FGFR4, STK4 and ACVR2B proteins, or fragments thereof susceptible of being recognized by said autoantibodies, separated from one another, optionally deposited on a suitable support, for the purpose of identifying autoantibodies to said proteins.

Step b) of the method of detection of autoantibodies comprises detecting the formation of an autoantibody-protein, or fragment thereof, complex susceptible of being recognized by said autoantibody. This step can be carried out by conventional methods known by the persons skilled in the art, for the detection of the formation of antibody-antigen (in this case, autoantibody-protein or fragment thereof susceptible of being recognized by said autoantibody) complexes.

In a particular embodiment, by way of non-limiting illustration, for the detection of said complex, a conjugate comprising an antibody which recognizes the autoantibody and a marker (labeled secondary antibody) can be added under conditions which allow the formation of an (autoantibody-protein or fragment thereof susceptible of being recognized by said autoantibody)-antibody/marker complex and detecting the formation of said complex. If the biological sample contains autoantibodies to one or more of said proteins (Pim1, SRC, MAPKAPK3, FGFR4, STK4 and/or ACVR2B) then the autoantibody-protein, or fragment thereof, complex susceptible of being recognized by said autoantibody will have been previously formed, with which, when said complex is contacted with said conjugate comprising the antibody and the marker in the suitable conditions, the (autoantibody-protein or fragment thereof susceptible of being recognized by said autoantibody)-antibody/marker complex is formed, which will be displayed by means of the suitable technique depending on the marker used, as is mentioned below; whereas, otherwise, i.e., when the biological sample does not contain autoantibodies to said protein/proteins, then said (autoantibody-protein or fragment thereof susceptible of being recognized by said autoantibody)-antibody/marker complex will not be formed. The conditions suitable for the formation of this latter complex to occur are known by the persons skilled in the art.

Virtually any indicator reagent which allows detecting said (autoantibody-protein or fragment thereof susceptible of being recognized by said autoantibody)-antibody/marker complex can be used in putting the present invention into practice; by way of non-limiting illustration, said marker can be an enzyme catalyzing a detectable reaction (e.g., peroxidase, glycosidase, alkaline phosphatase, glucose-6-phosphate dehydrogenase, β-galactosidase, β-glucosidase, β-glucuronidase, etc.), a compound generating a signal when it forms part of said complex (e.g., a fluorescent compound or fluorophore, such as fluoresceine, rhodamine, etc.; a (chemo)luminescent compound, such as a dioxetane, an acridinium, a phenanthridinium, ruthenium, luminol, etc.), etc., a radioactive element (e.g., sulfur, iodine, etc.), etc. In a particular embodiment, said marker is a peroxidase. The selection of a particular marker is not critical, provided that it is capable of producing a signal by itself or together with one or more additional substances. The (autoantibody-protein or fragment thereof susceptible of being recognized by said autoantibody)-antibody/marker complex formed can be thereby detected or displayed by any suitable technique, depending on the chosen marker, known by the persons skilled in the art, using the suitable devices, for example, by means of techniques based on colorimetric, fluorometric, (chemo)luminescent, radioactive methods, etc., all of them known by the persons skilled in the art.

The conjugate comprising said antibody which recognizes said autoantibody and said marker can be obtained by conventional methods known by the persons skilled in the art.

By way of illustration, when the marker is an enzyme, the detection of the complex in question can be carried out by contacting said complex with a suitable substrate and, optionally, with suitable enzymatic amplification agents and/or activators. Illustrative non-limiting examples of said substrates include:
- For the alkaline phosphatase:
   Chromogenic: substrates based on p-nitrophenyl phosphate (p-NPP), 5-bromo-4-chloro-3-indolyl phosphate/nitroblue tetrazolium (BCIP/NPT), etc. Fluorogenic: 4-methylumbeliphenyl phosphate (4-MUP), 2-(5'-chloro-2'-phosphoryloxyphenyl)-6-chloro-4-(3H)-quinazolinone (CPPCQ), 3, 6-fluorescein-diphosphate (3,6-FDP), etc.
- For peroxidases:
   Chromogenic: substrates based on 2,2-azinobis(3-ethylbenzothiazolin-6-sulfonic) (ABTS) acid, o-phenylendiamine (OPT), 3,3',5,5'-tetramethylbenzidine (TMB), o-dianisidine, 5-aminosalicylic acid, 3-dimethylaminobenzoic (DMAB) acid and 3-methyl-2-benzothiazolinhydrazone (MBTH), 3-amino-9-ethylcarbazol (AEC) and 3,3'-diaminobenzidine (DAB) tetrachloride, etc.
   Fluorogenic: 4-hydroxy-3-methoxyphenylacetic acid, reduced phenoxazines and reduced benzothiazines, including the reagent Amplex^{®} Red, Amplex UltraRed, reduced dihydroxanthenes, etc.
- For glycosidases:
   Chromogenic: substrates based on o-nitrophenyl-β-D-galactoside (o-NPG), p-nitrophenyl-β-D-galactoside and 4-methylumbeliphenyl-β-D-galactoside (MUG) for β-D-galactosidase, etc.
   Fluorogenic: resorufin β-D-galactopyranoside, fluorescein digalactoside (FDG), fluorescein diglucuronide, 4-methylumbeliferyl beta-D-galactopyranoside, carboxyumbeliferyl beta-D-galactopyranoside, fluorinated coumarin beta-D-galactopyranosides, etc.

In a particular embodiment, said marker is a peroxidase, such as a peroxidase and the chromogenic substrate is TMB.

Therefore, by means of putting into practice the method of detection of autoantibodies, it is possible to detect and obtain an autoantibody selected from the group consisting of an autoantibody to the Pim1 protein, an autoantibody to the SRC protein, an autoantibody to the MAPKAPK3 protein, an autoantibody to the FGFR4 protein, an autoantibody to the STK4 protein, an autoantibody to the ACVR2B protein, and combinations of said autoantibodies. In a particular embodiment, the autoantibodies identified by means of the method of detection of autoantibodies are specific, i.e., they recognize the protein in question (or fragment thereof susceptible of being recognized by said autoantibody) with a preference over other proteins or fragments of 2 or more times, more than 3 times, more than 10 times, more than 20 times, more than 100 times, or even a greater number of times.

Optionally, if desired, the autoantibody-protein, or fragment thereof, complex susceptible of being recognized by said autoantibody formed, for example, by means of using immunoprecipitation techniques, etc., can be isolated, and the sequence of the autoantibody responsible for binding to the protein or fragment thereof susceptible of being recognized by said autoantibody can be subsequently sequenced by means of the use of standard proteomic methods described in the art, such as the determination of the peptide fingerprint or MS/MS analysis (Vikas Dhingraa, et al. 2005. International Journal of Pharmaceutics 299 (1-2): pp. 1-18.; Hanash SM et al. Nature. 2008 Apr 3; 452(7187):571-9).

The method for the detection of autoantibodies can also be used for determining the level or amount (quantifying) of autoantibodies to said proteins (Pim1, SRC, MAPKAPK3, FGFR4, STK4 and/or ACVR2B) present in the biological sample under study because, with many markers, e.g., enzymes, the amount of autoantibody present in the biological sample is proportional to the generated signal.

The detection of the autoantibody-protein, or fragment thereof, complex susceptible of being recognized by said autoantibody is indicative of the presence of autoantibodies specific to said protein/proteins in the biological sample and, furthermore, if desired, the amount of autoantibodies to said proteins present in said biological sample can be quantified. Within the context of the present invention, said information can be used in the diagnosis, prognosis or tracking of the progress of diseases, particularly colorectal cancer (CRC), in a subject.

### Methods of obtaining Data

The description also describes a method of obtaining data in a biological sample from a subject, hereinafter "method of obtaining data 1", which comprises detecting at least one autoantibody to a protein, wherein said autoantibody is selected from the group consisting of an autoantibody to the Pim1 protein, an autoantibody to the SRC protein, an autoantibody to the MAPKAPK3 protein, an autoantibody to the FGFR4 protein, an autoantibody to the STK4 protein, and an autoantibody to the ACVR2B protein, and, if desired, determining the level of said autoantibody in said biological sample.

By means of the method of obtaining data 1 the level of one or more of the aforementioned autoantibodies can be detected and identified, and, if desired, quantified.

The detection of said autoantibodies can be carried out by conventional methods known by persons skilled in the art; in a particular embodiment, the detection of said autoantibodies is carried out by means of an immunoassay (e.g., immunoblot, ELISA, LIA, RIA, IF, IHQ, protein microarrays, etc.), such as an immunoassay suitable for detecting and identifying said autoantibodies to the Pim1, SRC, MAPKAPK3, FGFR4, STK4 and/or ACVR2B proteins, for example, as it has been described in relation to the method of detection of autoantibodies of the invention. In a particular embodiment, said immunoassay is a protein microarray or an ELISA.

A protein microarray consists of a collection of proteins immobilized on a solid support in a regular and pre-established arrangement. There are several important factors to be taken into account in the design of protein microarrays, which include, for example, the nature of the support on which the proteins (or suitable fragments thereof) are immobilized, the technique of immobilizing the proteins, the format of the microarray, the capture agent used or the method of detection to be used. Different formats, supports and techniques which can be used for performing this inventive aspect are known in the state of the art.

In a particular embodiment, the detection of autoantibodies to Pim1, SRC, MAPKAPK3, FGFR4, STK4 and/or ACVR2B, by means of protein microarrays comprises the following steps: (a) covering a solid support with one or more proteins, preferably separated from one another, selected from the group consisting of the Pim1, SRC, MAPKAPK3, FGFR4, STK4 and ACVR2B proteins, or with fragments thereof susceptible of being recognized by the autoantibodies to the corresponding proteins; (b) incubating the covered support of step (a) with a biological sample from a subject under conditions which allow the formation of an immunocomplex of the autoantibody to the Pim1, SRC, MAPKAPK3, FGFR4, STK4 or ACVR2B protein present in said sample with the corresponding antigenic determinants present in said Pim1, SRC, MAPKAPK3, FGFR4, STK4 or ACVR2B proteins, or in their fragments susceptible of being recognized by said autoantibodies; and (c) adding a secondary antibody, which recognizes the autoantibody to the Pim1, SRC, MAPKAPK3, FGFR4, STK4 or ACVR2B protein, conjugated or bound to a marker compound.

The ELISA is based on the premise that an immunoreagent (antigen of the biological sample or antibody) can be immobilized on a solid support, later contacting this system with a fluid phase which contains the complementary reagent which can be bound to a marker compound. There are different types of ELISA: direct ELISA, indirect ELISA or sandwich ELISA.

In another particular embodiment, the detection of autoantibodies to Pim1, SRC, MAPKAPK3, FGFR4, STK4 and/or ACVR2B, is carried out by means of an ELISA, preferably, by means of an indirect ELISA, which comprises the following steps: (a) covering a solid support with one or more proteins, preferably separated from one another, selected from the group consisting of the Pim1, SRC, MAPKAPK3, FGFR4, STK4 and ACVR2B proteins, or with fragments thereof susceptible of being recognized by the autoantibodies to the corresponding proteins; (b) incubating the covered support of step (a) with a biological sample from a subject under conditions which allow the formation of an immunocomplex of the autoantibody to the Pim1, SRC, MAPKAPK3, FGFR4, STK4 or ACVR2B protein present in said biological sample with the corresponding antigenic determinants present in said Pim1, SRC, MAPKAPK3, FGFR4, STK4 or ACVR2B proteins, or in their fragments susceptible of being recognized by said autoantibodies; and (c) adding a secondary antibody, which recognizes the autoantibody to the Pim1, SRC, MAPKAPK3, FGFR4, STK4 or ACVR2B protein, conjugated or bound to a marker.

As previously mentioned, said marker is a compound capable of giving rise to a chromogenic, fluorogenic, radioactive and/or chemoluminescent signal which allows the detection, identification and, optionally, quantification of the amount of the autoantibody to the Pim1, SRC, MAPKAPK3, FGFR4, STK4 or ACVR2B protein present in the analyzed sample. In a particular embodiment, said marker compound is selected from the group consisting of radioisotopes, enzymes, fluorophores or any molecule susceptible of being conjugated with another molecule or detected and/or quantified directly. This marker compound can bind to the autoantibody directly, or through another compound. Illustrative non-limiting examples, of said marker compounds which bind directly to the autoantibody include enzymes, such as alkaline phosphatase, peroxidase, etc., radioactive isotopes, such as ³²P, ³⁵S, etc., fluorochromes, such as fluoresceine, etc., or metal particles, for their direct detection by means of colorimetry, auto-radiography, fluorometry, or metallography, respectively.

In a particular embodiment, the method of obtaining data 1 comprises, in addition to detecting at least one autoantibody selected from the group of autoantibodies formed by autoantibodies to the Pim1 protein, an autoantibody to the SRC protein, an autoantibody to the MAPKAPK3 protein, an autoantibody to the FGFR4 protein, an autoantibody to the STK4 protein, and an autoantibody to the ACVR2B protein, the step of determining the level or amount (quantifying) of said autoantibody in said biological sample under study since, with many markers, e.g., enzymes, the amount of autoantibody present in the biological sample is proportional to the generated signal. In this case, the signal obtained using the different methods described above for detecting the autoantibodies can be analyzed and quantified by conventional methods which allow the quantification of said signal.

The method for obtaining data 1 can also be used for determining the amount (quantifying) of autoantibodies to said proteins (Pim1, SRC, MAPKAPK3, FGFR4, STK4 and/or ACVR2B) present in the biological sample under study since, with many markers, e.g., enzymes, the amount of autoantibody present in the biological sample is proportional to the generated signal.

The detection of the autoantibody-protein, or fragment thereof, complex susceptible of being recognized by said autoantibody is indicative of the presence of autoantibodies specific to said protein/proteins in the biological sample and, furthermore, if desired, the amount of autoantibodies to said proteins present in said biological sample can be quantified. Within the context of the present invention, said information can be used in the diagnosis, prognosis or tracking of the progress of diseases, particularly colorectal cancer (CRC), in a subject.

In a particular embodiment, only the level of autoantibodies to a single protein, for example, to the Pim1, SRC, MAPKAPK3, FGFR4, STK4 or ACVR2B protein, is detected and optionally quantified. In another particular embodiment, the level of autoantibodies to two or more proteins of the group consisting of the Pim1, SRC, MAPKAPK3, FGFR4, STK4 and ACVR2B proteins is detected and optionally quantified. By way of illustration, combinations of 2, 3, 4, 5 or 6 autoantibodies to the selected proteins of the group consisting of the Pim1, SRC, MAPKAPK3, FGFR4, STK4 and ACVR2B proteins can be detected and, if desired, quantified. Thus, by way of illustration, the level of autoantibodies to the combinations of said proteins mentioned in the following List of Combinations can be detected and optionally quantified:

### List of Combinations (proteins)

Pim1, SRC
Pim1, MAPKAPK3
Pim1, FGFR4
Pim1, STK4
Pim1, ACVR2B
SRC, MAPKAPK3
SRC, FGFR4
SRC, STK4
SRC, ACVR2B
MAPKAPK3, FGFR4
MAPKAPK3, STK4
MAPKAPK3, ACVR2B
FGFR4, STK4
FGFR4, ACVR2B
STK4, ACVR2B
Pim1, SRC, MAPKAPK3
Pim1, SRC, FGFR4
Pim1, SRC, STK4
Pim1, SRC, ACVR2B
Pim1, MAPKAPK3, FGFR4
Pim1, MAPKAPK3, STK4
Pim1, MAPKAPK3, ACVR2B
Pim1, FGFR4, STK4
Pim1, FGFR4, ACVR2B
Pim1, STK4, ACVR2B
SRC, MAPKAPK3, FGFR4
SRC, MAPKAPK3, STK4
SRC, MAPKAPK3, ACVR2B
SRC, FGFR4, STK4
SRC, FGFR4, ACVR2B
SRC, STK4, ACVR2B
MAPKAPK3, FGFR4, STK4
MAPKAPK3, FGFR4, ACVR2B
MAPKAPK3, STK4, ACVR2B
FGFR4, STK4, ACVR2B
Pim1, SRC, MAPKAPK3, FGFR4
Pim1, SRC, MAPKAPK3, STK4
Pim1, SRC, MAPKAPK3, ACVR2B
Pim1, SRC, FGFR4, STK4
Pim1, SRC, FGFR4, ACVR2B
Pim1, SRC, STK4, ACVR2B
Pim1, MAPKAPK3, FGFR4, STK4
Pim1, MAPKAPK3, FGFR4, ACVR2B
Pim1, MAPKAPK3, STK4, ACVR2B
Pim1, FGFR4, STK4, ACVR2B
SRC, MAPKAPK3, FGFR4, STK4
SRC, MAPKAPK3, FGFR4, ACVR2B
SRC, MAPKAPK3, STK4, ACVR2B
SRC, FGFR4, STK4, ACVR2B
MAPKAPK3, FGFR4, STK4, ACVR2B
Pim1, SRC, MAPKAPK3, FGFR4, STK4
Pim1, SRC, MAPKAPK3, FGFR4, ACVR2B
Pim1, SRC, MAPKAPK3, STK4, ACVR2B
Pim1, SRC, FGFR4, STK4, ACVR2B
Pim1, MAPKAPK3, FGFR4, STK4, ACVR2B
SRC, MAPKAPK3, FGFR4, STK4, ACVR2B
Pim1, SRC, MAPKAPK3, FGFR4, STK4, ACVR2B

In a particular embodiment, the level of autoantibodies to the Pim1 protein is detected and optionally quantified; in another particular embodiment, the level of autoantibodies to the Pim1 protein is detected and optionally quantified, and, furthermore, the level of autoantibodies to one or more of the following SRC, MAPKAPK3, FGFR4, STK4 and/or ACVR2B proteins, according to the previously mentioned combinations. Additionally, if desired, the level of autoantibodies to other proteins, for example, to proteins potentially useful in the diagnosis of CRC, such as CEA, etc., can be detected and optionally determined.

In another particular embodiment, the level of autoantibodies to the SRC protein is detected and optionally quantified; in another particular embodiment, the level of autoantibodies to the SRC protein is detected and optionally quantified, and, furthermore, the level of autoantibodies to one or more of the following Pim1, MAPKAPK3, FGFR4, STK4 and/or ACVR2B proteins, according to the previously mentioned combinations. Additionally, if desired, the level of autoantibodies to other proteins, for example, to proteins potentially useful in the diagnosis of CRC, such as CEA, etc., can be detected and optionally determined.

In a particular embodiment, the level of autoantibodies to the MAPKAPK3 protein is detected and optionally quantified; in another particular embodiment, the level of autoantibodies to the MAPKAPK3 protein is detected and optionally quantified, and, furthermore, the level of autoantibodies to one or more of the following Pim1, SRC, FGFR4, STK4 and/or ACVR2B proteins, according to the previously mentioned combinations. Additionally, if desired, the level of autoantibodies to other proteins, for example, to proteins potentially useful in the diagnosis of CRC, such as CEA, etc., can be detected and optionally determined.

In a particular embodiment, the level of autoantibodies to the FGFR4 protein is detected and optionally quantified; in another particular embodiment, the level of autoantibodies to the FGFR4 protein is detected and optionally quantified, and, furthermore, the level of autoantibodies to one or more of the following Pim1, SRC, MAPKAPK3, STK4 and/or ACVR2B proteins, according to the previously mentioned combinations. Additionally, if desired, the level of autoantibodies to other proteins, for example, to proteins potentially useful in the diagnosis of CRC, such as CEA, etc., can be detected and optionally determined.

In a particular embodiment, the level of autoantibodies to the STK4 protein is detected and optionally quantified; in another particular embodiment, the level of autoantibodies to the STK4 protein is detected and optionally quantified, and, furthermore, the level of autoantibodies to one or more of the following Pim1, SRC, MAPKAPK3, FGFR4, and/or ACVR2B proteins, according to the previously mentioned combinations. Additionally, if desired, the level of autoantibodies to other proteins, for example, to proteins potentially useful in the diagnosis of CRC, such as CEA, etc., can be detected and optionally determined.

In a particular embodiment, the level of autoantibodies to the ACVR2B protein is detected and optionally quantified; in another particular embodiment, the level of autoantibodies to the ACVR2B protein is detected and optionally quantified, and, furthermore, the level of autoantibodies to one or more of the following Pim1, SRC, MAPKAPK3, FGFR4 and/or STK4 proteins, according to the previously mentioned combinations. Additionally, if desired, the level of autoantibodies to other proteins, for example, to proteins potentially useful in the diagnosis of CRC, such as CEA, etc., can be detected and optionally determined.

In a particular embodiment, the level of autoantibodies to the Pim1 protein, the level of autoantibodies to the MAPKAPK3 protein or the level of autoantibodies to the ACVR2B protein, preferably, the level of autoantibodies to the MAPKAPK3 protein or the level of autoantibodies to the ACVR2B protein is detected and optionally quantified.

In another particular embodiment, the level of autoantibodies to the MAPKAPK3 protein and the level of autoantibodies to the ACVR2B protein, and, optionally, the level of autoantibodies to the FGFR4 protein are detected and optionally quantified.

In another particular embodiment, the level of autoantibodies to the Pim1 protein, the level of autoantibodies to the MAPKAPK3 protein and the level of autoantibodies to the ACVR2B protein, and, optionally, the level of autoantibodies to the FGFR4 protein are detected and optionally quantified.

Within the context of the present invention, the data obtained according to the method of obtaining data 1 relating to the detection and, optionally, quantification of autoantibodies to one or more of the Pim1, SRC, MAPKAPK3, FGFR4, STK4 and/or ACVR2B proteins, can be used in the diagnosis, prognosis or tracking of the progress of diseases, particularly colorectal cancer (CRC), in a subject.

In another aspect, the invention relates to a method of obtaining data in a biological sample from a subject, hereinafter "method of obtaining data 2 of the invention", which comprises detecting the expression product of the Pim1 gene and the expression product of at least one gene, wherein said gene is selected from the group consisting of the *SRC, MAPKAPK3, FGFR4, STK4* and *ACVR2B* genes, and, if desired, quantifying the level of expression of said expression product of said gene in said biological sample wherein said sample comprises colorectal (CRC) tumor cells.

By means of the method of obtaining data 2 of the invention the level of an expression product of the Pim1 gene and the level of an expression product of one or more of the aforementioned genes can be detected and identified and, if desired, quantified, thus allowing the possibility of establishing the presence or absence of an expression product of the Pim1 gene and the presence or absence of an expression product of one or more of the *SRC, MAPKAPK3, FGFR4, STK4* and/or *ACVR2B* genes, and, where appropriate, if desired, quantifying the level of expression of said expression product.

The biological sample used for putting into practice the method of obtaining data 2 of the invention is a biological sample comprising CRC tumor cells. By way of non-limiting illustration, said biological sample comprising tumor cells can be a sample of a biological fluid or, preferably, a sample of a tissue, e.g., a tumor biopsy, a fine needle aspiration biopsy, etc. The biological sample can be, for example but not limited to, fresh, frozen, fixed or embedded in paraffin.

In a particular embodiment, said biological sample is a tumor biopsy which comprises CRC tumor cells from a patient suffering CRC or a colon or rectal tissue biopsy from a subject under study for the purpose, for example, of evaluating whether or not he/she suffers CRC.

The methods for the detection and quantification of an expression product of a gene are widely known by a person skilled in the art and include a number of alternatives. Virtually any method which allows the detection, and, if desired, the quantification, of an expression product of a gene selected from the group of genes consisting of the *Pim1* and one or more of the *SRC, MAPKAPK3, FGFR4, STK4* and *ACVR2B* genes, can be used in putting into practice the method of obtaining data 2 of the invention.

Thus, in a particular embodiment, the detection of the expression product of a determined gene (e.g., *Pim1* and one or more of the *SRC*, *MAPKAPK3, FGFR4, STK4* or *ACVR2B*) is carried out by analyzing the level of mRNA derived from its transcription; in this case, the analysis of the level of mRNA can be performed, by way of non-limiting illustration, by means of an enzymatic amplification process, for example, by means of the polymerase chain reaction (PCR), reverse transcription combined with the polymerase chain reaction (RT-PCR), reverse transcription combined with the ligase chain reaction (RT-LCR), or any other method of nucleic acid amplification; DNA microarrays prepared with oligonucleotides deposited by any mechanism; DNA microarrays prepared with oligonucleotides synthesized *in situ* by means of photolithography or by any other mechanism; in situ hybridization using specific probes labeled with any labeling method; by means of electrophoresis gels; by means of membrane transfer and hybridization with a specific probe; by means of nuclear magnetic resonance or any other diagnostic imaging technique using paramagnetic nanoparticles or any other type of detectable nanoparticles functionalized with antibodies or by any other means. Additionally, this method of obtaining data 2 of the invention can include performing an extraction step for the purpose of obtaining the total RNA, which can be done by means of conventional techniques (Chomczynski et al., Anal. Biochem., 1987, 162:156; Chomczynski P., Biotechniques, 1993, 15:532). Additional information on methods for detecting and quantifying the levels of expression of an expression product of a gene can be found, for example, in Sambrook et al., 2001 "Molecular cloning: a Laboratory Manual", 3rd ed., Cold Spring Harbor Laboratory Press, N.Y., Vol. 1-3. In a particular embodiment of the method of obtaining data 2 of the invention, the quantification of the levels of expression of the genes identified above (*Pim1* and one or more of *SRC, MAPKAPK3, FGFR4, STK4* or *ACVR2B*) is carried out by means of multiplex quantitative PCR or by means of a DNA or RNA array.

In another particular embodiment, the detection, and, optionally, quantification of the level of expression of said expression product of the *Pim1* gene and one or more of the *SRC, MAPKAPK3, FGFR4, STK4* and *ACVR2B* genes in the sample to be analyzed is performed by analyzing the level of the Pim1 protein and one or more of the SRC, MAPKAPK3, FGFR4, STK4 and ACVR2B proteins, or fragments thereof; in this case, the analysis of the level of said proteins can be performed, by way of non-limiting illustration, by means of an immunoassay, by means of nuclear magnetic resonance or by means of any other suitable technique known in the state of the art. In a preferred embodiment, the determination of the amount of the Pim1 protein and one or more of the SRC, MAPKAPK3, FGFR4, STK4 or ACVR2B proteins, or of their fragments, is performed by means of an immunoassay.

In a particular preferred embodiment, said immunoassay is an immunoblot (Western blot or membrane immunodetection). To that end, in summary, a protein extract is obtained from a biological sample isolated from a subject and the proteins are separated by means of electrophoresis in a support medium capable of retaining them. Once the proteins are separated, they are transferred to a different support or membrane where they can be detected by means of using specific antibodies which recognize the proteins in question (e.g., Pim1, SRC, MAPKAPK3, FGFR4, STK4 or ACVR2B) or the functionally equivalent fragments thereof. Said membrane is hybridized with a first specific antibody (or primary antibody) which recognizes the protein in question (e.g., Pim1, SRC, MAPKAPK3, FGFR4, STK4 or ACVR2B) or a functionally equivalent fragment thereof. The membrane is then hybridized with a second antibody (or secondary antibody) capable of specifically recognizing the primary antibody and which is conjugated or bound to a marker compound. In an alternative embodiment, it is the antibody which recognizes the Pim1, SRC, MAPKAPK3, FGFR4, STK4 or ACVR2B protein, or the functionally equivalent fragment thereof, which is conjugated or bound to a marker compound, and the use of a secondary antibody is not necessary. Different formats, supports and techniques which can be used for performing this preferred aspect of the method of obtaining data 2 of the invention are known.

In another particular preferred embodiment, the immunoassay comprises an immunohistochemical assay. The immunohistochemistry techniques allow the identification, on tissue or cytological samples, of characteristic antigenic determinants. The analysis by means of immunohistochemistry (IHQ) is performed on tissue sections, either frozen or included in paraffin, from a biological sample isolated from a subject. These sections are hybridized with a specific antibody or primary antibody which recognizes specific antibodies which recognize the Pim1, SRC, MAPKAPK3, FGFR4, STK4 or ACVR2B proteins, or functionally equivalent fragments thereof. The sections are then hybridized with a secondary antibody capable of specifically recognizing the primary antibody and which is conjugated or bound to a marker compound. In an alternative embodiment, it is the antibody which recognizes the Pim1, SRC, MAPKAPK3, FGFR4, STK4 or ACVR2B protein, or the functionally equivalent fragment thereof, that is conjugated or bound to a marker compound, and the use of a secondary antibody is not necessary.

In a particular embodiment, the level of an expression product of two or more genes of the group consisting of the *Pim1, SRC, MAPKAPK3, FGFR4 STK4* and *ACVR2B* genes wherein one of said expression products is an expression product of the Pim1 gene is detected and optionally quantified. By way of illustration, expression products of 2, 3, 4, 5 or 6 of said genes can be detected and, if desired, quantified , wherein one of said expression products is an expression product of the Pim1 gene.

In a particular embodiment, the level of an expression product of the *Pim1* gene, the level of an expression product of the *MAPKAPK3* gene or the level of an expression product of the *ACVR2B* gene, preferably, the level of an expression product of the *MAPKAPK3* gene or the level of an expression product of the *ACVR2B* gene is detected and optionally quantified.

In another particular embodiment, the level of an expression product of the *MAPKAPK3* gene and the level of an expression product of the *ACVR2B* gene, and, optionally the level of an expression product of the *FGFR4* gene are detected and optionally quantified.

In another particular embodiment, the level of an expression product of the *Pim1* gene, the level of an expression product of the *MAPKAPK3* gene and the level of an expression product of the *ACVR2B* gene, and, optionally the level of an expression product of the *FGFR4* gene are detected and optionally quantified.

Within the context of the present invention, the data obtained according to the method of obtaining data 2 of the invention, relating to the detection and, optionally, quantification of expression products of the Pim1 gene and one or more of the *SRC, MAPKAPK3, FGFR4, STK4* and/or *ACVR2B* genes, can be used in the diagnosis, prognosis or tracking of the progress of diseases, particularly colorectal cancer (CRC), in a subject.

### Methods of Diagnosis

In another aspect, the invention relates to a method for diagnosing if a subject suffers colorectal cancer (CRC), hereinafter "method of diagnosis 1 of the invention", which comprises comparing the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from said subject, with the reference level for said autoantibodies wherein if the level of said autoantibody to the Pim1 protein in said sample is greater than the corresponding reference level for said autoantibody, and wherein if the level, of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said subject is diagnosed with CRC.

The method of diagnosis 1 of the invention comprises previously determining the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from the subject in question. In a particular embodiment, said biological sample is a blood, plasma or serum sample from said subject. The level of said autoantibodies can be determined as has been previously indicated in relation to the method of detection of autoantibodies of the invention or with the method of obtaining data 1 of the inventions.

Once the level of the autoantibodies to the Pim1 protein and the level of one or more of the autoantibodies to the SRC, MAPKAPK3, FGFR4, STK4, and/or ACVR2B proteins is determined in said biological sample, the method of diagnosis 1 of the invention comprises comparing the level of said autoantibody (or autoantibodies) with the reference level for said autoantibody (or with the reference levels for the autoantibodies in question), wherein if the level of said autoantibody to the Pim1 protein in said sample is greater than the corresponding reference level for said autoantibody, and wherein if the level, of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said subject is diagnosed with CRC.

In a particular embodiment of the invention, said reference level is the level or amount of autoantibodies to said proteins (Pim1, SRC, MAPKAPK3, FGFR4, STK4, and ACVR2B) in a control sample, such as for example, a blood, serum or plasma sample, from a population of control subjects (i.e., who do not suffer CRC). It will generally be considered that the level of an autoantibody to a protein (e.g., Pim1, SRC, MAPKAPK3, FGFR4 or STK4) in the biological sample from the subject under study is "greater" than the reference level of said autoantibody when the level of said autoantibody in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, the reference level of said autoantibody. Similarly, it will generally be considered that the level of an autoantibody to a protein (e.g., ACVR2B) in the biological sample from the subject under study is "less" than the reference level of said autoantibody when the level of said autoantibody in the biological sample from the subject is at least 1.5 times, 5 times, 10 times, 20 times, 30 times, 40 times, 50 times, 60 times, 70 times, 80 times, 90 times, 100 times, or even more, lower than the reference level of said autoantibody.

In another particular embodiment, the level of autoantibodies to two or more proteins of the group consisting of the Pim1, SRC, MAPKAPK3, FGFR4, STK4 and ACVR2B proteins wherein at least one of the proteins is Pim1, in the sample from the subject to be analyzed is quantified and the levels obtained are compared with the reference levels of the autoantibodies to the corresponding proteins. By way of illustration, the autoantibodies to 2, 3, 4, 5 and 6 selected proteins of the group consisting of the Pim1, SRC, MAPKAPK3, FGFR4, STK4 and ACVR2B proteins, as is mentioned in the aforementioned List of Combinations, can be quantified.

Thus, in a particular embodiment, the level of autoantibodies to the Pim1 protein, and the level of autoantibodies to the MAPKAPK3 protein or the level of autoantibodies to the ACVR2B protein, preferably, the level of autoantibodies to the MAPKAPK3 protein or the level of autoantibodies to the ACVR2B protein is quantified and compared with its reference level.

In another particular embodiment, the level of autoantibodies to the Pim1 protein, the level of autoantibodies to the MAPKAPK3 protein and the level of autoantibodies to the ACVR2B protein, and, optionally, the level of autoantibodies to the FGFR4 protein are quantified and compared with their reference level.

The method of diagnosis 1 of the invention allows diagnosing if a subject suffers CRC with a high degree of reliability since it allows correctly detecting said disease (CRC) in at least 60%, at least 70%, at least 80%, or at least 90% of the subjects of a determined group or population analyzed. Said method can be used in any stage of CRC. In a particular embodiment, the subject is a patient suffering CRC in the initial stages, such as stages 0, I and II.

In another aspect, the invention relates to a method for diagnosing if a subject suffers colorectal cancer (CRC), hereinafter "method of diagnosis 2 of the invention", which comprises comparing the level of expression of of an expression product of the Pim1 gene and the level of expression of at least one expression product of a gene, wherein said gene is selected from the group consisting of the *SRC, MAPKAPK3, FGFR4, STK4* and *ACVR2B* genes, in a sample from said subject, with the reference level for said expression product of said genes, wherein if the level of said expression product of the *Pim1* gene is greater than the corresponding reference level for said expression product of said gene and the level, of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, said subject is diagnosed with CRC.

The method of diagnosis 2 of the invention comprises previously determining the level of expression of an expression product (e.g., RNA or protein) of the Pim1 gene and at least one expression product of a gene selected from the group consisting of the *SRC, MAPKAPK3, FGFR4, STK4* and *ACVR2B* genes, in a biological sample from the subject in question. In a particular embodiment, said biological sample is a colon or rectal tissue sample, or of tumor tissue (where appropriate), blood, plasma or serum from said subject. The level of said expression product can be determined, depending on its nature (RNA or protein), as has been previously indicated in relation to the method of obtaining data 2 of the invention.

Once the level of expression of the expression product of the Pim1 gene and of one or more of the expression products of the *SRC, MAPKAPK3, FGFR4, STK4* and *ACVR2B* genes is determined in said biological sample, the method of diagnosis 2 of the invention comprises comparing the level of said expression product (or expression products) with the reference level for said expression product (or with the reference levels for the expression products in question), wherein if the level of said expression product of the *Pim1* gene is greater than the corresponding reference level for said expression product of said gene and the level, of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, then said subject is diagnosed with CRC.

In a particular embodiment of the invention, said reference level is the level or amount of expression product of said gene (*Pim1, SRC, MAPKAPK3, FGFR4, STK4,* and *ACVR2B*) in a biological sample, preferably of the colon, of a population of control subjects (i.e., subjects who do not suffer CRC). It will generally be considered that the level of an expression product of a gene (e.g., *Pim1, SRC, MAPKAPK3, FGFR4* or *STK4*) in the sample from the subject under study is "greater" than the reference level of said expression product when the relationship between the level of the expression product of the gene in question determined in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, the reference level of said expression product of said gene. Similarly, it will generally be considered that the level of an expression product of a gene (e.g., *ACVR2B*) in the biological sample from the subject under study is "less" than the reference level of said expression product of said gene when the level of said autoantibody in the biological sample from the subject is at least 1.5 times, 5 times, 10 times, 20 times, 30 times, 40 times, 50 times, 60 times, 70 times, 80 times, 90 times, 100 times, or even more, lower than the reference level of said expression product of said gene.

In another particular embodiment, the levels of expression of expression products of two or more genes of the group consisting of the *Pim1, SRC, MAPKAPK3, FGFR4, STK4* or *ACVR2B* genes wherein one of the genes is the Pim1 gene, in the sample from the subject to be analyzed are quantified and the levels obtained are compared with the corresponding reference levels of said expression products of the genes in question. By way of illustration, expression products of 2, 3, 4, 5 and 6 genes selected from the group consisting of the *Pim1, SRC, MAPKAPK3, FGFR4, STK4* and *ACVR2B* genes, such as, for example, the following expression products can be quantified:

### List of Combinations of Genes

*Pim1, SRC*
*Pim1, MAPKAPK3*
*Pim1, FGFR4*
*Pim1, STK4*
*Pim1, ACVR2B*
*Pim1, SRC, MAPKAPK3*
*Pim1, SRC, FGFR4*
*Pim1, SRC, STK4*
*Pim1, SRC, ACVR2B*
*Pim1, MAPKAPK3, FGFR4*
*Pim1, MAPKAPK3, STK4*
*Pim1, MAPKAPK3, ACVR2B*
*Pim1, FGFR4, STK4*
*Pim1, FGFR4, ACVR2B*
*Pim1, STK4, ACVR2B*
*Pim1, SRC, MAPKAPK3, FGFR4*
*Pim1, SRC, MAPKAPK3, STK4*
*Pim1, SRC, MAPKAPK3, ACVR2B*
*Pim1, SRC, FGFR4, STK4*
*Pim1, SRC, FGFR4, ACVR2B*
*Pim1, SRC, STK4, ACVR2B*
*Pim1, MAPKAPK3, FGFR4, STK4*
*Pim1, MAPKAPK3, FGFR4, ACVR2B*
*Pim1, MAPKAPK3, STK4, ACVR2B*
*Pim1, FGFR4, STK4, ACVR2B*
*Pim1, SRC, MAPKAPK3, FGFR4, STK4*
*Pim1, SRC, MAPKAPK3, FGFR4, ACVR2B*
*Pim1, SRC, MAPKAPK3, STK4, ACVR2B*
*Pim1, SRC, FGFR4, STK4, ACVR2B*
*Pim1, MAPKAPK3, FGFR4, STK4, ACVR2B*
*Pim1, SRC, MAPKAPK3, FGFR4, STK4, ACVR2B*

In a particular embodiment, the level of an expression product of the *Pim1* gene and the level of an expression product of the *MAPKAPK3* gene or the level of an expression product of the *ACVR2B* gene, preferably, the level of an expression product of the *MAPKAPK3* gene or the level of an expression product of the *ACVR2B* gene is quantified and compared with their reference levels.

In another particular embodiment, the level of an expression product of the *Pim1* gene, the level of an expression product of the *MAPKAPK3* gene and the level of an expression product of the *ACVR2B* gene, and, optionally the level of an expression product of the *FGFR4* gene are quantified and compared with their reference level.

The method of diagnosis 2 of the invention allows diagnosing if a subject suffers CRC with a high degree of reliability since it allows correctly detecting said disease (CRC) in at least 60%, at least 70%, at least 80%, or at least 90% of the subjects of a determined group or population analyzed. Said method can be used in any stage of the CRC. In a particular embodiment, the subject is a patient suffering CRC in initial stages, such as stages 0, I and II.

### Methods of Prognosis/Tracking

The teachings of the present invention are also useful for predicting or evaluating the response to a determined treatment. The main treatment of CRC is typically surgical treatment (surgery), for example but not limited to, by means of local excision or resection. Some patients with CRC before surgery receive "neoadjuvant" therapy for the purpose of reducing the size of the CRC, in order to enable or facilitate the surgery. After the surgery, many patients receive adjuvant therapy for the purpose of preventing the relapse of the cancer in the colon, in the rectum or in other site. In the treatment of CRC, adjuvant or neoadjuvant therapy can consist, for example but not limited to, of radiotherapy, chemotherapy or biological therapy. Some examples of compounds used in chemotherapy or biological therapy include but are not limited to folic acid, fluorouracil, irinotecan, oxaliplatin, leucovorin, levamisole, cetuximab or bevacizumab.

Therefore, in another aspect, the invention relates to a method for evaluating the prognosis or tracking of the progress of a patient suffering colorectal cancer (CRC), hereinafter "method of prognosis 1 of the invention", which comprises comparing the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from said patient suffering CRC, with the reference level for said autoantibody, wherein if the level of said autoantibody to the Pim1 protein, is greater than the corresponding reference level for said autoantibody, and wherein if the level of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said patient suffers a CRC with a poor prognosis or presents a CRC with an unfavorable progress. The method of prognosis 1 of the invention comprises previously determining the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from the patient suffering CRC in question. In a particular embodiment, said biological sample is a blood, plasma or serum sample from said patient suffering CRC for the purpose of evaluating the tracking of the progress of the disease (CRC). The level of said autoantibodies can be determined as has been previously indicated in relation to the method of detection of autoantibodies of the invention or with the method of obtaining data 1 of the invention.

Once the level of autoantibodies to the Pim1 protein and the level of one or more of the autoantibodies to the SRC, MAPKAPK3, FGFR4, STK4, and/or ACVR2B proteins, in said biological sample is determined, the method of prognosis 1 of the invention comprises comparing the level of said autoantibody (or autoantibodies) with the reference level for said autoantibodies (or with the reference levels for the autoantibodies in question), wherein if the level of said autoantibody to the Pim1 protein is greater to the corresponding reference level for said autoantibody, and wherein if the level of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said patient suffers a CRC with a poor prognosis or presents a CRC with an unfavorable progress.

In a particular embodiment of the invention, said reference level is the level or amount of autoantibodies to said proteins (Pim1, SRC, MAPKAPK3, FGFR4, STK4, and ACVR2B) in a sample, preferably of serum, from subjects who do not present CRC. In another particular embodiment of the invention, said reference level is the level or amount of autoantibodies to said proteins (Pim1, SRC, MAPKAPK3, FGFR4, STK4, and ACVR2B) in a sample, preferably of serum, from the same patient suffering CRC previously obtained, for example, before the administration of a treatment for CRC, for the purpose of being able to evaluate the effectiveness of said treatment. It will generally be considered that the level of an autoantibody to a protein (e.g., Pim1, SRC, MAPKAPK3, FGFR4 or STK4) in the biological sample from the subject under study is "greater" than the reference level of said autoantibody when the level of said autoantibody in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, the reference level of said autoantibody. Similarly, it will generally be considered that the level of an autoantibody to a protein (e.g., ACVR2B) in the biological sample from the subject under study is "less" than the reference level of said autoantibody when the level of said autoantibody in the biological sample from the subject is at least 1.5 times, 5 times, 10 times, 20 times, 30 times, 40 times, 50 times, 60 times, 70 times, 80 times, 90 times, 100 times, or even more, lower than the reference level of said autoantibody.

In another particular embodiment, the level of autoantibodies to two or more proteins of the group consisting of the Pim1, SRC, MAPKAPK3, FGFR4, STK4 and ACVR2B proteins wherein one of the proteins is the Pim1 protein, in the sample from the subject to be analyzed is quantified and the levels obtained are compared with the reference levels of the autoantibodies to the corresponding proteins. By way of illustration, the autoantibodies to 2, 3, 4, 5 and 6 selected proteins of the group consisting of the Pim1, SRC, MAPKAPK3, FGFR4, STK4 and ACVR2B proteins, as is mentioned in the aforementioned List of Combinations of proteins, can be quantified.

Thus, in a particular embodiment, the level of autoantibodies to the Pim1 protein, and the level of autoantibodies to the MAPKAPK3 protein or the level of autoantibodies to the ACVR2B protein, preferably, the level of autoantibodies to the MAPKAPK3 protein or the level of autoantibodies to the ACVR2B protein is quantified and compared with their reference levels.

In another particular embodiment, the level of autoantibodies to the Pim1 protein, the level of autoantibodies to the MAPKAPK3 protein and the level of autoantibodies to the ACVR2B protein, and, optionally the level of autoantibodies to the FGFR4 protein are quantified and compared with their reference level.

The method of prognosis 1 of the invention allows evaluating the prognosis of a patient suffering CRC and/or tracking the progress of said patient suffering CRC, i.e., if he has a good prognosis and will progress favorably or if he has a poor prognosis and will progress unfavorably. Said method can be used in any stage of CRC. In a particular embodiment, the subject is a patient suffering CRC in initial stages, such as stages 0, I and II.

In another aspect, the invention relates to a method for evaluating the prognosis or tracking of the progress of a patient suffering colorectal cancer (CRC), hereinafter "method of prognosis 2 of the invention", which comprises comparing the level of expression of an expression product of the Pim1 gene and the level of expression of at least one expression product of a gene, wherein said gene is selected from the group consisting of the *SRC, MAPKAPK3, FGFR4, STK4* and *ACVR2B* genes, in a sample from said patient suffering CRC, with the reference level for said expression product of said gene, wherein if the level of said expression product of the *Pim1* gene is greater than the corresponding reference level for said expression product of said gene and the level of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, said patient suffers a CRC with a poor prognosis or presents a CRC with an unfavorable progress.

The method of prognosis 2 of the invention comprises previously determining the level of expression of an expression product of the Pim1 gene and the level of expression of at least one expression product (e.g., RNA or protein) of a gene selected from the group consisting of the *SRC, MAPKAPK3, FGFR4, STK4* and *ACVR2B* genes, in a biological sample from the patient suffering CRC in question. In a particular embodiment, said biological sample is a colon or rectal tissue sample adjacent to the tumor, tumor tissue, blood, plasma or serum of said patient suffering CRC. The level of said expression product can be determined, depending on its nature (RNA or protein), as has been previously indicated in relation to the method of obtaining data 2 of the invention.

Once the level of expression of the expression product of the Pim1 gene and the level of expression of one or more of the expression products of the *SRC, MAPKAPK3, FGFR4, STK4* and *ACVR2B* genes is determined in said biological sample, the method of diagnosis 2 of the invention comprises comparing the level of said expression product (or expression products) in the sample from the patient suffering CRC with the reference level for said expression product (or with the reference levels for the expression products in question), wherein if the level of said expression product of the *Pim1* gene is greater than the corresponding reference level for said expression product of said gene and the level of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, then said patient suffers a CRC with a poor prognosis or presents a CRC with an unfavorable progress.

In a particular embodiment of the invention, said reference level is the level or amount of expression product of said gene (*Pim1, SRC, MAPKAPK3, FGFR4, STK4,* and *ACVR2B*) in a biological sample, preferably of the colon, of a population of control subjects (i.e., who do not suffer CRC). In another particular embodiment of the invention, said reference level is the level or amount of expression product of said gene (*Pim1, SRC, MAPKAPK3, FGFR4, STK4,* and *ACVR2B*) in a biological sample, preferably of tumor tissue or of colon or rectal tissue obtained, for example, from a non-cancerous area continuous or adjacent to the tumor from the same patient suffering CRC previously obtained, for example, before the administration of a treatment for CRC, for the purpose of being able to evaluate the effectiveness of said treatment.

It will generally be considered that the level of an expression product of a gene (e.g., *Pim1, SRC, MAPKAPK3, FGFR4* or *STK4*) in the sample from the subject under study is "greater" than the reference level of said expression product when the relationship between the level of the expression product of the gene in question determined in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, the reference level of said expression product of said gene. Similarly, it will generally be considered that the level of an expression product of a gene (e.g., *ACVR2B*) in the biological sample from the subject under study is "less" than the reference level of said expression product of said gene when the level of said autoantibody in the biological sample from the subject is at least 1.5 times, 5 times, 10 times, 20 times, 30 times, 40 times, 50 times, 60 times, 70 times, 80 times, 90 times, 100 times, or even more, lower than the reference level of said expression product of said gene.

In another particular embodiment, the levels of expression of expression products of two or more genes of the group consisting of the *Pim1, SRC, MAPKAPK3, FGFR4, STK4* or *ACVR2B* genes wherein one of the genes is the Pim1 gene, in the sample from the subject to be analyzed are quantified and the levels obtained are compared with the reference levels corresponding of said expression products of the genes in question. By way of illustration, expression products of 2, 3, 4, 5 and 6 genes selected from the group consisting of the *Pim1, SRC, MAPKAPK3, FGFR4, STK4* and *ACVR2B* genes, such as, for example, expression products of the combinations of genes mentioned in the aforementioned List of Combinations of genes, can be quantified.

In a particular embodiment, the level of an expression product of the *Pim1* gene, and the level of an expression product of the *MAPKAPK3* gene or the level of an expression product of the *ACVR2B* gene, preferably, the level of an expression product of the *MAPKAPK3* gene or the level of an expression product of the *ACVR2B* gene is quantified and compared with their reference levels.

In another particular embodiment, the level of an expression product of the *Pim1* gene, the level of an expression product of the *MAPKAPK3* gene and the level of an expression product of the *ACVR29* gene, and, optionally the level of an expression product of the *FGFR4* gene are quantified and compared with their reference level.

The method of prognosis 2 of the invention allows evaluating the prognosis of a patient suffering CRC and/or tracking the progress of said patient suffering CRC, i.e., if he has a good prognosis and will progress favorably or if he has a poor prognosis and will progress unfavorably. Said method can be used in any stage of the CRC. In a particular embodiment, the subject is a patient suffering CRC in initial stages, such as stages 0, I and II.

### Methods of Diagnosis of Metastasis

The teachings of the present invention are also useful for analyzing the possibility that a patient suffering CRC will develop a lung or liver metastasis.

Therefore, the description describes to a "method for diagnosing lung metastasis in a patient suffering colorectal cancer (CRC)" which comprises comparing the level of at least one autoantibody to a protein in a sample from said patient, wherein said protein is a protein selected from the group of proteins mentioned in Table 2, with the reference level for said autoantibody, wherein if the level of autoantibody to said protein in said sample is greater than the reference level for said autoantibody, the CRC patient presents lung metastasis.

**Table 2**

| Proteins related to lung metastasis | |
|---|---|
| **PROTEIN** | **ACCESSION NUMBER** |
| PAK1 | Q13153 |
| HOMER2 | Q9NSB8 |
| IRAK4 | Q9NWZ3 |
| PRKCD2 | A0JLT6 |
| AK075484 | Q8N2G5 |
| C2ORF13 | Q8IW19 |
| PSCD3 | Q75ML1 |
| SH3BGRL2 | Q9UJC5 |
| CDK2 | P24941 |
| DAPK2 | Q1RMF4 |
| TRPT1 | Q86TN4 |
| PDGFRB | P09619, B5A957, Q5UBV6 |
| NEK1 | Q96PY6 |
| SOCS3 | 014543 |
| EPHA4 | Q53TA0, C9JEM6, C9JIX8, Q584H6, C9JFX5 |

### Databases: UniProtKB/TrEMBL - UniProtKB/Swiss-Prot

The method of diagnosis of lung metastasis in a patient suffering CRC herein described comprises previously determining the level of at least one autoantibody to a protein selected from among the proteins mentioned in Table 2, in a biological sample from the patient suffering CRC in question. In a particular embodiment, said biological sample is a blood, plasma or serum sample from said patient suffering CRC. The level of said autoantibodies can be determined as it has been previously indicated in relation to the method of detection of autoantibodies of the invention or to the method of obtaining data 1 of the invention but applied on the proteins of Table 2 instead of on the proteins therein mentioned (Pim1, SRC, MAPKAPK3, FGFR4, STK4 and ACVR2B).

Once the level of one or more of the autoantibodies to said proteins is determined in said biological sample, the method of diagnosis of lung metastasis in a patient suffering CRC herein described comprises comparing the level of said autoantibody (or autoantibodies) with the reference level for said autoantibody (or with the reference levels for the autoantibodies in question), wherein if the level of autoantibody/autoantibodies to said protein/proteins in said sample is greater than the reference level for said autoantibody/autoantibodies, the CRC patient presents lung metastasis.

In a particular embodiment, said reference level is the level or amount of autoantibodies to said proteins mentioned in Table 2 in a sample, preferably of serum, from subjects who do not present CRC. In another particular embodiment, said sample is from patients suffering CRC but who do not have metastasis. It will generally be considered that the level of an autoantibody to a protein (e.g., the proteins mentioned in Table 2) in the sample from the patient suffering CRC to be analyzed is "greater" than the reference level of said autoantibody when the level of said autoantibody in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, the reference level of said autoantibody.

In a particular embodiment, only the level of autoantibodies to a single protein of the mentioned in Table 2 in the sample from the patient suffering CRC to be analyzed is quantified and is compared with the reference level of autoantibodies to said protein.

In another particular embodiment, the level of autoantibodies to two or more proteins of the group consisting of the proteins mentioned in Table 2 in the sample from the patient suffering CRC to be analyzed is quantified and the levels obtained are compared with the reference levels of the autoantibodies to the corresponding proteins. By way of illustration, the autoantibodies to 2, 3, 4, 5, 6, 7, 8, 9, 10, 11, 12, 13, 14 and 15 selected proteins of the group consisting of the proteins shown in Table 2 can be quantified.

The method of diagnosis of lung metastasis in a patient suffering CRC herein described allows evaluating if a CRC patient presents lung metastasis.

The description also describes a "method for diagnosing liver metastasis in a patient suffering colorectal cancer (CRC)" which comprises comparing the level of at least one autoantibody to a protein in a sample from said patient, wherein said protein is a protein selected from the group of proteins mentioned in Table 3, with the reference level for said autoantibody, wherein if the level of autoantibody to said protein in said sample is greater than the reference level for said autoantibody, the CRC patient presents liver metastasis.

**Table 3**

| Proteins related to the liver metastasis | |
|---|---|
| **PROTEIN** | **ACCESSION NUMBER** |
| PHLDB1 | Q96D60, Q96C94, Q86UU1 |
| AKT3 | Q56A86 |
| PRKCH | P24723 |
| MAPKAPK3 | Q16644 |
| C9ORF43 | Q8TAL5 |
| EGFR | Q504U8, P00533, A2VCQ7, Q147T7 |
| CAMKV | Q8NCB2 C9JSB2 C9J9E2 C9JNE8 |
| THAP3 | Q8WTV1 |
| C15ORF38 | Q7Z6K5 |
| EPB41L5 | Q4ZG32 Q9HCM4 Q53RT1 Q53T34 |
| PGAM1 | P18669 |
| PADI4 | Q9UM07 Q6EVJ4 Q6EVJ1 Q6EVJ5 Q6EVJ7 Q6EVJ2 Q6EVJ6 |
| UBE2T | Q9NPD8 |
| C9ORF78 | Q9NZ63 Q6GVN4 |
| WDR61 | Q9GZS3 |
| PRKCB1 | P05771 D3DWF5 |
| PRKCD | C9J9P1 C9JZU8 |
| ZAP70 | P43403 |
| ABL2 | P42684 B5MEB6 D1MPS6 |
| WEE1 | P30291 |
| DCAMKL2 | Q8N568 |
| TRIM21 | P19474 Q5XPV5 |

The method of diagnosis of liver metastasis in a patient suffering CRC herein described comprises previously determining the level of at least one autoantibody to a protein selected from among the proteins mentioned in Table 3, in a biological sample from the patient suffering CRC in question. In a particular embodiment, said biological sample is a blood, plasma or serum sample from said patient suffering CRC. The level of said autoantibodies can be determined as has been previously indicated in relation to the method of detection of autoantibodies of the invention or to the method of obtaining data 1 of the invention but applied on the proteins of Table 3 instead of on the proteins therein mentioned (Pim1, SRC, MAPKAPK3, FGFR4, STK4 and ACVR2B).

Once the level of one or more of the autoantibodies to said proteins is determined in said biological sample, the method of diagnosis of liver metastasis in a patient suffering CRC herein described comprises comparing the level of said autoantibody (or autoantibodies) with the reference level for said autoantibody (or with the reference levels for the autoantibodies in question), wherein if the level of autoantibody/autoantibodies to said protein/proteins in said sample is greater than the reference level for said autoantibody/autoantibodies, the CRC patient presents liver metastasis.

In a particular embodiment of the invention, said reference level is the level or amount of autoantibodies to said proteins mentioned in Table 3 in a sample, preferably of serum, from subjects who do not present CRC. In another particular embodiment, said sample is from patients suffering CRC but who do not have metastasis. It will generally be considered that the level of an autoantibody to a protein (e.g., the proteins mentioned in Table 3) in the sample from the patient suffering CRC to be analyzed is "greater" than the reference level of said autoantibody when the level of said autoantibody in the biological sample from the subject is at least 1.5 times, 2 times, 3 times, 4 times, 5 times, 10 times, 20 times, 30 times, 40 times, 50 times, 60 times, 70 times, 80 times, 90 times, 100 times or even more, the reference level of said autoantibody. In a particular embodiment, only the level of autoantibodies to a single protein of the mentioned in Table 3 in the sample from the patient suffering CRC to be analyzed is quantified and is compared with the reference level of autoantibodies to said protein.

In another particular embodiment, the level of autoantibodies to two or more proteins of the group consisting of the proteins mentioned in Table 3 in the sample from the patient suffering CRC to be analyzed is quantified and the levels obtained are compared with the reference levels of the autoantibodies to the corresponding proteins. By way of illustration, the autoantibodies to 2, 3, 4, 5, 6, 7, 8, 9, 10, 11, 12, 13, 14, 15, 16, 17, 18, 19, 20, 21 and 22 selected proteins of the group consisting of the proteins shown in Table 3 can be quantified.

The method of diagnosis of liver metastasis in a patient suffering CRC herein described allows evaluating if a CRC patient presents liver metastasis.

### Kits and Applications

In another aspect, the invention relates to a kit, hereinafter "kit of the invention", which comprises
- the elements necessary for detecting at least one autoantibody to the Pim1 protein and an autoantibody selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, or alternatively
- the elements necessary for detecting an expression product of the Pim1 gene and at least one expression product of a gene selected from the group consisting of the *SRC*, *MAPKAPK3, FGFR4, STK4* and ACVR2B genes.

In a particular embodiment, the kit of the invention furthermore comprises the elements necessary for comparing the amount of autoantibodies to at least one of the Pim1, SRC, MAPKAPK3, FGFR4, STK4 or ACVR2B proteins with a reference amount.

In another particular embodiment, the kit of the invention furthermore comprises the elements necessary for detecting and/or comparing the amount of autoantibodies to a protein mentioned in Table 2 with a reference amount.

In another particular embodiment, the kit of the invention furthermore comprises the elements necessary for detecting and/or comparing the amount of autoantibodies to a protein mentioned in Table 3 with a reference amount.

In another particular embodiment, the kit of the invention comprises the elements necessary for detecting the amount of the expression product of the Pim1 gene and for detecting the amount of the expression product of at least one of the genes selected from the list which comprises *SRC, MAPKAPK3, FGFR4, STK4* or *ACVR2B* in a biological sample isolated from a subject. In a preferred embodiment of this aspect of the invention, the kit of the invention furthermore comprises the elements necessary for comparing the detected amount of the expression product of the *Pim1* gene and for comparing the detected amount of the expression product of the *SRC, MAPKAPK3, FGFR4, STK4* or *ACVR2B* genes with a reference amount.

The kit of the invention can furthermore contain all those reagents necessary for detecting the amount of autoantibodies to the Pim1 protein and for detecting the amount of autoantibodies to the SRC, MAPKAPK3, FGFR4, STK4 or ACVR2B proteins, or to the proteins mentioned in Tables 2 or 3, or of the expression product of the *Pim1* gene and of the *SRC, MAPKAPK3, FGFR4, STK4* or *ACVR2B* genes, by means of any of the methods previously described herein such as, for example, but not limited to
a) the Pim1 protein and the SRC, MAPKAPK3, FGFR4, STK4 or ACVR2B proteins, and/or their functionally equivalent fragments or variants;
b) the antibodies capable of specifically recognizing the proteins mentioned in paragraph a);
c) primers;
d) polymerases;
e) probes; or
f) positive and/or negative controls.

The kit of the invention can furthermore include without any type of limitation, buffers, agents for preventing the contamination, protein degradation inhibitors, etc. In addition, the kit of the invention can include all the supports and containers necessary for starting and optimizing it. Preferably, the kit furthermore comprises the instructions for carrying out the method of the invention.

In another aspect, the invention relates to the use of the kit of the invention for:
- diagnosing if a subject suffers colorectal cancer (CRC); or for
- evaluating the prognosis or tracking of the progress of a patient suffering CRC.

### EXAMPLES

The following specific examples provided in this patent serve to illustrate the nature of the present invention. These examples are included only for illustrative purposes and must not be interpreted as limitations to the invention that is herein claimed. Therefore, the examples described below illustrate the invention without limiting the field of application thereof.

### EXAMPLE 1

### Identification of autoantibodies specific to colorectal cancer (CRC)

Twelve sera from patients with CRC in advanced stages and who developed different types of liver metastasis (7 patients), liver and lung metastasis (4 patients) and liver and bone metastasis (1 patient) and 8 sera from healthy individuals [i.e., from individuals without CRC] (control sera) were tested using high-density protein microarrays for the purpose of identifying autoantibodies specific to CRC and their respective reactive antigens (Table 4). The control sera were selected to have exactly the same proportion of women and men and the same mean age of the patients with CRC (64.5 years). The healthy controls and the patients with CRC showed a different immunoreactivity pattern.

**Table 4**

| Clinical information of the patients with CRC tested in the protein microarrays | | | | | |
|---|---|---|---|---|---|
| **Serum** | **Age¹** | **Sex²** | **Progression³** | **Time of survival in months⁴** | **Metastasis** |
| VH1 | 84 | F | Alive | - | Liver |
| MH1 | 60 | F | Dead | 15 | Liver |
| MHP1 | 65 | M | Dead | 64 | Liver-lung |
| MHP2 | 41 | M | Dead | 62 | Liver-lung |
| MH2 | 55 | M | Dead | 14 | Liver |
| MHP3 | 62 | M | Dead | 51 | Liver-lung |
| VP1 | 71 | F | Alive | - | Liver-bones |
| VH2 | 75 | M | Alive | - | Liver |
| MH3 | 76 | M | Dead | 31 | Liver |
| MH4 | 64 | M | Dead | 28 | Liver |
| VHP1 | 51 | M | Alive | - | Liver-lung |
| VH3 | 74 | M | Alive | - | Liver |

| | | | | | |
|---|---|---|---|---|---|
| ¹Age in years. ²M, male; F, female. ³Progression of patients with CRC after obtaining serum. ⁴Time of survival in months after obtaining serum. ⁵Metastasis associated with the patient. | | | | | |

After quantifying the intensity of the different points (proteins) with the GenePix program, the data were normalized using the quantile method and were processed using the ProtoArray Prospector Analyser. The arrays used as control showed an excellent behavior, with a low level of background noise and specific reactivity.

For the purpose of studying the capacity of the antibody signature to discriminate between the different types of metastasis, an unsupervised cluster was formed with the processed data using the MeV program (Dana-Farber Cancer Institute, Boston, MA, USA). The metastatic samples were separated in two main branches which corresponded to the patients with metastasis in the liver and in the liver and lung and only two samples were not correctly classified. In addition, the supervised analysis with the processed data of the patients with CRC showed that the two types of patients could be satisfactorily separated.

Thus, a sample of TAAs with a prevalence greater than 60% was associated with the patients with CRC with lung metastasis (15 proteins) (Table 5) or with liver metastasis (22 proteins) (Table 6).

**Table 5**

| Proteins reactive to metastasis associated autoantibodies Increased reactivity in lung metastasis | | | | |
|---|---|---|---|---|
| Name | Lung prevalence | Liver prevalence | *p*-value | Function |
| PAK1 | 86% | 13% | 0.00216 | Cell motility and morphology |
| HOMER2 | 86% | 25% | 0.01299 | Cell growth |
| IRAK4 | 86% | 25% | 0.01299 | Signal transduction |
| PRKD2 | 86% | 25% | 0.01299 | Signal transduction |
| AK075484 | 86% | 13% | 0.01299 | Hypothetical protein |
| C2orf13 | 71% | 13% | 0.01515 | Hypothetical protein |
| PSCD3 | 71% | 13% | 0.01515 | Signal transduction |
| SH3BGRL2 | 71% | 13% | 0.01515 | Unknown |
| CDK2 | 71% | 13% | 0.01515 | Cell cycle |
| DAPK2 | 71% | 13% | 0.01515 | Apoptosis |
| TRPT1 | 86% | 38% | 0.04545 | RNA-binding protein |
| PDGFRB | 86% | 38% | 0.04545 | Signal transduction |
| NEK1 | 86% | 38% | 0.04545 | DNA repair |
| SOCS3 | 86% | 25% | 0.04545 | Cytokine signaling |
| EPHA4 | 86% | 25% | 0.04545 | Signal transduction, angiogenesis |

**Table 6**

| Proteins reactive to metastasis associated autoantibodies Increased reactivity in liver metastasis | | | | |
|---|---|---|---|---|
| Name | Liver prevalence | Lung prevalence | *p*-value | Function |
| PHLDB1 | 88% | 14% | 0.0022 | Unknown |
| AKT3 | 75% | 14% | 0.0130 | Signal transduction |
| PRKCH | 75% | 14% | 0.0130 | Signal transduction |
| MAPKAPK3 | 88% | 29% | 0.0152 | Ras protein signal transduction |
| C9orf43 | 88% | 29% | 0.0152 | Hypothetical protein |
| EGFR | 88% | 29% | 0.0152 | Signal transduction |
| CAMKV | 63% | 14% | 0.0455 | Kinase, cell signaling |
| THAP3 | 63% | 14% | 0.0455 | Apoptosis |
| C15orf38 | 63% | 14% | 0.0455 | Hypothetical protein |
| EPB41L5 | 63% | 14% | 0.0455 | Cell adhesion |
| PGAM1 | 63% | 14% | 0.0455 | Metabolism, energy pathway |
| PADI4 | 63% | 14% | 0.0455 | Metabolism, energy pathway |
| UBE2T | 63% | 14% | 0.0455 | Protein metabolism |
| C9orf78 | 63% | 14% | 0.0455 | Hypothetical protein |
| WDR61 | 63% | 14% | 0.0455 | Transcriptional regulation |
| PRKCB1 | 63% | 14% | 0.0455 | Signal transduction |
| PRKCD | 63% | 14% | 0.0455 | Signal transduction |
| ZAP70 | 63% | 14% | 0.0455 | T cell development activation |
| ABL2 | 63% | 14% | 0.0455 | Signal transduction |
| WEE1 | 63% | 14% | 0.0455 | Cell cycle |
| DCAMKL2 | 63% | 14% | 0.0455 | Unknown |
| TRIM21 | 63% | 14% | 0.0455 | Transcriptional regulation |

### EXAMPLE 2

### Characterization of the more prevalent TAAs in colorectal cancer

The proteins which showed discriminatory capacity between normal and tumor patients are shown in Table 7. The analysis was performed using the ProtoArray Prospector Analyzer program, classifying the data according to the p-value calculated for each protein and the prevalence of the autoantibodies in both groups. The p-value was established so that it was 0.04 at most and the prevalence greater than 50% in the population of patients with CRC. In total, 432 proteins showed immunoreactivity to the autoantibodies present in the serum. Among such proteins, 43 had a significant p-value less than 0.04. In terms of their classification, 25 of them had a greater prevalence in the serum from patients with CRC and 18 a lower prevalence in patients with CRC respect to control individuals. Six proteins -MAPKAPK3, Pim1, SRC, STK4, FGFR4 and ACVR2B- were selected according to the data of signal intensity of the microarrays.

Said proteins were among the most prevalent in the serum from patients with CRC according to the analysis using the Prospector Analyzer and showed prevalence in CRC between 50-70% and less than 20% of prevalence in the control subjects.

Although there were significant variations in terms of the individual response, MAPKAPK3, Pim1, SRC, STK4 and FGFR4 were significantly recognized by patients with CRC. In addition, ACVR2B showed a different recognition given that mainly the control subjects recognized it.

**Table 7**

| Proteins reactive to CRC associated autoantibodies*. Reduced reactivity in colorectal cancer | | | | |
|---|---|---|---|---|
| Increased reactivity in colorectal cancer | | | | |
| Name | Cancer prevalence | Control prevalence | *p*-value | Function |
| MAPKAP3 | 71.4% | 10% | 0.0099 | Ras protein signal transduction |
| PIM-1 | 71.4% | 20% | 0.0099 | Cell proliferation |
| STK4 | 71.4% | 20% | 0.0099 | Cell morphogenesis |
| FGFR4 | 71.4% | 20% | 0.0099 | Fibroblast growth factor receptor signaling pathway |
| TRIM21 | 71.4% | 20% | 0.0099 | Transcriptional regulation |
| SRC | 57.1% | 10% | 0.0102 | Ras protein signal transduction |
| AKT1 | 57.1% | 10% | 0.0102 | G protein-coupled receptor signaling pathway |
| KDR | 57.1% | 10% | 0.0102 | Angiogenesis |
| PKN1 | 57.1% | 10% | 0.0102 | JNK activity activation |
| CSNK1G2 | 92.9% | 50% | 0.0144 | Wnt receptor signaling pathway |
| DAPK1 | 92.9% | 50% | 0.0144 | Anti-apoptosis |
| PBK | 78.6% | 30% | 0.0154 | Mitosis |
| NEK3 | 85.7% | 30% | 0.0181 | Cell cycle |
| PRKCD | 85.7% | 40% | 0.0181 | Intracellular signaling cascade |
| SALL2 | 50.0% | 10% | 0.0238 | Transcriptional regulation, DNA-dependent |
| GRK7 | 50.0% | 10% | 0.0238 | G protein-coupled receptor kinase activity |
| IRAK4 | 50.0% | 10% | 0.0238 | I-kappaB Kinase/NF-kappaB cascade |
| MAPKAPK5 | 50.0% | 10% | 0.0238 | Ras protein signal transduction |
| PKN2 | 50.0% | 10% | 0.0238 | Signal transduction |
| ABL2 | 50.0% | 10% | 0.0238 | Cell adhesion |
| RPS6KA1 | 64.3% | 10% | 0.0249 | Signal transduction |
| BMX | 64.3% | 20% | 0.0249 | Intracellular signaling cascade |
| PDGFRB | 64.3% | 20% | 0.0249 | Platelet growth factor receptor signaling pathway |
| CDK5/p35 | 64.3% | 20% | 0.0249 | Muscarinic acetylcholine receptor pathway |
| RPS6KA2 | 71.4% | 30% | 0.0399 | Signal transduction |

| | | | | |
|---|---|---|---|---|
| * The proteins were classified according to the calculated p-value and the prevalence of the protein in the colorectal cancer group or in the control group. | | | | |

**Table 7 (cont.). Proteins reactive to CRC associated autoantibodies***

| Reduced reactivity in colorectal cancer | | | | |
|---|---|---|---|---|
| RBPJ | 60% | 7.1% | 0.0036 | DNA recombination |
| ITGA6 | 80% | 28.6% | 0.0099 | Cell adhesion |
| ACVR2B* | 70% | 21.4% | 0.0144 | BMP signaling pathway |
| NFYA | 50% | 7.1% | 0.0144 | Transcriptional regulation |
| TTLL7 | 50% | 7.1% | 0.0144 | Cell differentiation |
| C9orf43 | 50% | 7.1% | 0.0144 | Unknown |
| ZNF706 | 50% | 7.1% | 0.0144 | Unknown |
| HDAC1 | 50% | 7.1% | 0.0144 | Anti-apoptosis |
| TPM4 | 50% | 7.1% | 0.0144 | Cell motility |
| TSLP | 70% | 21.4% | 0.0154 | Cytokine, cell signaling |
| WBP2 | 70% | 21.4% | 0.0154 | Unknown |
| STAU1 | 60% | 14.3% | 0.0181 | RNA-binding protein |
| PFDN5 | 60% | 14.3% | 0.0181 | Protein folding |
| COASY | 60% | 14.3% | 0.0181 | Coenzyme A biosynthesis |
| IGLC1 | 80% | 35.7% | 0.0249 | tRNA aminoacylation for protein translation |
| MFAP2 | 70% | 21.4% | 0.0399 | Cytoskeleton |
| BHMT2 | 70% | 21.4% | 0.0399 | Methyltransferase |
| EFNA3 | 70% | 28.6% | 0.0399 | Cell signaling |

| | | | | |
|---|---|---|---|---|
| * The proteins were classified according to the calculated p-value and the prevalence of the protein in the colorectal cancer group or in the control group. | | | | |

### EXAMPLE 3

### Analysis of the differential expression of the selected TAA proteins in CRC cell lines and tumor tissue

A higher recognition by the autoantibodies present in the serum from patients with CRC would indicate overexpression of those proteins in CRC tumor tissue, whereas a weaker recognition in the serum from patients with CRC than in the normal individuals (subjects) would indicate a reduction of the expression or other modification of said proteins in the tumor. With this starting hypothesis, 3 autoantigens: Pim1, MAPKAPK3 and ACVR2B were selected for the initial validation.

Firstly, the levels of expression of the proteins in paired normal/tumor extracts of tissue from the same patient with CRC were analyzed by means of membrane immunodetection (Figure 1A). Pim1 and MAPKAPK3 showed a greater expression in tumor tissue, their expression being more abundant in advanced stages of the disease. ACVR2B exhibited a weak expression in tumor tissue and, generally, more expression in early stages of the disease. Subsequently, the levels of expression of the autoantigens in 6 CRC cell lines were analyzed in comparison with 5 cell lines used as reference (Figure 1B). The expression of Pim1 and MAPKAPK3 was detected in virtually all the CRC cell lines, except MAPKAPK3 in the cell line SW480. In terms of ACVR2B, its expression was observed in the reference cell lines including neutrophils and lymphocytes, but not in the colon cancer cell lines.

For the purpose of studying the correlation between the humoral response and the abundance in tissue, the differential expression of the 6 proteins was verified at the level of messenger RNA (mRNA) and at the protein level. In order to determine the levels of mRNA, the Oncomine database was used (Rhodes et al. 2004. Neoplasia New York, N.Y. 6 (1), 1-6), a web page which includes a database with the gene expression data results in cancer using microarrays. The data for FGFR4, MAPKAPK3, SRC and STK4 in CRC are shown in Figure 1C. They all showed greater levels of expression in CRC. No data were found for Pim1 and ACVR2B in CRC. In order to corroborate the differential expression of the proteins, a CRC tissue microarray with antibodies specific to ACVR2B and Pim1, which were the only ones commercially available for this technique out of the 6 proteins studied (Figure 1D), were used.

Pim1 showed an increased expression in the epithelial cells surrounding the tumor tissue crypts, the staining mainly being cytoplasmic. Furthermore, both the lymphocytes and the macrophages were very significantly stained.

In terms of ACVR2B, the expression was reduced in patients with CRC, whereas in normal tissue its normal expression was observed. In this case, ACVR2B staining was mainly located in the membrane of the epithelial cells given that it acts as a membrane receptor.

Figure 1D shows the result of the immunohistochemical analysis of Pim1 and ACVR2B in CRC tissue and normal adjacent mucosa of 45 patients with CRC. As can be seen, the level of the Pim1 protein is increased in the samples of CRC whereas that of ACVR2B is decreased.

### EXAMPLE 4

### Confirmation of using Pim1, ACVR2B and MAPKAPK3 as biomarkers in CRC

The ELISA technique is widely used in clinical practice because of its simplicity and sensitivity. For the purpose of corroborating the previous results, an ELISA assay was performed with the Pim1, MAPKAPK3 and ACVR2B recombinant proteins expressed in *E*. *coli*, since this would allow easily discriminating between sera of healthy individuals and individuals with CRC. Commercial CEA and recombinant Annexin IV expressed in mammal cells were used as controls. CEA was used because it is the most used marker in the diagnosis of CRC (Duffy, M. J. 2001 Clinical chemistry 47 (4), 624-630), and Annexin IV was used because of its overexpression in CRC tissue (Alfonso et al. 2005. Proteomics 5 (10), 2602-2611). Thus, 94 samples of serum, 52 sera from patients with CRC and 42 sera from healthy individuals, were tested in this direct ELISA assay. The results of the ELISA were consistent with those obtained in the protein array and in the membrane immunodetection; the autoantibodies to Pim1, MAPKAPK3 and ACVR2B allowed discriminating between patients with CRC and control sera.

Pim1 showed a significantly greater immunoreactivity in the serum from patients with CRC (mean= 0.606, 95% CI= 0.505 a 0.708, p<0.008) than in control subjects (mean = 0.439, 95% CI= 0.369 to 0.510).

For MAPKAPK3 similar results were obtained in sera from patients with CRC (mean= 0.929, 95% CI= 0.828 to 1.030, p<0.0001) and from control subjects (mean= 0.648, 95% CI= 0.574 to 0.722) (Figure 2).

In the case of ACVR2B, the immunoreactivity was greater in the serum from control subjects (mean= 0.863, 95% CI= 0.744 to 0.981, p<0.026) than in the serum from patients with CRC (mean= 0.668, 95% CI= 0.549 to 0.790), which confirmed the previous results obtained with the protoarrays.

In terms of the immunoreactivity of CEA and Annexin IV, no significant differences between the tumor samples from patients with CRC (CEA: mean= 0.787, 95% CI= 0.674 to 0.900, p<0.1); (Annexin IV: mean= 0.421, 95% CI= 0.360 to 0.481, p<0.16) and samples from control subjects (CEA: mean= 0.665, 95% CI= 0.588 to 0.742; Annexin IV: mean= 0.366, 95% CI= 0.318 to 0.414) were observed.

The capacity of this humoral response as a predictor for detecting CRC was subsequently investigated. The ROC curves were thus obtained from the response of the autoantibodies to the Pim1, MAPKAPK3 and ACVR2B proteins (Figure 3A). The specificity and sensitivity of the assay for Pim1 were 83.3% and 48.1% (using a cut-off of 0.534), respectively, and the area under the curve (AUC) was AUC= 0.651 (95% CI= 0.546 to 0.746). In the case of MAPKAPK3, the specificity was 74% and the sensitivity 72.7% (with a cut-off of 0.762), with AUC= 0.733 (95% CI= 0.632 to 0.819). Specificity and sensitivity for ACVR2B were found to be 76.2% and 60%, respectively, (cut-off = 0.548) and AUC= 0.666 (95% CI = 0.562 to 0.760). The CEA and Annexin IV control proteins gave lower specificity and sensitivity values; specificity was 52.4% and sensitivity was 67.3% for CEA with a cut-off= 0.61 and AUC= 0.61 (95% CI= 0.513 to 0.717). Annexin IV gave an AUC value = 0.556 (95% CI= 0.450 to 0.658), indicating the absence of autoantibodies specific for this protein (Figure 3C).

It was finally tested whether different combinations of these proteins would improve their diagnostic capacity. The data were fitted to a logistic curve, the logistic regressions were calculated and different models were obtained by incorporating combinations of the proteins (Figure 3B). The initial model included 4 proteins: Pim1, MAPKAPK3, ACVR2B and CEA; however, the tests of the linear discriminant method showed that CEA and Pim-1 had no relevance in the model. This was confirmed by comparing the complete model with the 4 proteins (AUC=0.85) with a model which included only MAPKAPK3 and ACVR2B (AUC=0.86). While CEA did not improve the model, Pim1 even slightly deteriorated it.

It was thus demonstrated that a model with the combination of only the autoantibodies to MAPKAPK3 and ACVR2B was a relevant predictor of CRC with a specificity and sensitivity of 73.9% and 83.3%, respectively, and an area under the curve AUC= 0.86 (Figure 3B).

Additionally, as can be seen in Figure 5C, there is no correlation between the MAPKAPK3 and ACVR2B signal. Likewise, Figures 5A and 5B show that the higher the signal for ACVR2B, the greater the possibility of belonging to the normal group; the opposite situation is observed for MAPKAPK3.

Figure 6 shows an ELISA analysis of serum samples using an ELISA with STK4 and FGFR4 as TAAs.

Figure 7C shows how the combination of the measurement of the autoantibodies to MAPKAPK3, ACVR2B, Pim1 and FGFR4 results in an optimal predictive combination. Likewise, in the case of early stages of CRC, the combination of MAPKAPK3, ACVR2B, Pim1 and FGFR4 also results in optimal specificity and sensitivity.

Figure 8 shows how the combination of CEA with an optimal combination of autoantibodies (autoantibodies to the MAPKAPK3, ACVR2B, Pim1 and FGFR4 proteins) does not improve the prediction capacity for the diagnosis of CRC, which indicates that the combination of the markers of the invention is more suitable for the diagnosis of CRC in early stages than CEA alone.

The autoantibodies to the MAPKAPK3, ACVR2B, Pim1 and FGFR4 proteins give as a result better diagnosis of CRC in early stages (Figure 9). As can be seen in the results of Figure 10, the presence of autoantibodies in serum from patients with CRC to some of the biomarkers selected in the present invention (MAPKAPK3, Pim1, SRC, FGFR4 and STK4) was constant during all stages, whereas the concentration of CEA was greater in later stages of CRC. Therefore, the use of the autoantibodies to the aforementioned biomarkers (MAPKAPK3, Pim1, SRC, FGFR4 and STK4) allows a better diagnosis of CRC not only in later stages but also in early stages of CRC.

### MATERIALS AND METHODS

### Clinical information and obtaining the sera (Examples 1, 2 and 4)

The sera from 12 patients with CRC were collected at diagnosis (Hospital Universitario of Salamanca). These samples were selected because the patients had advanced stages of CRC, in addition to having developed liver metastasis (7 patients), liver and lung metastasis (4 patients) or liver and bone metastasis (1 patient). The mean age of these patients was of 64.5 years (between 41 and 84 years). Eight control sera were obtained from healthy donors and were selected to have exactly the same mean age as the population of patients with CRC and the same proportion of men and women. The clinical data of the patients are shown in Table 4. An independent set of 52 sera from patients with CRC and 42 normal sera were used for the validation of the results by means of ELISA.

All the sera were processed using the same protocol. The blood samples were left at room temperature for 30 minutes in order to allow the formation of the clot and, after its centrifugation at 3000 g for 10 minutes at 4°C, the sera were frozen and stored at -80°C until their use.

### Protein arrays (Examples 1 and 2)

20 sera (12 from patients with CRC and 8 from healthy individuals) (Table 4) were incubated with the ProtoArray™ Human Protein *Microarrays* v.4.0 (Invitrogen, Carlsbad, CA). This microarray contains 8,000 GST (glutathione-S-transferase)-fused human proteins expressed in *Spodoptera frugiperda* insect cells Sf9 and printed in duplicate. In summary, the arrays were balanced at 4°C for 15 minutes and blocked with the blocking buffer (1% bovine serum albumin (BSA) in 0.1% phosphate buffered saline (PBS) - polysorbate 20 (Tween® 20)) for 1 hour at 4°C with gentle stirring. A total of 150 µL of serum diluted 1:50 in blocking buffer were applied on the surface of the array. The array was sealed with a CoverGlass (Corning) and incubated for 90 minutes at 4°C. The arrays were washed with incubation buffer (1% BSA in PBS with 0.5 mM dithiothreitol (DTT), 5% glycerol and 0.05% Triton X-100) and the autoantibodies of the serum bound to the proteins of the array were detected using a secondary anti-human IgG antibody (H+L) labeled with Alexa Fluor 647 (Invitrogen) diluted 1:2.000 in incubation buffer at 4°C for 90 minutes. The arrays were washed with 0.1% PBS-Tween® 20 and dried by centrifugation at 1,000 rpm for 1 minute. Finally, the slides were scanned in a ScanArray™ 5000 (Packard BioChip Technologies) using 635 nm and 532 nm lasers. The GenePix Pro 5.1 image analysis software was used for the quantification.

As controls, the ProtoArrays v4.0 were incubated with an anti-GST antibody before incubating the array with an anti-mouse IgG antibody labeled with Alexa Fluor 555 to test the uniformity and the amount of protein printed in the array. Another array was incubated directly with the secondary anti-human IgG antibody (H+L) labeled with Alexa Fluor 647 to determine the levels of noise in the assay.

### Antibodies, proteins and cell lines (Examples 3 and 4)

The antibodies and the proteins were obtained from different sources. CEA was obtained from Calbiochem and the human serum albumin (HSA) was obtained from Sigma.

The cDNA encoding human Pim1 was introduced in the pET28a vector, which allows 6xHis-Pim1 fusion, and was expressed in *Escherichia coli* using the BL21 (DE3) strain. The cDNA of human ACVR2B was introduced in the pDEST 527 vector, which allows 6xHis-ACVR2B fusion using the Gateway system, and was expressed in bacteria, whereas the human MAPKAPK3 protein was introduced in the pDEST565 expression vector, which allows 6xHis-GST-MAPKAPK3 fusion and was expressed in the same conditions as ACVR2B and Pim1. The proteins thus expressed were purified by means of affinity chromatography using a HiTrap Chelating column (GE Healthcare) followed by an additional purification step by means of a Superdex 200 penetrability column (GE Healthcare). The cDNA encoding the human Annexin IV was cloned into the pTT3 expression vector and was expressed in HEK293-EBNA cells. The recombinant protein was expressed after transfecting the cells with lipofectamine (Sigma) and it was purified by means of a Ni-chelating resin affinity column (GE Healthcare). The antibodies to MAPKAPK3 and Pim1 used in the ELISA assays were purchased from Abnova. The antibodies to MAPKAPK3, Pim1 and ACVR2B used in membrane immunodetection and tissue array were purchased from Abcam. The CRC cell lines (Rko, Hct116, Hct15, Sw45, Sw480, Colo 205), BxPc3 pancreatic adenocarcinoma cell line and the Molt4 lymphoblastoid line were grown using protocols pre-established for said cells. The neutrophils and lymphocytes used as controls were isolated from peripheral blood of a healthy individual. The murine embryonic fibroblasts (MEF) were immortalized by infecting a primary culture with the Epstein-Barr virus and were grown using protocols pre-established for this cell line.

### ELISA (Example 4)

An ELISA assay was developed for the purpose of testing the ability of the purified autoantigens to discriminate CRC using serum from 30 patients. In summary, 0.3 µg of the purified proteins or HSA as negative control were applied in Microtiter plates (Maxisorp, Nunc) in PBS overnight. The next day, the plates were washed 3 times with PBS and blocked with 3% skim milk in PBS for 2 hours at room temperature. After an additional washing, the 94 serum samples (diluted 1:50 in 3% skim milk in PBS) were incubated for 2 hours at room temperature. After washing, an anti-human IgG antibody conjugated with peroxidase (HRP) diluted 1:3,000 (v:v) was used for detection and 3,3',5,5'-tetramethylbenzidine (TMB) was used to develop the signal. The reaction was detained with 1 M H₂S0₄ and the absorption was measured at 450 nm.

### Membrane immunodetection (Example 3)

The paired tissue protein extracts from 6 patients with CRC (12 in total) were prepared as previously described in Alfonso, P. et al. (2005) Proteomic expression analysis of colorectal cancer by two-dimensional differential gel electrophoresis. Proteomic 5, 2602-2611). In summary, the protein extracts were obtained after their lysis with 0.1% sodium dodecylsulfate (SDS), 1% Triton X-100, 1% sodium deoxycholate, 150 mM NaCl, 5 mM ethylenediaminetetraacetic acid (EDTA), 10 mM Tris-HC1 (pH 7.2) supplemented with protease inhibitors (Roche). The protein concentration was determined using the 2-D Quant kit (GE Healthcare) after clarifying it by centrifugation at 12,000 g for 15 minutes.

For membrane immunodetection, 50 µg of the protein extracts from the 6 colon cancer cell lines, the 5 reference cell lines and the paired tissues were run in parallel in 10% SDS-PAGE gel and were transferred to nitrocellulose membranes (Hybond-C extra) according to standard protocols. After blocking, the membranes were incubated with optimal mono- or polyclonal antibodies to the selected antigens: Pim1 (1:100 dilution), MAPKAPK3 (1:500 dilution) and ACVR2B (1:200 dilution). An anti-goat IgG antibody (DakoCytomation) at a 1:5,000 dilution for ACVR2B and a 1:20,000 dilution for Pim1 and MAPKAPK3, or an anti-chicken IgG antibody (Jackson ImmunoResearch Laboratory) conjugated to HRP, were used as secondary antibodies. The signal was detected by means of ECL (GE Healthcare).

### Immunohistochemistry (Example 3)

The specific CRC tissue microarrays (TMA) with 45 different tumor samples were prepared as previously described (Madoz-Gurpide et al. 2007. Mol Cell Proteomics 6 (12), 2150-2164). The sections were cut at a maximum width of 3 µm and were dried at 56°C four 16 hours before deparaffinizing in xylene and rehydrating in water after previous steps in different percentages of ethanol. The exposure and recovery of epitopes was performed in 0.01 M sodium citrate buffer heated for 2 minutes in a pressure cooker. After the heating step, the slides were washed with water for 5 minutes and again in Tris buffered saline (TBS) at pH 7.4. The TMAs were incubated with a monoclonal antibody to Pim1 (Abcam) and a polyclonal antibody to ACVR2B (Abcam). The specific binding was detected by means of anti-goat IgG antibody conjugated to biotin. The specific interactions were viewed with the Envision HRP system (DakoCytomation).

### Statistical analysis (Examples 1, 2 and 4)

The slides were analyzed with the manufacturer's software -ProtoArray Prospector Analyser 4.0 (Invitrogen)-, which uses a statistical test based on Chebyshev's inequality principle (Hudson et al. 2007. Proceedings of the National Academy of Sciences of the United States of America 104 (44), 17494-17499). After normalization by quantiles, the algorithm compares the signal of each protein to the signal of the negative controls in the array and assigns a p-value to CI for each protein. The software identifies the significant signals (those which are identified as positive on the background noise) and calculates Z values which reflect the intensity of the signal in comparison with all the proteins. Finally, the program compares the 2 groups and identifies the proteins which have an increased signal in one of the 2 groups and the p-value is calculated for each protein according to the hypothesis that there is no signal increase in one group compared with the other.

The supervised clusters were obtained using the metric distance and the Pearson's correlation for viewing the discrimination between the groups using the Multi Experiment Viewer (MeV) program (Dana-Farber Cancer Institute, Boston, MA, USA). In order to determine if the mean of the normal group and the mean of the tumor group were statistically different, a nonparametric Wilcoxon test was performed with the data obtained from the ELISA. Each marker was subsequently evaluated individually using a ROC curve calculated with the JMP program (SAS, NC, USA). Finally, a discriminating analysis was performed using linear models to determine the effect of the combination of the biomarkers (Visintin et al. 2008. Clinical Cancer Research. 14 (4), 1065-1072).

## Claims

1. A method for diagnosing if a subject suffers colorectal cancer (CRC), which comprises comparing the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from said subject, with the reference level for said autoantibodies, wherein if the level of said autoantibody to the Pim1 protein in said sample is greater than the corresponding reference level for said autoantibody, and wherein if the level of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said subject is diagnosed with CRC.

2. A method for evaluating the prognosis or tracking of the progress of a patient suffering colorectal cancer (CRC), which comprises comparing the level of an autoantibody to the Pim1 protein and the level of at least one autoantibody to a protein, wherein said autoantibody is selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, in a biological sample from said patient suffering CRC, with the reference level for said autoantibodies, wherein if the level of said autoantibody to the Pim1 protein in said sample is greater than the corresponding reference level for said autoantibody, and wherein if the level of said autoantibody to the SRC protein, or of said autoantibody to the MAPKAPK3 protein, or of said autoantibody to the FGFR4 protein, or of said autoantibody to the STK4 protein, in said sample is greater than the corresponding reference level for said autoantibodies, and/or if the level of the autoantibody to ACVR2B in said sample is less than the reference level for said autoantibody, then said patient suffers a CRC with a poor prognosis or presents a CRC with an unfavorable progress.

3. The method according to any of claims 1 or 2, which comprises determining the level of an autoantibody to the Pim1 protein, and the level of an autoantibody to the MAPKAPK3 protein or the level of an autoantibody to the ACVR2B protein.

4. The method according to any of claims 1 or 2, which comprises determining the level of an autoantibody to the Pim1 protein, the level of an autoantibody to the MAPKAPK3 protein and the level of an autoantibody to the ACVR2B protein.

5. The method according to claim 4, which furthermore comprises determining the level of an autoantibody to the FGFR4 protein.

6. A method of obtaining data in a sample from a subject which comprises detecting the expression product of the *Pim1* gene and the expression product of at least one gene selected from the group consisting of the *SRC, MAPKAPK3, FGFR4, STK4* and *ACVR2B* genes, and, if desired, quantifying the level of expression of said expression product of said genes in said sample, wherein said sample comprises colorectal cancer (CRC) tumor cells.

7. A method for diagnosing if a subject suffers colorectal cancer (CRC), which comprises comparing the level of expression of an expression product of the *Pim1* gene and the level of expression of at least one expression product of a gene, wherein said gene is selected from the group consisting of the *SRC, MAPKAPK3, FGFR4, STK4* and *ACVR2B* genes, in a sample from said subject, with the reference level for said expression product of said genes, wherein if the level of said expression product of the *Pim1* gene is greater than the corresponding reference level for said expression product of said gene and the level of said expression product of the SRC gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, said subject is diagnosed with CRC.

8. A method for evaluating the prognosis or tracking of the progress of a patient suffering colorectal cancer (CRC), which comprises comparing the level of expression of an expression product of the *Pim1* gene and the level of expression of at least one product of expression of a gene, wherein said gene is selected from the group consisting of the *SRC, MAPKAPK3, FGFR4, STK4* and *ACVR2B* genes, in a sample from said patient suffering CRC, with the reference level for said expression product of said genes, wherein if the level of said expression product of the *Pim1* gene is greater than the corresponding reference level for said expression product of said gene and the level of said expression product of the *SRC* gene, or of said expression product of the *MAPKAPK3* gene, or of said expression product of the *FGFR4* gene, or of said expression product of the *STK4* gene, is greater than the corresponding reference level for said expression products of said genes and/or if the level of the expression product of the *ACVR2B* gene is less than the reference level for said expression product of said gene, said patient suffers a CRC with a poor prognosis or presents a CRC with an unfavorable progress.

9. The method according to any of claims 6 to 8, which comprises determining the level of expression of an expression product of the *Pim1* gene, and the level of expression of an expression product of the *MAPKAPK3* gene or the level of expression of a expression product of the *ACVR2B* gene.

10. The method according to any of claims 6 to 8, which comprises determining the level of expression of an expression product of the *Pim1* gene, the level of expression of an expression product of the *MAPKAPK3* gene and the level of expression of an expression product of the *ACVR2B* gene.

11. The method according to claim 10, which furthermore comprises determining the level of expression of an expression product of the *FGFR4* gene.

12. The method according to any of claims 6 to 8, wherein said expression product of the *Pim1, SRC, MAPKAPK3, FGFR4, STK4* or *ACVR2B* gene is the Pim1, SRC, MAPKAPK3, FGFR4, STK4 or ACVR2B protein, respectively, or a fragment thereof.

13. The method according to claim 12, wherein the level of said protein is determined by means of an immunoassay.

14. A kit comprising:
- the elements necessary for detecting at least one autoantibody to the Pim1 protein and an autoantibody selected from the group consisting of an autoantibody to the SRC protein, an autoantibody to the MAPKAPK3 protein, an autoantibody to the FGFR4 protein, an autoantibody to the STK4 protein, and an autoantibody to the ACVR2B protein, or alternatively
- the elements necessary for detecting an expression product of the *Pim1* gene and at least one expression product of a gene selected from the group consisting of the *SRC, MAPKAPK3, FGFR4, STK4* and *ACVR2B* genes.

15. Use of a kit according to claim 14 for
- diagnosing if a subject suffers colorectal cancer (CRC); or for
- evaluating the prognosis or tracking of the progress of a patient suffering CRC.

## Patentansprüche

1. Ein Verfahren zur Diagnose, ob ein Subjekt unter Kolorektalkarzinom (CRC) leidet, das das Vergleichen des Spiegels eines Autoantikörpers gegen das Pim1 Protein und den Spiegel von mindestens einem Autoantikörper gegen ein Protein, wobei der Autoantikörper ausgewählt ist aus der Gruppe bestehend aus einem Autoantikörper gegen das SRC Protein, einem Autoantikörper gegen das MAPKAPK3 Protein, einem Autoantikörper gegen das FGFR4 Protein, einem Autoantikörper gegen das STK4 Protein und einem Autoantikörper gegen das ACVR2B Protein, mit dem Referenzspiegel für die besagten Autoantikörper in einer biologischen Probe von dem Subjekt umfasst, wobei, wenn der Spiegel des besagten Autoantikörpers gegen das Pim1 Protein in der Probe größer als der entsprechende Referenzspiegel des besagten Autoantikörpers ist, und wobei, wenn der Spiegel des Autoantikörpers gegen das SRC Protein, oder des Autoantikörpers gegen das MAPKAPK3 Protein, oder des Autoantikörpers gegen das FGFR4 Protein, oder des Autoantikörpers gegen das STK4 Protein, in einer Probe größer ist als der entsprechende Referenzspiegel des besagten Autoantikörpers und/oder wenn der Spiegel des Autoantikörpers gegen ACVR2B in der Probe geringer ist als der Referenzspiegel des besagten Autoantikörpers, dann das Subjekt mit CRC diagnostiziert ist.

2. Ein Verfahren zur Bewertung der Prognose oder Verfolgung des Fortschritts eines Patienten, der unter Kolorektalkarzinom (CRC) leidet, das das Vergleichen des Spiegels eines Autoantikörpers gegen das Pim1 Protein und des Spiegels von mindestens einem Autoantikörper gegen ein Protein, wobei der besagte Autoantikörper ausgewählt ist aus der Gruppe bestehend aus einem Autoantikörper gegen das SRC Protein, einem Autoantikörper gegen das MAPKAPK3 Protein, einem Autoantikörper gegen das FGFR4 Protein, einem Autoantikörper gegen das STK4 Protein und einem Autoantikörper gegen das ACVR2B Protein, mit dem Referenzspiegel der besagten Autoantikörper in einer biologischen Probe von dem Patienten umfasst, der unter CRC leidet, wobei, wenn der Spiegel des Autoantikörpers gegen das Pim1 Protein in der Probe größer als der entsprechende Referenzspiegel des besagten Autoantikörpers ist, und wobei, wenn der Spiegel des Autoantikörpers gegen das SRC Protein, oder des Autoantikörpers gegen das MAPKAPK Protein, oder des Autoantikörpers gegen das FGFR4 Protein, oder des Autoantikörpers gegen das STK4 Protein, in der Probe größer als der entsprechende Referenzspiegel des besagten Autoantikörpers ist, und/oder wenn der Spiegel des Autoantikörpers gegen ACVR2B in der Probe kleiner als der Referenzspiegel des besagten Autoantikörpers ist, dann der Patient unter einem CRC mit einer schlechten Prognose leidet oder ein CRC mit einem ungünstigen Verlauf zeigt.

3. Das Verfahren nach irgendeinem der Ansprüche 1 oder 2, das das Bestimmen des Spiegels eines Autoantikörpers gegen das Pim1 Protein, und des Spiegels eines Autoantikörpers gegen das MAPKAPK3 Protein oder des Spiegels eines Autoantikörpers gegen das ACVR2B Protein umfasst.

4. Das Verfahren nach irgendeinem der Ansprüche 1 oder 2, das das Bestimmen des Spiegels eines Autoantikörpers gegen das Pim1 Protein, des Spiegels eines Autoantikörpers gegen das MAPKAPK3 Protein und des Spiegels eines Autoantikörpers gegen das ACVR2B Protein umfasst.

5. Das Verfahren nach Anspruch 4, das weiterhin das Bestimmen des Spiegels eines Autoantikörpers gegen das FGFR4 Protein umfasst.

6. Ein Verfahren zum Gewinnen von Daten in einer Probe von einem Subjekt, das das Nachweisen des Expressionsprodukts des *Pim1* Gens und des Expressionsprodukts von mindestens einem Gen ausgewählt aus der Gruppe bestehend aus den *SRC, MAPKAPK3, FGFR4, STK4* und *ACVR2B* Genen, und wenn gewünscht, das Quantifizieren des Spiegels der Expression des Expressionsprodukts der besagten Gene in der Probe umfasst, wobei die Probe Tumorzellen des Kolorektalkarzinoms (CRC) umfasst.

7. Ein Verfahren zur Diagnose, ob ein Subjekt unter Kolorektalkarzinom (CRC) leidet, die das Vergleichen des Spiegels der Expression eines Expressionsprodukts des *Pim1* Gens und des Expressionsspiegels von mindestens einem Expressionsprodukt eines Gens, wobei das Gen ausgewählt aus der Gruppe bestehend aus den *SRC, MAPKAPK3, FGFR4, STK4* und *ACVR2B* Genen, mit den Referenzspiegeln des Expressionsprodukts der besagten Gene in einer Probe von dem Subjekt umfasst, wobei, wenn der Spiegel des Expressionsprodukts des *Pim1* Gens größer als der entsprechende Referenzspiegel des Expressionsprodukts des besagten Gens und des Spiegels des Expressionsprodukts des *SRC* Gens oder des Expressionsprodukts des *MAPKAPK3* Gens oder des Expressionsprodukts des *FGFR4* Gens oder des Expressionsprodukts des *STK4* Gens größer als der entsprechende Referenzspiegel des Expressionsprodukts des besagten Gens ist und/oder wenn der Spiegel des Expressionsprodukts des *ACVR2B* Gens geringer als der Referenzspiegel des Expressionsprodukts des besagten Gens ist, ist das Subjekt mit CRC diagnostiziert.

8. Ein Verfahren zur Bewertung der Prognose oder Verfolgung des Fortschritts eines Patienten, der unter Kolorektalkarzinom (CRC) leidet, das das Vergleichen des Spiegels der Expression eines Expressionsprodukts des *Pim1* Gens und des Spiegels der Expression mindestens einem Expressionsprodukt eines Gens, wobei das Gen ausgewählt ist aus der Gruppe bestehend aus dem *SRC, MAPKAPK3, FGFR4, STK4* und *ACVR2B* Genen, mit dem Referenzspiegel des Expressionsprodukts des besagten Gens in einer Probe von dem Patienten umfasst, der unter CRC leidet, wobei, wenn der Spiegel des Expressionsprodukts des *Pim1* Gens größer als der entsprechende Referenzspiegel für das Expressionsprodukt des besagten Gens und des Spiegels des Expressionsprodukts des *SRC* Gens, oder des Expressionsprodukts des *MAPKAPK3* Gens, oder des Expressionsprodukts des *FGFR4* Gens oder des Expressionsprodukts des *STK4* Gens größer als der entsprechende Referenzspiegel des Expressionsprodukts des besagten Gens ist und/oder wenn der Spiegel des Expressionsprodukts des *ACVR2B* Gens geringer als der Referenzspiegels des Expressionsprodukts des besagten Gens ist, der Patient an einem CRC mit einer schlechten Prognose leidet oder ein CRC mit einem ungünstigen Verlauf zeigt.

9. Das Verfahren nach irgendeinem der Ansprüche 6 bis 8, das das Bestimmen des Spiegels der Expression des Expressionsprodukts des *Pim1* Gens und des Spiegels der Expression eines Expressionsprodukts des *MAPKAPK3* Gens oder des Spiegels der Expression eines Expressionsprodukts des *ACVR2B* Gens umfasst.

10. Das Verfahren nach irgendeinem der Ansprüche 6 bis 8, das das Bestimmen des Spiegels der Expression eines Expressionsprodukts des *Pim1* Gens, des Spiegels der Expression eines Expressionsprodukts des *MAPKAPK3* Gens und des Spiegels der Expression eines Expressionsprodukts des *ACVR2B* Gens umfasst.

11. Das Verfahren nach Anspruch 10, das weiterhin das Bestimmen des Spiegels der Expression eines Expressionsprodukts des *FGFR4* Gens umfasst.

12. Das Verfahren nach irgendeinem der Ansprüche 6 bis 8, wobei das Expressionsprodukt des *Pim1, SRC, MAPKAPK3, FGFR4, STK4* oder *ACVR2B* Gens das Pim1, SRC, MAPKAPK3, FGFR4, STK4 oder ACVR2B Protein oder ein Fragment davon ist.

13. Das Verfahren nach Anspruch 12, wobei der Spiegel des besagten Proteins mittels eines Immunoassays bestimmt wird.

14. Ein Kit, umfassend:
- die für das Bestimmen von mindestens einem Autoantikörper gegen das Pim1 Protein und einen Autoantikörper ausgewählt aus der Gruppe bestehend aus einem Autoantikörper gegen das SRC Protein, einem Autoantikörper gegen das MAPKAPK3 Protein, einem Autoantikörper gegen das FGFR4 Protein, einem Autoantikörper gegen das STK4 Protein und einem Autoantikörper gegen das ACVR2B Protein notwendigen Bestandteile, oder alternativ
- die für das Bestimmen eines Expressionsprodukts des *Pim1* Gens und mindestens eines Expressionsprodukts eines Gens ausgewählt aus der Gruppe bestehend aus den *SRC, MAPKAPK3, FGFR4, STK4* und *ACVR2B* Genen notwendigen Bestandteile.

15. Verwendung eines Kits nach Anspruch 14 für
- die Diagnose, ob ein Subjekt unter Kolorektalkarzinom (CRC) leidet; oder für
- die Beurteilung der Prognose oder das Verfolgen des Verlaufs eines Patienten, der unter CRC leidet.

## Revendications

1. Procédé pour diagnostiquer si un sujet souffre d'un cancer colorectal (CRC), qui comprend la comparaison du taux d'un auto-anticorps dirigé contre la protéine Pim1 et du taux d'au moins un auto-anticorps dirigé contre une protéine, dans lequel ledit auto-anticorps est choisi dans le groupe constitué par un auto-anticorps dirigé contre la protéine SRC, un auto-anticorps dirigé contre la protéine MAPKAPK3, un auto-anticorps dirigé contre la protéine FGFR4, un auto-anticorps dirigé contre la protéine STK4, et un auto-anticorps dirigé contre la protéine ACVR2B, dans un échantillon biologique issu dudit sujet, avec le taux de référence pour lesdits auto-anticorps, dans lequel si le taux dudit auto-anticorps dirigé contre la protéine Pim1 dans ledit échantillon est plus grand que le taux de référence correspondant pour ledit auto-anticorps, et dans lequel si le taux dudit auto-anticorps dirigé contre la protéine SRC, ou dudit auto-anticorps dirigé contre la protéine MAPKAPK3, ou dudit auto-anticorps dirigé contre la protéine FGFR4, ou dudit auto-anticorps dirigé contre la protéine STK4, dans ledit échantillon est plus grand que le taux de référence correspondant pour lesdits auto-anticorps et/ou si le taux de l'auto-anticorps dirigé contre ACVR2B dans ledit échantillon est plus petit que le taux de référence pour ledit auto-anticorps, alors ledit sujet est diagnostiqué avec un CRC.

2. Procédé d'évaluation du pronostic ou de suivi de la progression d'un patient souffrant de cancer colorectal (CRC), qui comprend la comparaison du taux d'un auto-anticorps dirigé contre la protéine Pim1 et du taux d'au moins un auto-anticorps dirigé contre une protéine, dans lequel ledit auto-anticorps est choisi dans le groupe constitué par un auto-anticorps dirigé contre la protéine SRC, un auto-anticorps dirigé contre la protéine MAPKAPK3, un auto-anticorps dirigé contre la protéine FGFR4, un auto-anticorps dirigé contre la protéine STK4 et un auto-anticorps dirigé contre la protéine ACVR2B, dans un échantillon biologique issu du patient souffrant de CRC, avec le taux de référence pour lesdits auto-anticorps, dans lequel si le taux dudit auto-anticorps dirigé contre la protéine Pim1 dans ledit échantillon est plus grand que le taux de référence correspondant pour ledit auto-anticorps, et dans lequel si le taux dudit auto-anticorps dirigé contre la protéine SRC, ou dudit auto-anticorps dirigé contre la protéine MAPKAPK3, ou dudit auto-anticorps dirigé contre la protéine FGFR4, ou dudit auto-anticorps dirigé contre la protéine STK4, dans ledit échantillon est plus grand que le taux de référence correspondant pour lesdits auto-anticorps et/ou si le taux de l'auto-anticorps dirigé contre ACVR2B dans ledit échantillon est plus petit que le taux de référence pour ledit auto-anticorps, alors ledit patient souffre d'un CRC avec un mauvais pronostic ou présente un CRC avec une progression défavorable.

3. Procédé selon l'une quelconque des revendications 1 ou 2, qui comprend la détermination du taux d'un auto-anticorps dirigé contre la protéine Pim1, et du taux d'un auto-anticorps dirigé contre la protéine MAPKAPK3 ou du taux d'un auto-anticorps dirigé contre la protéine ACVR2B.

4. Procédé selon l'une quelconque des revendications 1 ou 2, qui comprend la détermination du taux d'un auto-anticorps dirigé contre la protéine Pim1, du taux d'un auto-anticorps dirigé contre la protéine MAPKAPK3 et du taux d'un auto-anticorps dirigé contre la protéine ACVR2B.

5. Procédé selon la revendication 4, qui comprend de plus la détermination du taux d'un auto-anticorps dirigé contre la protéine FGFR4.

6. Procédé d'obtention de données dans un échantillon issu d'un sujet qui comprend la détection du produit d'expression du gène *Pim1* et du produit d'expression d'au moins un gène choisi dans le groupe constitué par les gènes *SRC, MAPKAPK3, FGFR4, STK4* et *ACVR2B,* et, si on le souhaite, la quantification du taux d'expression dudit produit d'expression desdits gènes dans ledit échantillon, dans lequel ledit échantillon comprend des cellules tumorales du cancer colorectal (CRC).

7. Procédé pour diagnostiquer si un sujet souffre de cancer colorectal (CRC), qui comprend la comparaison du taux d'expression d'un produit d'expression du gène *Pim1* et du taux d'expression d'au moins un produit d'expression d'un gène, dans lequel ledit gène est choisi dans le groupe constitué par les gènes *SRC, MAPKAPK3, FGFR4, STK4* et *ACVR2B,* dans un échantillon issu dudit sujet, avec le taux de référence pour ledit produit d'expression desdits gènes, dans lequel si le taux dudit produit d'expression du gène *Pim1* est plus grand que le taux de référence correspondant pour ledit produit d'expression dudit gène et le taux dudit produit d'expression du gène *SRC,* ou dudit produit d'expression du gène *MAPKAPK3,* ou dudit produit d'expression du gène FGFR4, ou dudit produit d'expression du gène *STK4,* est plus grand que le taux de référence correspondant pour lesdits produits d'expression desdits gènes et/ou si le taux du produit d'expression du gène *ACVR2B* est plus petit que le taux de référence pour ledit produit d'expression dudit gène, ledit sujet est diagnostiqué avec un CRC.

8. Procédé d'évaluation du pronostic ou de suivi de la progression d'un patient souffrant de cancer colorectal (CRC), qui comprend la comparaison du taux d'expression d'un produit d'expression du gène *Pim1* et du taux d'expression d'au moins un produit d'expression d'un gène, dans lequel ledit gène est choisi dans le groupe constitué par les gènes *SRC, MAPKAPK3, FGFR4, STK4* et *ACVR2B,* dans un échantillon issu dudit patient souffrant de CRC, avec le taux de référence pour ledit produit d'expression desdits gènes, dans lequel si le taux dudit produit d'expression du gène *Pim1* est plus grand que le taux de référence correspondant pour ledit produit d'expression dudit gène et le taux dudit produit d'expression du gène *SRC,* ou dudit produit d'expression du gène *MAPKAPK3,* ou dudit produit d'expression du gène *FGFR4,* ou dudit produit d'expression du gène *STK4,* est plus grand que le taux de référence correspondant pour lesdits produits d'expression desdits gènes et/ou si le taux du produit d'expression du gène *ACVR2B* est plus petit que le taux de référence pour ledit produit d'expression dudit gène, ledit patient souffre d'un CRC avec un mauvais pronostic ou présente un CRC avec une progression défavorable.

9. Procédé selon l'une quelconque des revendications 6 à 8, qui comprend la détermination du taux d'expression d'un produit d'expression du gène *Pim1,* et du taux d'expression d'un produit d'expression du gène *MAPKAPK3* ou du taux d'expression d'un produit d'expression du gène *ACVR2B.*

10. Procédé selon l'une quelconque des revendications 6 à 8, qui comprend la détermination du taux d'expression d'un produit d'expression du gène *Pim1,* du taux d'expression d'un produit d'expression du gène *MAPKAPK3* et du taux d'expression d'un produit d'expression du gène *ACVR2B.*

11. Procédé selon la revendication 10, qui comprend de plus la détermination du taux d'expression d'un produit d'expression du gène *FGFR4.*

12. Procédé selon l'une quelconque des revendications 6 à 8, dans lequel ledit produit d'expression du gène *Pim1, SRC, MAPKAPK3, FGFR4, STK4* ou *ACVR2B* est la protéine Pim1, SRC, MAPKAPK3, FGFR4, STK4 ou ACVR2B, respectivement, ou un fragment de celles-ci.

13. Procédé selon la revendication 12, dans lequel le taux de ladite protéine est déterminé au moyen d'un immunodosage.

14. Kit comprenant :
- les éléments nécessaires pour détecter au moins un auto-anticorps dirigé contre la protéine Pim1 et un auto-anticorps choisi dans le groupe constitué par un auto-anticorps dirigé contre la protéine SRC, un auto-anticorps dirigé contre la protéine MAPKAPK3, un auto-anticorps dirigé contre la protéine FGFR4, un auto-anticorps dirigé contre la protéine STK4, et un auto-anticorps dirigé contre la protéine ACVR2B ou en variante
- les éléments nécessaires pour détecter un produit d'expression du gène *Pim1* et au moins un produit d'expression d'un gène choisi dans le groupe constitué par les gènes *SRC, MAPKAPK3, FGFR4, STK4* et *ACVR2B.*

15. Utilisation d'un kit selon la revendication 14 pour
- diagnostiquer si un sujet souffre de cancer colorectal (CRC) ; ou pour
- évaluer le pronostic ou le suivi de la progression d'un patient souffrant de CRC.
